# EUROPEAN PATENT APPLICATION

(11) **EP 4 137 495 A1**
(43) Date of publication of application: **22.02.2023**
(21) Application number: 21809732.7
(22) Date of filing: 18.05.2021
(51) Int. Cl.: C07D 498/18, A61K 31/439, A61P 35/00, A61P 9/00, A61P 29/00, A61P 37/00, A61P 37/06, A61P 31/00, A61P 31/12, A61P 3/00

(54) **SOLID FORM OF MACROCYCLIC COMPOUND, PREPARATION THEREFOR AND USE THEREOF**

(30) Priority: 18.05.2020 CN 202010421308
(71) Applicant: Shenzhen TargetRx, Inc., Shenzhen, Guangdong 518057 (CN)
(72) Inventor: WANG, Yihan, Shenzhen, Guangdong 518057 (CN); LI, Huanyin, Shenzhen, Guangdong 518057 (CN)
(74) Representative: Novitas Patent AB
(86) International application number: PCT/CN2021/094362
(87) International publication number: WO 2021/233296

(57) **Abstract**

The present invention relates to a crystalline form of a free base of a compound of formula (A) (compound A) or a pharmaceutically acceptable salt thereof, a preparation method therefor, and use of the compound in the preparation of a medicament for treating and/or preventing diseases mediated by ALK kinase and mutants thereof, such as cell proliferative diseases, inflammation, infections, immunological diseases, organ transplantation, viral diseases, cardiovascular diseases or metabolic diseases.

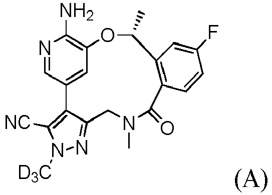

## Description

### FIELD OF THE INVENTION

The present disclosure belongs to the field of pharmaceutical technology and relates in particular to crystalline forms of the free base of macrocyclic compound (10R)-7-amino-12-fluoro-2-(methyl-d3)-10,16-dimethyl-15-oxo-10,15,16,17-tetrahydro-2H-8 ,4-(metheno)pyrazolo[4,3-h][2,5,11]benzoxadiazacyclotetradecine-3-carbonitrile (compound of formula (A) or compound A) or pharmaceutically acceptable salts thereof, as well as methods of preparation thereof, and use of the compounds in the manufacture of a medicament for the treatment of diseases mediated by anaplastic lymphoma kinase (ALK), c-ros oncogene 1 (ROS1) or mutants thereof, such as non-small cell lung cancer.

### BACKGROUND OF THE INVENTION

The chemical formula of compound A is C₂₁H₁₆D₃FN₆O₂, the molecular weight is 409.17 g/mol, and the chemical structure is:

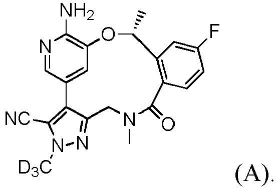

Compound A is an ALK- and ROS 1-kinase inhibitor having a deuterium atom, which can be used for the treatment of diseases mediated by ALK and ROS1 kinases and mutants thereof, cell proliferative diseases, inflammation, infection, immunological diseases, organ transplantation, viral diseases, cardiovascular diseases or metabolic diseases, such as non-small cell lung cancer, lung cancer, head and neck cancer, breast cancer, prostate cancer, esophageal cancer, rectal cancer, colon cancer, nasopharyngeal cancer, uterine cancer, pancreatic cancer, lymphoma, blood cancer, osteosarcoma, melanoma, kidney cancer, stomach cancer, liver cancer, bladder cancer, thyroid cancer, large intestine cancer, rheumatoid arthritis, osteoarthritis, rheumatoid spondylitis, gout, asthma, bronchitis, rhinitis, chronic obstructive pulmonary disease, or cystic fibrosis. International Patent Publication No. WO 2017/148325 A1 first disclosed this compound, but did not disclose the crystalline form of Compound A. The applicant of WO 2017/148325 A1 is Shenzhen TargetRx Inc. The corresponding Chinese application CN 201780013374.9 of WO 2017/148325 A1 was published with Grant No. CN 108699081 B on October 18, 2019. US application No. US 16/081,611 was published with Grant No. US 10543199 B2 on January 28, 2020. An intention to grant a patent for European application No. EP 17759176.5 was issued. Japanese application JP 2018-545928 is still under examination. The contents of each of the above applications are incorporated herein by reference in their entirety.

ALK is a receptor-type protein tyrosine kinase that belongs to the insulin receptor superfamily. It was discovered by Morris and Shiota et al. in 1994 as a product of chromosome rearrangement in anaplastic large cell lymphoma (ALCL). The most frequently found fusion is the one that the NPM (Nucleophosmin) gene on chromosome 5 fuses with the ALK gene on chromosome 2. NPM-ALK fusion protein was detected in nearly 75% of ALK-positive ALCL patients. It was found in subsequent studies that different forms of ALK fusion were present in various cancers, including inflammatory myofibroblastoma and diffuse large B-cell lymphoma. In 2007, Soda et al. discovered that the occurrence rate of the EML4-ALK fusion protein in non-small-cell lung cancer (NSCLC) is 5%.

ROS1 is a proto-oncogene receptor tyrosine kinase that belongs to the insulin receptor subfamily, and is involved in cell proliferation and differentiation processes. ROS1 is expressed, in humans, in epithelial cells of a variety of different tissues. ROS1 expression and/or activation have been found in glioblastoma, as well as tumors of the central nervous system. Genetic alterations involving ROS1 result in aberrant fusion proteins of ROS1 kinase, including the FIG-ROS1 deletion translocation in glioblastoma and non-small cell lung cancer (NSCLC), the SLC34A2-ROS1 translocation in NSCLC, and the CD74-ROS1 translocation in NSCLC and cholangiocarcinoma. Additional fusions, including TPM3-ROS1, SDC4-ROS1, EZR-ROS1 and LRIG3-ROS1, have been reported in tumor samples of lung cancer patients.

### SUMMARY OF THE INVENTION

In one aspect, the present disclosure provides a variety of crystalline forms of the free base of compound A.

In one embodiment, the present disclosure provides crystal form I of compound A (compound A crystal form I).

In one embodiment, the present disclosure provides crystal form II of compound A (compound A crystal form II).

In one embodiment, the present disclosure provides crystal form III of compound A (compound A crystal form III).

In one embodiment, the present disclosure provides crystal form IV of compound A (compound A crystal form IV).

In one embodiment, the present disclosure provides crystal form V of compound A (compound A crystal form V).

In one embodiment, the present disclosure provides crystal form VI of compound A (compound A crystal form VI).

In one embodiment, the present disclosure provides crystal form VII of compound A (compound A crystal form VII).

In another aspect, the present disclosure provides a variety of crystalline forms of salts of compound A.

In one embodiment, the present disclosure provides crystal form I of compound A maleate (compound A maleate crystal form I).

In one embodiment, the present disclosure provides crystal form I of compound A acetate (compound A acetate crystal form I).

In one embodiment, the present disclosure provides crystal form I of compound A p-toluenesulfonate (compound A p-toluenesulfonate crystal form I).

In one embodiment, the present disclosure provides crystal form I of compound A oxalate (compound A oxalate crystal form I).

In one embodiment, the present disclosure provides crystal form I of compound A sulfate (compound A sulfate crystal form I).

In one embodiment, the present disclosure provides crystal form I of compound A hydrobromide (compound A hydrobromide crystal form I).

In one embodiment, the present disclosure provides crystal form I of compound A hydrochloride (compound A hydrochloride crystal form I).

In one embodiment, the present disclosure provides crystal form I of compound A mesylate (compound A mesylate crystal form I).

In another aspect, the present disclosure provides a pharmaceutical composition comprising any of the crystal forms of the present disclosure, and a pharmaceutically acceptable excipient.

In another aspect, the present disclosure provides a pharmaceutical composition comprising (i) a pharmaceutically active ingredient: a crystal form of the free base of compound A or a crystal form of a pharmaceutically acceptable salt thereof, (ii) a diluent, (iii) a disintegrant, (iv) a binder, and (v) a lubricant.

In another aspect, the present disclosure provides the use of any of the crystal forms of the present disclosure in the manufacture of a medicament for the treatment and/or prevention of diseases mediated by ALK and ROS1 kinases and mutants thereof.

In another aspect, the present disclosure provides any of the crystal forms of the present disclosure for use in the treatment and/or prevention of diseases mediated by ALK and ROS1 kinases and mutants thereof.

In another aspect, the present disclosure provides a method of treating and/or preventing diseases mediated by ALK and ROS1 kinases and mutants thereof in a subject, comprising administering to the subject any of the crystal forms of the present disclosure.

In one embodiment, the above diseases include cell proliferative diseases, inflammation, infection, immunological diseases, organ transplantation, viral diseases, cardiovascular diseases or metabolic diseases, such as non-small cell lung cancer, lung cancer, head and neck cancer, breast cancer, prostate cancer, esophageal cancer, rectal cancer, colon cancer, nasopharyngeal cancer, uterine cancer, pancreatic cancer, lymphoma, blood cancer, osteosarcoma, melanoma, kidney cancer, stomach cancer, liver cancer, bladder cancer, thyroid cancer, large intestine cancer, rheumatoid arthritis, osteoarthritis, rheumatoid spondylitis, gout, asthma, bronchitis, rhinitis, chronic obstructive pulmonary disease, or cystic fibrosis.

### BRIEF DESCRIPTION OF THE DRAWINGS

FIG. 1 XRPD pattern of compound A crystal form I.
FIG. 2 DSC curve of compound A crystal form I.
FIG. 3 TGA curve of compound A crystal form I.
FIG. 4 DVS curve of compound A crystal form I.
FIG. 5 XRPD comparison of compound A crystal form I before and after DVS test.
FIG. 6 ¹H NMR spectrum of compound A crystal form I.
FIG. 7 ¹³C NMR spectrum of compound A crystal form I.
FIG. 8 DEPT spectrum of compound A crystal form I.
FIG. 9 D NMR spectrum of compound A crystal form I.
FIG. 10 ¹⁹F NMR spectrum of compound A crystal form I.
FIG. 11 HSQC-NMR spectrum of compound A crystal form I.
FIG. 12 HMBC-NMR spectrum of compound A crystal form I.
FIG. 13 COSY-NMR spectrum of compound A crystal form I.
FIG. 14 IR spectrum of compound A crystal form I.
FIG. 15 UV spectrum of compound A crystal form I.
FIG. 16 HR-MS spectrum of compound A crystal form I.
FIG. 17 XRPD pattern of compound A crystal form II.
FIG. 18 XRPD pattern of compound A crystal form III.
FIG. 19 XRPD pattern of compound A crystal form IV
FIG. 20 XRPD pattern of compound A crystal form V
FIG. 21 XRPD pattern of compound A crystal form VI.
FIG. 22 XRPD pattern of compound A crystal form VII.
FIG. 23 XRPD pattern of the solid obtained by slurrying the free base of compound A at room temperature.
FIG. 24 XRPD pattern of compound A crystal form V heated to 190 °C.
FIG. 25 XRPD pattern of compound A maleate crystal form I.
FIG. 26 DVS curve of compound A maleate crystal form I.
FIG. 27 XRPD comparison of compound A maleate crystal form I before and after DVS test.
FIG. 28 XRPD pattern of compound A acetate crystal form I.
FIG. 29 DVS curve of compound A acetate crystal form I.
FIG. 30 XRPD comparison of compound A acetate crystal form I before and after DVS test.
FIG. 31 XRPD pattern of compound A p-toluenesulfonate crystal form I.
FIG. 32 DVS curve of compound A p-toluenesulfonate crystal form I.
FIG. 33 XRPD comparison of compound A p-toluenesulfonate crystal form I before and after DVS test.
FIG. 34 XRPD pattern of compound A oxalate crystal form I.
FIG. 35XRPD pattern of compound A sulfate crystal form I.
FIG. 36 XRPD pattern of compound A hydrobromide crystal form I.
FIG. 37 XRPD pattern of compound A hydrochloride crystal form I.
FIG. 38 DVS curve of compound A hydrochloride crystal form I.
FIG. 39 XRPD comparison of compound A hydrochloride crystal form I before and after DVS test.
FIG. 40 XRPD pattern of compound A mesylate crystal form I.
FIG. 41 XRPD pattern of accelerated stability study of compound A crystal form I.
FIG. 42 XRPD pattern of accelerated stability study of compound A maleate crystal form I.
FIG. 43 XRPD pattern of accelerated stability study of compound A acetate crystal form I.
FIG. 44 XRPD pattern of accelerated stability study of compound A p-toluenesulfonate crystal form I.
FIG. 45 XRPD pattern of compound A crystal form I in different dissolution media.
FIG. 46 XRPD pattern of compound A maleate crystal form I in different dissolution media.
FIG. 47 XRPD pattern of compound A acetate crystal form I in different dissolution media.
FIG. 48 Single crystal structure of compound A crystal form I.
FIG. 49 XRPD comparison of calculated and measured values of the single crystal sample of compound A.

### DETAILED DESCRIPTION OF THE INVENTION

The present disclosure can be understood more readily by reference to the following detailed description of embodiments of the disclosure and the examples included herein. It is to be understood that the terms used herein are intended to describe specific embodiments only and are not intended to be limiting. It is to be further understood that unless specifically defined by the context, terms used herein shall be given their ordinary meanings as known in the relevant art.

As used herein, the singular forms "a", "an" and "the" include plural references unless specified otherwise. For example, the term "a" compound includes one or more compounds.

The term "about" means having a value that falls within the standard error of the accepted mean when considered by a person of ordinary skill in the art. For example, "about" means ±10% of the indicated amount, or ±5% of the indicated amount.

As used herein, the term "substantially" means taking into account the typical variability of a particular method and the standard error of a measured value. For example, with respect to the location of an X-ray powder diffraction peak, the term "substantially" is meant to take into account the typical variability in peak location and intensity. Those skilled in the art will recognize that peak location (2θ) will exhibit some variability, typically up to ±0.2°. In addition, those skilled in the art will recognize that relative peak intensities will reveal inter-device variability as well as variability due to crystallinity, preferred orientation, sample surface tested, and other factors known to those of skill in the art. Similarly, the NMR spectra (ppm) of ¹H, ¹³C and ¹⁹F show variability, typically up to ±0.2 ppm.

As used herein, the terms "crystalline" and "crystal form" refer to a solid composed of molecules with regular repetitive arrangement. Crystalline forms may differ in terms of thermodynamic stability, physical parameters, X-ray structure and preparation processes.

The term "amorphous" refers to a solid composed of molecules with disordered arrangement.

As used herein, the term "solvate" refers to a crystalline form having a stoichiometric or non-stoichiometric amount of a solvent (e.g., water, methanol, ethyl acetate, etc., or mixtures thereof) in the crystal lattice through non-covalent intermolecular bonding. The term "hydrate" refers to a solvate in which the solvent is water.

As used herein, the term "anhydrous" refers to a crystalline form that contains less than about 1% (w/w) adsorbed moisture as determined by standard methods such as Karl Fisher analysis.

### Compound A and crystal forms thereof

The compound, (10R)-7-amino-12-fluoro-2-(methyl-d3)-10,16-dimethyl-15-oxo-10,15,16,17-tetrahydro-2H-8 ,4-(metheno)pyrazolo[4,3-h][2,5,11]benzoxadiazacyclotetradecine-3-carbonitrile, is referred to herein as compound A, or the free base of compound A, and has the formula of:

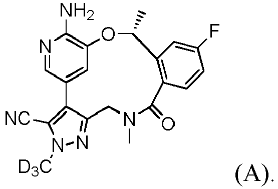

The present disclosure relates to a variety of crystalline forms of compound A, such as "compound A crystal form I", "compound A crystal form II", "compound A crystal form III", "compound A crystal form IV", "compound A crystal form V", "compound A crystal form VI" and "compound A crystal form VII". In some embodiments, these crystal forms of compound A may be solvated, hydrated, or unsolvated.

### Compound A crystal form I

In one embodiment, the present disclosure provides compound A crystal form I, which is an anhydrate.

In another embodiment, the X-ray powder diffraction pattern of the crystal form I obtained using CuK_{α} radiation includes at least the characteristic peaks located at the following °2θ: 16.175±0.2, 17.299±0.2 and 21.218±0.2. In another embodiment, the X-ray powder diffraction pattern further includes the characteristic peaks located at the following °2θ: 9.637±0.2, 12.555±0.2, 14.343±0.2 and 19.366±0.2. In another embodiment, the X-ray powder diffraction pattern further includes the characteristic peaks located at the following °2θ: 7.435±0.2, 10.11±0.2, 11.808±0.2, 14.922±0.2, 18.359±0.2, 19.859±0.2, 23.401±0.2, 23.939±0.2, 25.117±0.2, 25.727±0.2, 26.831±0.2 and 28.862±0.2.

In another embodiment, the X-ray powder diffraction pattern has the following characteristic peaks:

| Angle °2θ±0.2 °2θ | Relative intensity% |
|---|---|
| 9.637 | 38.2 |
| 12.555 | 38.1 |
| 14.343 | 34.5 |
| 16.175 | 100 |
| 17.299 | 68.5 |
| 19.366 | 34.7 |
| 21.218 | 54.1 |

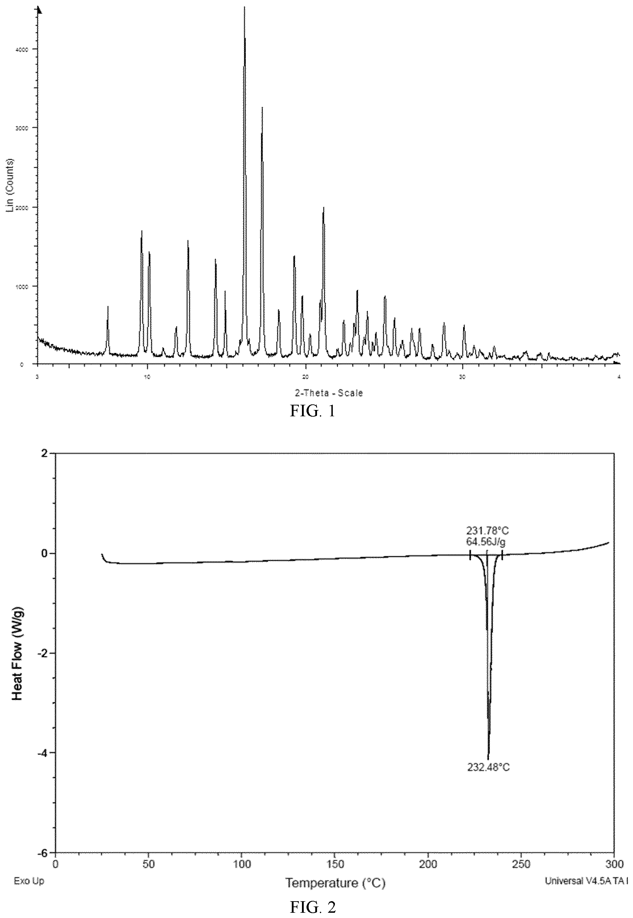

In another embodiment, the X-ray powder diffraction pattern comprises one or more peaks at the 2θ value in Table 3.1. In another embodiment, the X-ray powder diffraction pattern is substantially as shown in FIG. 1.

In another embodiment, the crystal form I has a melting endothermic peak at 232±2°C in differential scanning calorimetry analysis.

In another embodiment, the crystal form I has substantially no weight loss prior to 150°C in thermogravimetric analysis.

In another embodiment, the crystal form I has the following single crystal parameters:

| Space groups | *P2₁* |
|---|---|
| *a*/Å | 11.81120(10) |
| *b*/Å | 14.3957(2) |
| *c*/Å | 11.81800(10) |
| *a*/° | 90 |
| *β*/° | 94.5590(10) |
| γ/° | 90 |
| Volume/Å³ | 2003.06(4) |

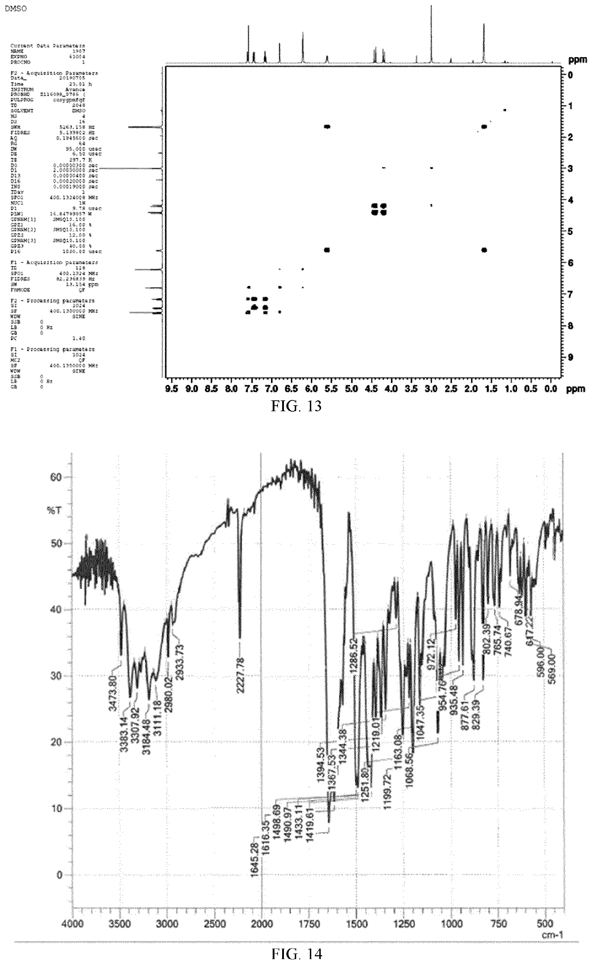

In another embodiment, the crystal form I has absorption peaks in an infrared absorption spectrum at the following cm⁻¹: 829±2, 878±2, 1069±2, 1252±2, 1344±2, 1368±2, 1395±2, 1420±2, 1433±2, 1491±2, 1499±2, 1616±2, 1645±2, 2228±2, 2934±2, 2980±2, 3111±2, 3184±2, 3308±2, 3383±2 and 3474±2. In another embodiment, the crystal form I has an infrared absorption spectrum substantially as shown in FIG. 14.

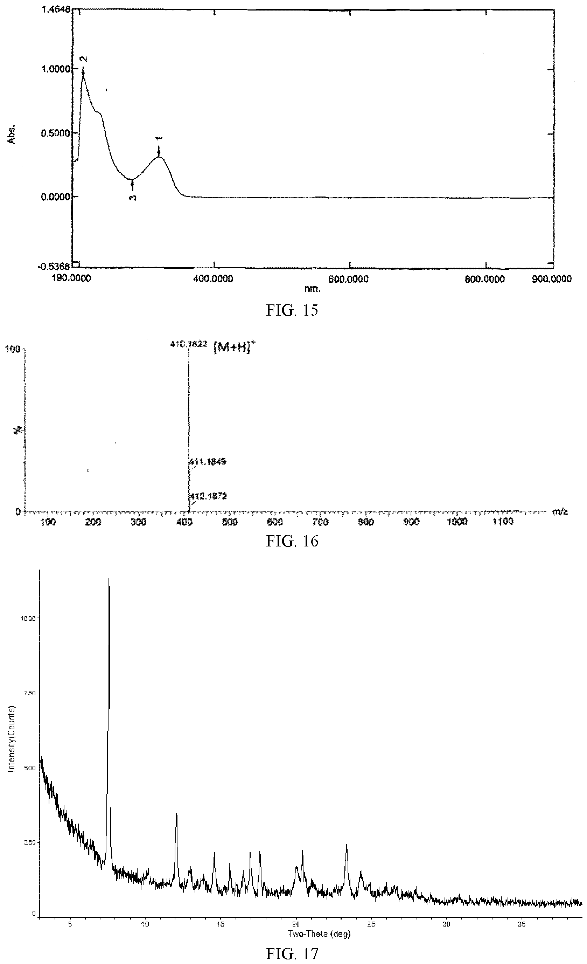

In another embodiment, the crystal form I has absorption peaks in the UV spectrum at the following nm: 206±2 and 317±2. In another embodiment, the crystal form I has a UV spectrum substantially as shown in FIG. 15.

In one embodiment, the present disclosure provides compound A crystal form II, which is a solvate of butanone.

In another embodiment, the X-ray powder diffraction pattern of the crystal form II obtained using CuK_{α} radiation includes at least the characteristic peaks located at the following °2θ: 7.591±0.2, 12.081±0.2 and 23.364±0.2. In another embodiment, the X-ray powder diffraction pattern further includes the characteristic peaks located at the following °2θ: 14.577±0.2, 15.595±0.2, 16.948±0.2, 17.615±0.2 and 20.448±0.2.

In another embodiment, the X-ray powder diffraction pattern has the following characteristic peaks:

| Angle °2θ±0.2 °2θ | Relative intensity% |
|---|---|
| 7.591 | 100 |
| 12.081 | 24.7 |
| 14.577 | 12.9 |
| 15.595 | 10.2 |
| 16.948 | 14.2 |
| 17.615 | 14.4 |
| 20.448 | 14.6 |
| 23.364 | 17.4 |

In another embodiment, the X-ray powder diffraction pattern comprises one or more peaks at the 2θ value in Table 3.2. In another embodiment, the X-ray powder diffraction pattern is substantially as shown in FIG. 17.

In another embodiment, the crystal form I has a melting endothermic peak at 230±2°C in differential scanning calorimetry analysis.

In another embodiment, the crystal form I has a weight loss of about 6.69% prior to 160°C in thermogravimetric analysis.

In one embodiment, the present disclosure provides compound A crystal form III, which is a solvate of butanone.

In another embodiment, the X-ray powder diffraction pattern of the crystal form III obtained using CuK_{α} radiation includes at least the characteristic peaks located at the following °2θ: 23.149±0.2. In another embodiment, the X-ray powder diffraction pattern further includes the characteristic peaks located at the following °2θ: 11.429±0.2, 13.027±0.2, 14.542±0.2, 17.949±0.2 and 26.994±0.2. In another embodiment, the X-ray powder diffraction pattern further includes the characteristic peaks located at the following °2θ: 10.543±0.2, 15.353±0.2, 18.362±0.2, 21.161±0.2, 22.506±0.2 and 26.006±0.2.

In another embodiment, the X-ray powder diffraction pattern has the following characteristic peaks:

| Angle °2θ±0.2 °2θ | Relative intensity% |
|---|---|
| 11.429 | 22.2 |
| 13.027 | 21.7 |
| 14.542 | 21.3 |
| 17.949 | 19.1 |
| 23.149 | 100 |
| 26.994 | 20.4 |

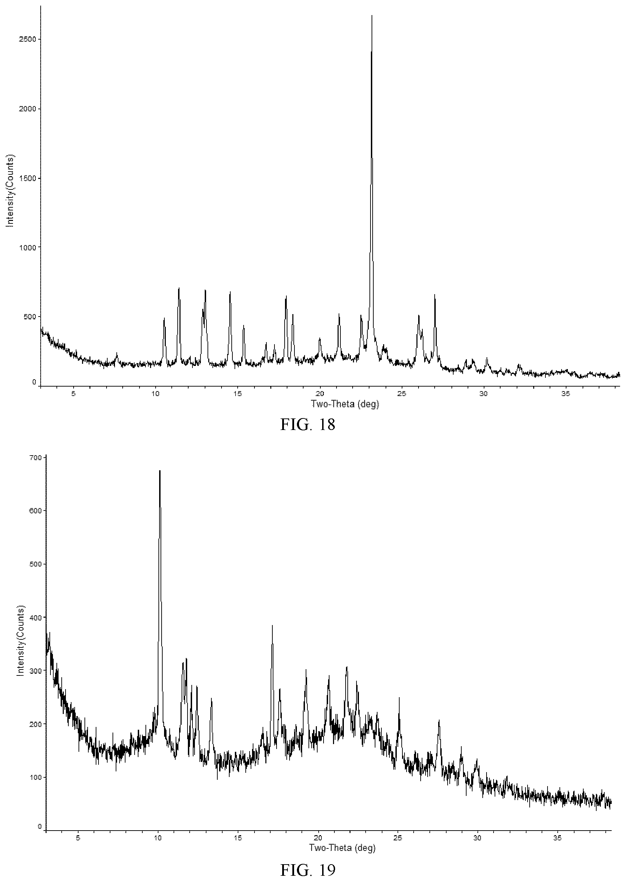

In another embodiment, the X-ray powder diffraction pattern comprises one or more peaks at the 2θ value in Table 3.3. In another embodiment, the X-ray powder diffraction pattern is substantially as shown in FIG. 18.

In another embodiment, the crystal form III has a melting endothermic peak at 226±2°C in differential scanning calorimetry analysis.

In another embodiment, the crystal form III has a weight loss of about 5.31% prior to 165°C in thermogravimetric analysis.

In one embodiment, the present disclosure provides compound A crystal form IV, which is an anhydrate.

In another embodiment, the X-ray powder diffraction pattern of the crystal form IV obtained using CuK_{α} radiation includes at least the characteristic peaks located at the following °2θ: 10.113±0.2. In another embodiment, the X-ray powder diffraction pattern further includes the characteristic peaks located at the following °2θ: 11.583±0.2, 11.768±0.2, 12.098±0.2, 17.143±0.2 and 19.267±0.2. In another embodiment, the X-ray powder diffraction pattern further includes the characteristic peaks located at the following °2θ: 9.718±0.2, 12.439±0.2, 13.339±0.2, 17.649±0.2, 20.703±0.2, 21.809±0.2, 22.427±0.2, 25.081±0.2, 27.576±0.2 and 28.959±0.2.

In another embodiment, the X-ray powder diffraction pattern has the following characteristic peaks:

| Angle °2θ±0.2 °2θ | Relative intensity% |
|---|---|
| 10.113 | 100 |
| 11.583 | 31 |
| 11.768 | 35.2 |
| 12.098 | 25.9 |
| 17.143 | 46.2 |
| 19.267 | 26.6 |

In another embodiment, the X-ray powder diffraction pattern comprises one or more peaks at the 2θ value in Table 3.4. In another embodiment, the X-ray powder diffraction pattern is substantially as shown in FIG. 19.

In another embodiment, the crystal form IV has a melting endothermic peak at 232±2°C in differential scanning calorimetry analysis.

In another embodiment, the crystal form IV has a weight loss of about 0.28% prior to 200°C in thermogravimetric analysis.

In one embodiment, the present disclosure provides compound A crystal form V, which is an anhydrate.

In another embodiment, the X-ray powder diffraction pattern of the crystal form V obtained using CuK_{α} radiation includes at least the characteristic peaks located at the following °2θ: 6.939±0.2, 16.276±0.2 and 17.494±0.2. In another embodiment, the X-ray powder diffraction pattern further includes the characteristic peaks located at the following °2θ: 4.912±0.2, 9.774±0.2, 12.709±0.2, 14.246±0.2, 14.482±0.2, 17.242±0.2, 18.519±0.2, 19.425±0.2, 21.001±0.2, 21.317±0.2, 22.734±0.2, 25.218±0.2 and 29.688±0.2. In another embodiment, the X-ray powder diffraction pattern further includes the characteristic peaks located at the following °2θ: 10.326±0.2, 10.859±0.2, 15.289±0.2, 16.708±0.2, 19.941±0.2, 23.09±0.2, 23.424±0.2, 24.25±0.2, 25.808±0.2, 26.241±0.2, 26.987±0.2, 28.841±0.2, 29.332±0.2, 31.071±0.2 and 31.856±0.2.

In another embodiment, the X-ray powder diffraction pattern has the following characteristic peaks:

| Angle °2θ±0.2 °2θ | Relative intensity% |
|---|---|
| 4.912 | 28.1 |
| 6.939 | 100 |
| 9.774 | 30.1 |
| 12.709 | 41.4 |
| 14.246 | 29.4 |
| 14.482 | 33.4 |
| 16.276 | 55.9 |
| 17.242 | 42.4 |
| 17.494 | 76.9 |
| 18.519 | 25.7 |
| 19.425 | 34.7 |
| 21.001 | 27.3 |
| 21.317 | 29.5 |
| 22.734 | 26.7 |
| 25.218 | 28.2 |
| 29.688 | 26.1 |

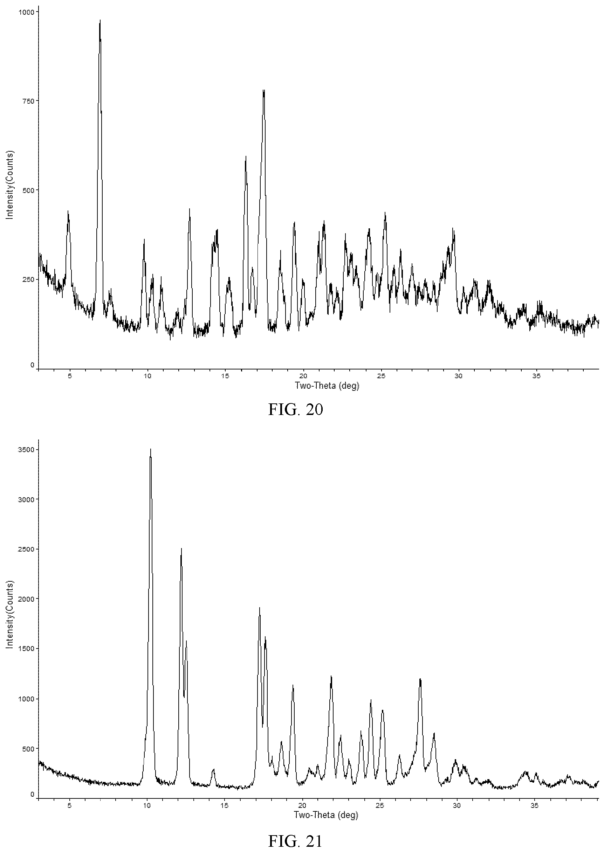

In another embodiment, the X-ray powder diffraction pattern comprises one or more peaks at the 2θ value in Table 3.5. In another embodiment, the X-ray powder diffraction pattern is substantially as shown in FIG. 20.

In another embodiment, the crystal form V has a melting endothermic peak at 232±2°C in differential scanning calorimetry analysis.

In another embodiment, the crystal form V has a weight loss of about 0.22% prior to 200°C in thermogravimetric analysis.

In one embodiment, the present disclosure provides compound A crystal form VI, which is an anhydrate.

In another embodiment, the X-ray powder diffraction pattern of the crystal form VI obtained using CuK_{α} radiation includes at least the characteristic peaks located at the following °2θ: 10.247±0.2, 12.198±0.2 and 17.258±0.2. In another embodiment, the X-ray powder diffraction pattern further includes the characteristic peaks located at the following °2θ: 12.514±0.2, 17.596±0.2, 19.406±0.2, 21.888±0.2 and 27.599±0.2. In another embodiment, the X-ray powder diffraction pattern further includes the characteristic peaks located at the following °2θ: 18.659±0.2, 22.479±0.2, 23.799±0.2, 24.41±0.2, 25.158±0.2 and 28.504±0.2.

In another embodiment, the X-ray powder diffraction pattern has the following characteristic peaks:

| Angle °2θ±0.2 °2θ | Relative intensity% |
|---|---|
| 10.247 | 100 |
| 12.198 | 70.6 |
| 12.514 | 42.8 |
| 17.258 | 53.8 |
| 17.596 | 44.5 |
| 19.406 | 27.9 |
| 21.888 | 30.3 |
| 27.599 | 27.5 |

In another embodiment, the X-ray powder diffraction pattern comprises one or more peaks at the 2θ value in Table 3.6. In another embodiment, the X-ray powder diffraction pattern is substantially as shown in FIG. 21.

In another embodiment, the crystal form VI has a melting endothermic peak at 233±2°C in differential scanning calorimetry analysis.

In another embodiment, the crystal form VI has substantially no weight loss prior to 200°C in thermogravimetric analysis.

In one embodiment, the present disclosure provides compound A crystal form VII, which is a solvate.

In another embodiment, the X-ray powder diffraction pattern of the crystal form VII obtained using CuK_{α} radiation includes at least the characteristic peaks located at the following °2θ: 7.138±0.2 and 9.876±0.2. In another embodiment, the X-ray powder diffraction pattern further includes the characteristic peaks located at the following °2θ: 12.572±0.2, 12.945±0.2, 14.675±0.2 and 17.16±0.2.

In another embodiment, the X-ray powder diffraction pattern has the following characteristic peaks:

| Angle °2θ±0.2 °2θ | Relative intensity% |
|---|---|
| 7.138 | 100 |
| 9.876 | 51.2 |
| 12.572 | 31.6 |
| 12.945 | 38.5 |
| 14.675 | 30.3 |
| 17.16 | 26.2 |

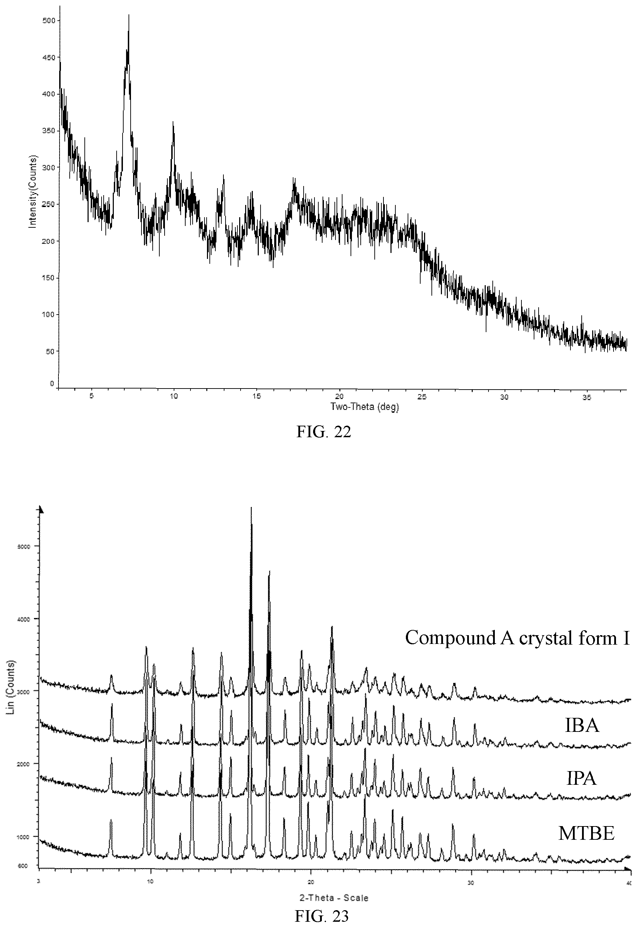

In another embodiment, the X-ray powder diffraction pattern comprises one or more peaks at the 2θ value in Table 3.7. In another embodiment, the X-ray powder diffraction pattern is substantially as shown in FIG. 22.

In another embodiment, the crystal form VII has a melting endothermic peak at 232±2°C in differential scanning calorimetry analysis.

In another embodiment, the crystal form VII has a weight loss of about 0.35% prior to 200°C in thermogravimetric analysis.

### Salts of compound A and crystal forms thereof

The present disclosure relates to a variety of salts of compound A, such as maleate, acetate, p-toluenesulfonate, hydrobromide, sulfate, oxalate, hydrochloride, and mesylate.

The present disclosure also relates to crystalline forms of a variety of salts of compound A, such as "compound A maleate crystal form I", "compound A acetate crystal form I", "compound A p-toluenesulfonate crystal form I", "compound A hydrobromide crystal form I", "compound A sulfate crystal form I", "compound A oxalate crystal form I", "compound A hydrochloride crystal form I" and "compound A mesylate crystal form I".

### Compound A maleate crystal form I

In one embodiment, the present disclosure provides compound A maleate (1:1) crystal form I, which is an anhydrate.

In another embodiment, the X-ray powder diffraction pattern of the crystal form obtained using CuK_{α} radiation includes at least the characteristic peaks located at the following °2θ: 9.737±0.2, 12.241±0.2 and 23.08±0.2. In another embodiment, the X-ray powder diffraction pattern further includes the characteristic peaks located at the following °2θ: 11.982±0.2, 13.601±0.2, 16.495±0.2, 17.186±0.2, 19.625±0.2 and 24.527±0.2. In another embodiment, the X-ray powder diffraction pattern further includes the characteristic peaks located at the following °2θ: 7.577±0.2, 15.286±0.2, 17.358±0.2, 17.553±0.2, 19.971±0.2, 22.087±0.2, 23.879±0.2, 25.239±0.2, 25.844±0.2, 26.189±0.2, 29.644±0.2 and 31.501±0.2.

In another embodiment, the X-ray powder diffraction pattern has the following characteristic peaks:

| Angle °2θ±0.2 °2θ | Relative intensity% |
|---|---|
| 9.737 | 80 |
| 11.982 | 41 |
| 12.241 | 100 |
| 13.601 | 28 |
| 16.495 | 32 |
| 17.186 | 25 |
| 19.625 | 38 |
| 23.08 | 98 |
| 24.527 | 36 |

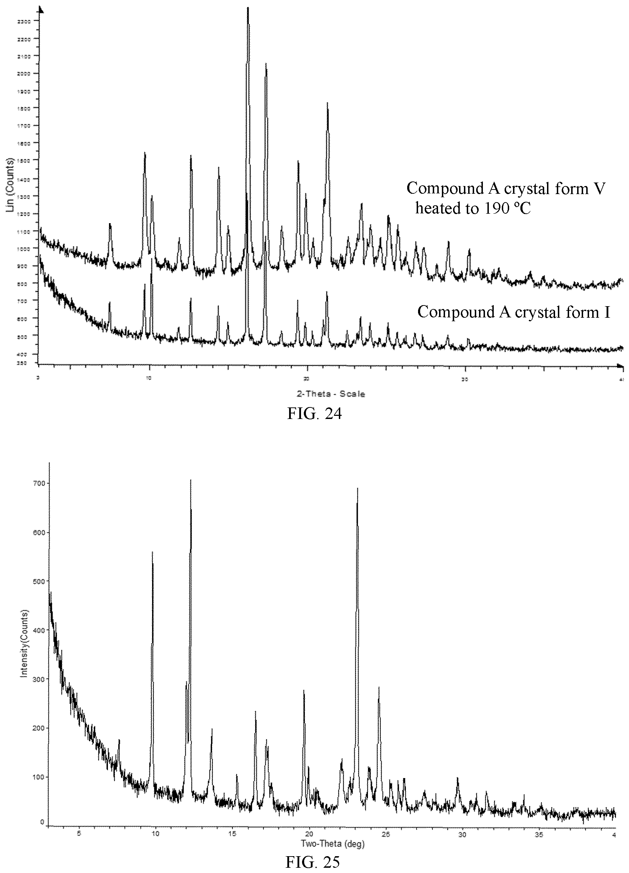

In another embodiment, the X-ray powder diffraction pattern comprises one or more peaks at the 2θ value in Table 7.1. In another embodiment, the X-ray powder diffraction pattern is substantially as shown in FIG. 25.

In another embodiment, the crystal form has a melting endothermic peak at 209±2°C in differential scanning calorimetry analysis.

In another embodiment, the crystal form has a weight loss of about 0.44% prior to 175°C in thermogravimetric analysis.

### Compound A acetate (1:1) crystal form I

In one embodiment, the present disclosure provides compound A acetate (1:1) crystal form I, which is an anhydrate.

In another embodiment, the X-ray powder diffraction pattern of the crystal form obtained using CuK_{α} radiation includes at least the characteristic peaks located at the following °2θ: 12.866±0.2 and 23.129±0.2. In another embodiment, the X-ray powder diffraction pattern further includes the characteristic peaks located at the following °2θ: 10.521±0.2, 11.409±0.2, 13.005±0.2, 14.521±0.2, 17.91±0.2 and 21.14±0.2. In another embodiment, the X-ray powder diffraction pattern further includes the characteristic peaks located at the following °2θ: 15.349±0.2, 16.707±0.2, 17.236±0.2, 18.343±0.2, 19.961±0.2, 22.536±0.2, 25.985±0.2 and 26.993±0.2.

In another embodiment, the X-ray powder diffraction pattern has the following characteristic peaks:

| Angle °2θ±0.2 °2θ | Relative intensity% |
|---|---|
| 10.521 | 35.1 |
| 11.409 | 46.7 |
| 12.866 | 50.7 |
| 13.005 | 26.2 |
| 14.521 | 45.3 |
| 17.91 | 39.3 |
| 21.14 | 27.3 |
| 23.129 | 100 |

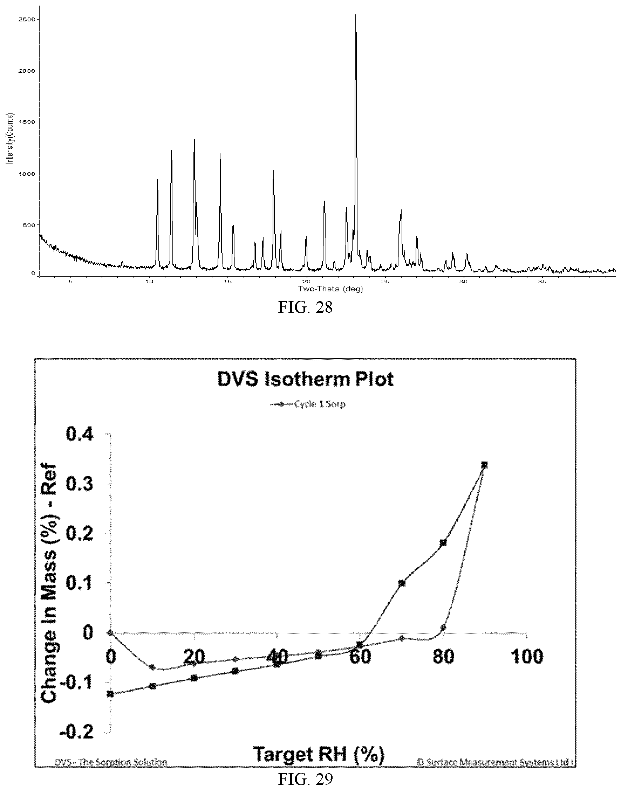

In another embodiment, the X-ray powder diffraction pattern comprises one or more peaks at the 2θ value in Table 7.2. In another embodiment, the X-ray powder diffraction pattern is substantially as shown in FIG. 28.

In another embodiment, the crystal form has a melting endothermic peak at 232±2°C in differential scanning calorimetry analysis.

In another embodiment, the crystal form has a weight loss of about 0.67% prior to 140°C in thermogravimetric analysis.

### Compound A p-toluenesulfonate (1: 1) crystal form I

In one embodiment, the present disclosure provides compound A p-toluenesulfonate (1:1) crystal form I, which is an anhydrate.

In another embodiment, the X-ray powder diffraction pattern of the crystal form obtained using CuK_{α} radiation includes at least the characteristic peaks located at the following °2θ: 10.583±0.2 and 21.674±0.2. In another embodiment, the X-ray powder diffraction pattern further includes the characteristic peaks located at the following °2θ: 12.968±0.2 and 14.503±0.2. In another embodiment, the X-ray powder diffraction pattern further includes the characteristic peaks located at the following °2θ: 5.544±0.2, 14.012±0.2, 16.886±0.2, 18.417±0.2, 19.607±0.2, 21.298±0.2, 23.266±0.2 and 26.437±0.2.

In another embodiment, the X-ray powder diffraction pattern has the following characteristic peaks:

| Angle °2θ±0.2 °2θ | Relative intensity% |
|---|---|
| 10.583 | 100 |
| 12.968 | 29.6 |
| 14.503 | 38.6 |
| 21.674 | 51.3 |

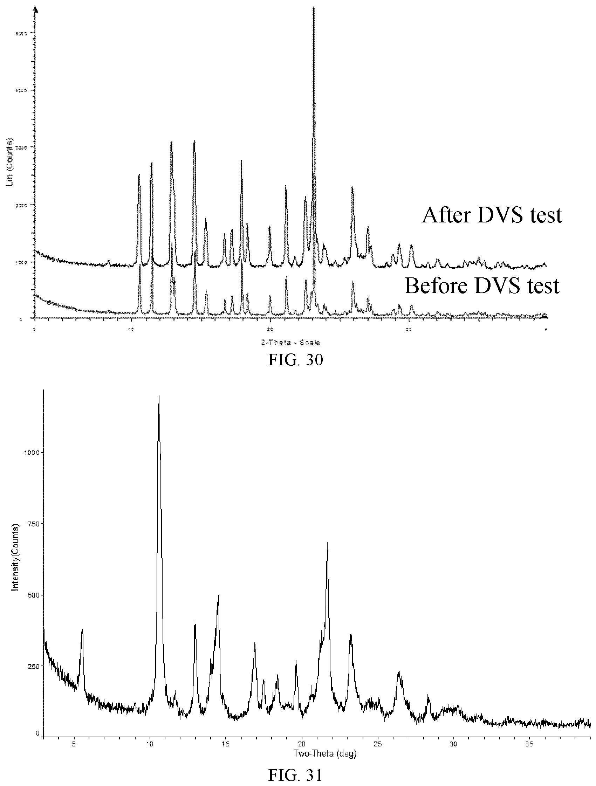

In another embodiment, the X-ray powder diffraction pattern comprises one or more peaks at the 2θ value in Table 7.3. In another embodiment, the X-ray powder diffraction pattern is substantially as shown in FIG. 31.

In another embodiment, the crystal form has a melting endothermic peak at 269±2°C in differential scanning calorimetry analysis.

In another embodiment, the crystal form has a weight loss of about 0.6% prior to 220°C in thermogravimetric analysis.

### Compound A oxalate (1:1) crystal form I

In one embodiment, the present disclosure provides compound A oxalate (1: 1) crystal form I, which is a solvate.

In another embodiment, the X-ray powder diffraction pattern of the crystal form obtained using CuK_{α} radiation includes at least the characteristic peaks located at the following °2θ: 4.32±0.2 and 6.642±0.2. In another embodiment, the X-ray powder diffraction pattern further includes the characteristic peaks located at the following °2θ: 5.54±0.2, 10.366±0.2, 10.98±0.2 and 13.242±0.2.

In another embodiment, the X-ray powder diffraction pattern has the following characteristic peaks:

| Angle °2θ±0.2 °2θ | Relative intensity% |
|---|---|
| 4.32 | 31.2 |
| 5.54 | 27.5 |
| 6.642 | 100 |
| 10.366 | 18.1 |
| 10.98 | 15 |
| 13.242 | 19.6 |

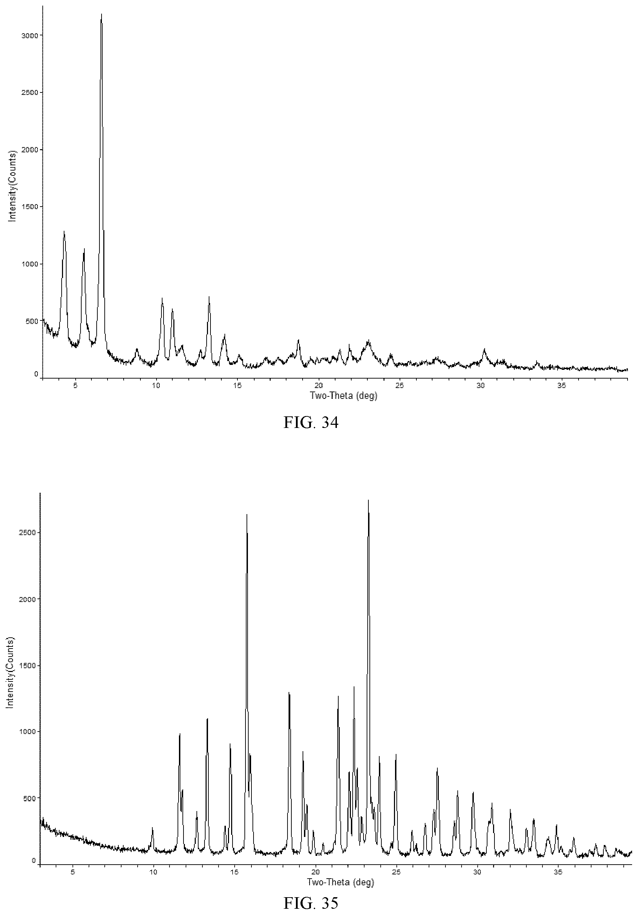

In another embodiment, the X-ray powder diffraction pattern comprises one or more peaks at the 2θ value in Table 7.4. In another embodiment, the X-ray powder diffraction pattern is substantially as shown in FIG. 34.

In another embodiment, the crystal form has a melting endothermic peak at 209±2°C in differential scanning calorimetry analysis.

In another embodiment, the crystal form has a weight loss of about 0.86% prior to 130°C in thermogravimetric analysis.

### Compound A sulfate crystal form I

In one embodiment, the present disclosure provides compound A sulfate crystal form I, which is a monohydrate.

In another embodiment, the X-ray powder diffraction pattern of the crystal form obtained using CuK_{α} radiation includes at least the characteristic peaks located at the following °2θ: 15.763±0.2 and 23.266±0.2. In another embodiment, the X-ray powder diffraction pattern further includes the characteristic peaks located at the following °2θ: 11.604±0.2, 13.318±0.2, 14.74±0.2, 15.961±0.2, 18.385±0.2, 19.228±0.2, 21.413±0.2, 22.361±0.2, 23.936±0.2 and 24.958±0.2. In another embodiment, the X-ray powder diffraction pattern further includes the characteristic peaks located at the following °2θ: 11.78±0.2, 12.667±0.2, 19.447±0.2, 22.083±0.2, 22.576±0.2, 23.618±0.2, 27.303±0.2, 27.522±0.2, 28.765±0.2, 29.725±0.2, 30.906±0.2 and 32.032±0.2.

In another embodiment, the X-ray powder diffraction pattern has the following characteristic peaks:

| Angle °2θ±0.2 °2θ | Relative intensity% |
|---|---|
| 11.604 | 33.5 |
| 13.318 | 37.9 |
| 14.74 | 30.9 |
| 15.763 | 96.3 |
| 15.961 | 28.5 |
| 18.385 | 45.4 |
| 19.228 | 28.6 |
| 21.413 | 44.1 |
| 22.361 | 44.3 |
| 23.266 | 100 |
| 23.936 | 27.4 |
| 24.958 | 28.3 |

In another embodiment, the X-ray powder diffraction pattern comprises one or more peaks at the 2θ value in Table 7.5. In another embodiment, the X-ray powder diffraction pattern is substantially as shown in FIG. 35.

In another embodiment, the crystal form has a melting endothermic peak at 251±2°C in differential scanning calorimetry analysis.

In another embodiment, the crystal form has a weight loss of about 2.95% prior to 90°C in thermogravimetric analysis.

### Compound A hydrobromide crystal form I

In one embodiment, the present disclosure provides compound A hydrobromide crystal form I, which is a monohydrate.

In another embodiment, the X-ray powder diffraction pattern of the crystal form obtained using CuK_{α} radiation includes at least the characteristic peaks located at the following °2θ: 13.206±0.2, 23.995±0.2 and 24.941±0.2. In another embodiment, the X-ray powder diffraction pattern further includes the characteristic peaks located at the following °2θ: 9.222±0.2, 11.905±0.2, 19.937±0.2, 26.773±0.2 and 27.5±0.2. In another embodiment, the X-ray powder diffraction pattern further includes the characteristic peaks located at the following °2θ: 12.652±0.2, 14.702±0.2, 16.396±0.2, 16.924±0.2, 18.636±0.2, 19.148±0.2, 20.294±0.2, 21.102±0.2, 21.532±0.2, 25.492±0.2 and 33.154±0.2.

In another embodiment, the X-ray powder diffraction pattern has the following characteristic peaks:

| Angle °2θ±0.2 °2θ | Relative intensity% |
|---|---|
| 9.222 | 25.3 |
| 11.905 | 39.8 |
| 13.206 | 100 |
| 19.937 | 28 |
| 23.995 | 83.3 |
| 24.941 | 60.9 |
| 26.773 | 33.3 |
| 27.5 | 45.1 |

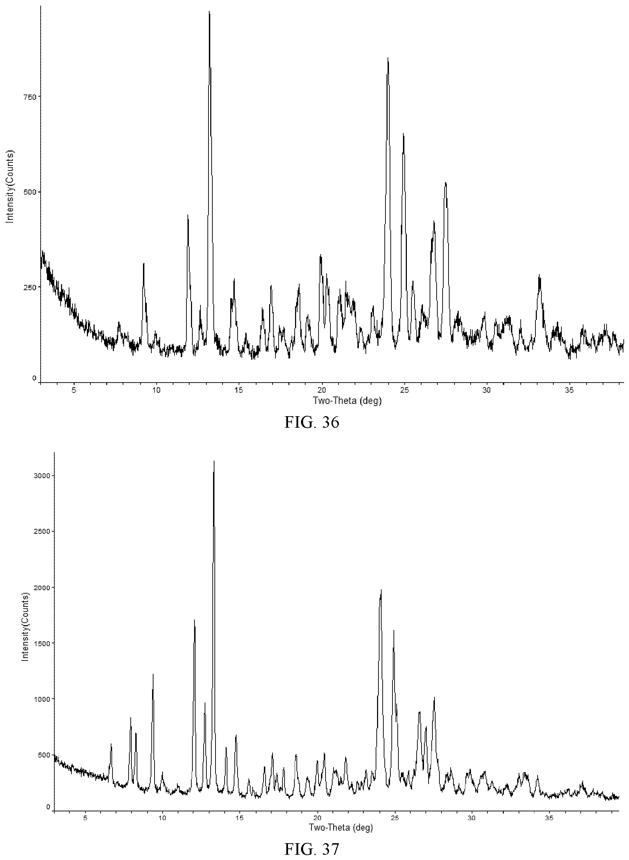

In another embodiment, the X-ray powder diffraction pattern comprises one or more peaks at the 2θ value in Table 7.6. In another embodiment, the X-ray powder diffraction pattern is substantially as shown in FIG. 36.

In another embodiment, the crystal form has a melting endothermic peak at 241±2°C in differential scanning calorimetry analysis.

In another embodiment, the crystal form has a weight loss of about 8.18% prior to 150°C in thermogravimetric analysis.

### Compound A hydrochloride crystal form I

In one embodiment, the present disclosure provides compound A hydrochloride crystal form I, which is a solvate.

In another embodiment, the X-ray powder diffraction pattern of the crystal form obtained using CuK_{α} radiation includes at least the characteristic peaks located at the following °2θ: 12.079±0.2, 13.319±0.2 and 24.093±0.2. In another embodiment, the X-ray powder diffraction pattern further includes the characteristic peaks located at the following °2θ: 9.38±0.2, 12.749±0.2, 24.92±0.2 and 27.559±0.2. In another embodiment, the X-ray powder diffraction pattern further includes the characteristic peaks located at the following °2θ: 6.699±0.2, 7.944±0.2, 8.28±0.2, 14.111±0.2, 14.758±0.2, 17.103±0.2, 18.618±0.2, 19.996±0.2, 20.449±0.2, 21.83±0.2, 25.118±0.2, 26.613±0.2 and 27.006±0.2.

In another embodiment, the X-ray powder diffraction pattern has the following characteristic peaks:

| Angle °2θ±0.2 °2θ | Relative intensity% |
|---|---|
| 9.38 | 35.3 |
| 12.079 | 52 |
| 12.749 | 26.8 |
| 13.319 | 100 |
| 24.093 | 59.9 |
| 24.92 | 47 |
| 27.559 | 25.7 |

In another embodiment, the X-ray powder diffraction pattern comprises one or more peaks at the 2θ value in Table 7.7. In another embodiment, the X-ray powder diffraction pattern is substantially as shown in FIG. 37.

In another embodiment, the crystal form has a melting endothermic peak at 221±2°C in differential scanning calorimetry analysis.

In another embodiment, the crystal form has a weight loss of about 1.67% prior to 125°C, and a weight loss of about 3.84% between 125 and 230°C in thermogravimetric analysis.

### Compound A mesylate (1: 1) crystal form I

In one embodiment, the present disclosure provides compound A mesylate (1:1) crystal form I.

In another embodiment, the X-ray powder diffraction pattern of the crystal form obtained using CuK_{α} radiation includes at least the characteristic peaks located at the following °2θ: 8.338±0.2. In another embodiment, the X-ray powder diffraction pattern further includes the characteristic peaks located at the following °2θ: 11.706±0.2, 13.932±0.2, 14.738±0.2, 18.341±0.2, 21.148±0.2, 21.588±0.2, 22.597±0.2 and 25.732±0.2. In another embodiment, the X-ray powder diffraction pattern further includes the characteristic peaks located at the following °2θ: 9.363±0.2, 10.092±0.2, 12.513±0.2, 15.053±0.2, 15.742±0.2, 19.093±0.2, 23.17±0.2, 23.716±0.2, 24.469±0.2, 24.65±0.2, 24.824±0.2, 30.238±0.2 and 32.189±0.2.

In another embodiment, the X-ray powder diffraction pattern has the following characteristic peaks:

| Angle °2θ±0.2 °2θ | Relative intensity% |
|---|---|
| 8.338 | 100 |
| 11.706 | 91.7 |
| 13.932 | 80.9 |
| 14.738 | 56.7 |
| 18.341 | 82.2 |
| 21.148 | 50.3 |
| 21.588 | 72 |
| 22.597 | 51 |
| 25.732 | 69.4 |

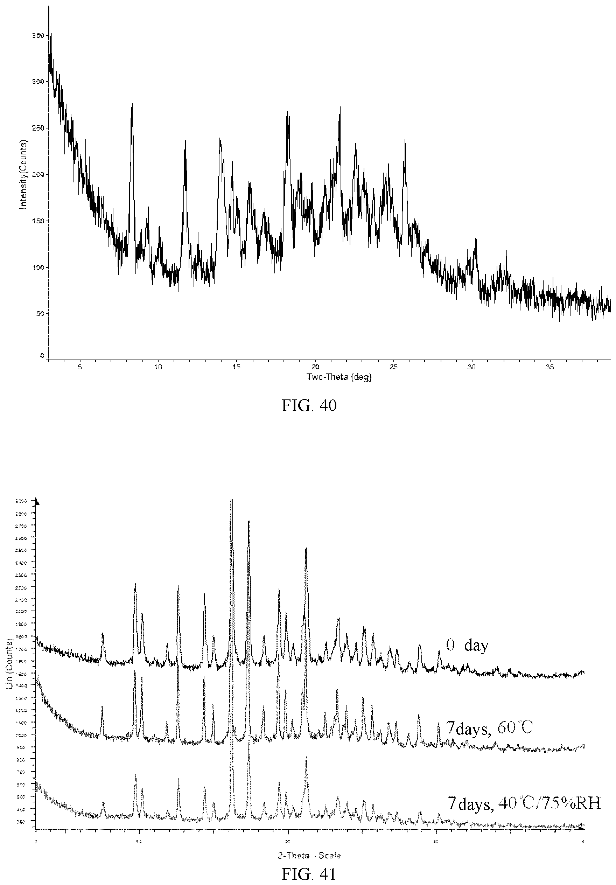

In another embodiment, the X-ray powder diffraction pattern comprises one or more peaks at the 2θ value in Table 7.8. In another embodiment, the X-ray powder diffraction pattern is substantially as shown in FIG. 40.

### Substantially pure crystal form of compound A

The present disclosure provides a method for synthesizing the crystal form of compound A in high purity and high chiral purity, which is safe and suitable for large-scale production and can be used in compositions comprising the crystal form of compound A. On the one hand, the crystal form of compound A is produced by a commercial scale method. The term "commercial scale method" refers to a method that is operated in a single batch of at least about 100 g. On the other hand, the method of the present application produces the crystal form of compound A with limited impurities at an improved yield (>90%).

The term "purity" as used herein refers to the percentage content of the crystal form of compound Abased on HPLC. The purity is based on the "organic" purity of the compound. The purity is not associated with water, solvents, metals, inorganic salts, etc. The purity of the crystal form of compound A is compared to the purity of the reference standard by comparing the area under the peak.

In one embodiment, the crystal form of compound A has a purity of not less than about 96%. In another embodiment, the crystal form of compound A has a purity of not less than about 98%. In yet another embodiment, the crystal form of compound A has a purity of not less than about 98.5%. In yet another embodiment, the crystal form of compound A has a purity of not less than about 99%. In yet another embodiment, the crystal form of compound A has a purity of not less than about 99.5%. In yet another embodiment, the crystal form of compound A has a purity of 96.0%, 96.1%, 96.2%, 96.3%, 96.4%, 96.5%, 96.6%, 96.7%, 96.8%, 96.9%, 97.0%, 97.1%, 97.2%, 97.3%, 97.4%, 97.5%, 97.6%, 97.7%, 97.8%, 97.9%, 98.0%, 98.1%, 98.2%, 98.3%, 98.4%, 98.5%, 98.6%, 98.7%, 98.8%, 98.9%, 99.0%, 99.1%, 99.2%, 99.3%, 99.4%, 99.5%, 99.6%, 99.7%, 99.8% or 99.9%.

The crystal form of compound A prepared in the present disclosure contains a chiral carbon atom which is the R-configuration. The chiral center of the crystal form of compound A is introduced from the starting material and is not involved in the subsequent steps. No racemization is observed.

The term "chiral purity" as used herein refers to the chiral purity of the crystal form of compound A as determined by chiral HPLC. Chiral purity is based on the "organic" purity of the compound. Chiral purity is not associated with water, solvents, metals, inorganic salts, etc. The chiral purity of the crystal form of compound A is compared to the chiral purity of the reference standard by comparing the area under the peak.

In one embodiment, the crystal form of compound A has a chiral purity of not less than about 96%. In another embodiment, the crystal form of compound A has a chiral purity of not less than about 98%. In yet another embodiment, the crystal form of compound A has a chiral purity of not less than about 99%. In yet another embodiment, the crystal form of compound A has a chiral purity of not less than about 99.4%. In another embodiment, the crystal form of compound A has a chiral purity of 96.0%, 96.1%, 96.2%, 96.3%, 96.4%, 96.5%, 96.6%, 96.7%, 96.8%, 96.9%, 97.0%, 97.1%, 97.2%, 97.3%, 97.4%, 97.5%, 97.6%, 97.7%, 97.8%, 97.9%, 98.0%, 98.1%, 98.2%, 98.3%, 98.4%, 98.5%, 98.6%, 98.7%, 98.8%, 98.9%, 99.0%, 99.1%, 99.2%, 99.3%, 99.4%, 99.5%, 99.6%, 99.7%, 99.8% or 99.9%.

In one embodiment, the present disclosure relates to the crystal form of compound A containing less than about 0.8% of total impurities. In another embodiment, the total impurities are less than about 0.5%. In yet another embodiment, the total impurities are less than about 0.3%. In yet another embodiment, the total impurities are less than about 0.2%.

In one embodiment, the present disclosure relates to the crystal form of compound A containing not more than about 1% of water, not more than about 0.8% of water, not more than about 0.7% of water, not more than about 0.6% of water, not more than about 0.5% of water, not more than about 0.4% of water, not more than about 0.3% of water, not more than about 0.2% of water, not more than about 0.1% of water, not more than about 0.09% of water, not more than about 0.08% of water, not more than about 0.07% of water, not more than about 0.06% of water, not more than about 0.05% of water. In another embodiment, the present disclosure relates to the crystal form of compound A containing not more than about 0.11% of water. In yet another embodiment, the present disclosure relates to the crystal form of compound A containing not more than about 0.1% of water. In yet another embodiment, the present disclosure relates to the crystal form of compound A containing not more than about 0.09% of water.

### Pharmaceutical composition

In another aspect, the present disclosure provides a pharmaceutical composition comprising (i) a pharmaceutically active ingredient: a crystal form of the free base of compound A or pharmaceutically acceptable salts thereof, (ii) a diluent, (iii) a disintegrant, (iv) a binder, and (v) a lubricant.

In a specific embodiment of the above aspect, the pharmaceutically active ingredient is the crystal form of the free base of compound A; alternatively, the crystal form is selected from compound A crystal form I, compound A crystal form II, compound A crystal form III, compound A crystal form IV, compound A crystal form V, compound A crystal form VI and compound A crystal form VII; alternatively, the crystal form is compound A crystal form I. In another specific embodiment, the pharmaceutically active ingredient is selected from compound A maleate crystal form I, compound A acetate crystal form I, compound A p-toluenesulfonate crystal form I, compound A hydrobromide crystal form I, compound A sulfate crystal form I, compound A oxalate crystal form I, compound A hydrochloride crystal form I and compound A mesylate crystal form I; alternatively, the crystal form is selected from compound A maleate crystal form I and compound A acetate crystal form I.

In another aspect, the present disclosure provides the pharmaceutical composition described above, wherein the crystal form accounts for 1-30%, alternatively, 2-20%, alternatively, 3-15%, yet alternatively, about 4%, 5%, 6%, 7%, 8%, 9% or 10% by weight of the total weight of the pharmaceutical composition, based on the weight of the free base of the compound; alternatively, wherein the amount of the crystal form in a unit dose is 1-100 mg, alternatively, 2-50 mg, alternatively, 3-40 mg, alternatively, about 5, 10, 15, 20, 25, 30, 35 or 40 mg.

In another aspect, the present disclosure provides the pharmaceutical composition described above, wherein the diluent accounts for 65-95%, alternatively, 70-90%, alternatively, about 80%, 81%, 82%, 83%, 84%, 85%, 86%, 87%, 88%, 89% or 90% by weight of the total weight of the pharmaceutical composition; alternatively, wherein the amount of the diluent in a unit dose is 50-380 mg, alternatively, 60-360 mg, alternatively, 70-350 mg, such as, about 70 mg or 350 mg.

In another aspect, the present disclosure provides the pharmaceutical composition described above, wherein the diluent is selected from the group consisting of microcrystalline cellulose, anhydrous calcium hydrogen phosphate, and mannitol, e.g., microcrystalline cellulose 102, mannitol 100SD, and mannitol 50C, and mixtures thereof; alternatively, where both of microcrystalline cellulose 102 and mannitol 50C are present, the weight ratio of microcrystalline cellulose 102 to mannitol 50C is 5:1 to 1:5, alternatively 3:1 to 1:2, alternatively about 2:1.

In another aspect, the present disclosure provides the pharmaceutical composition described above, wherein the disintegrant accounts for 1-5%, alternatively, 2-4%, alternatively, about 2%, 2.5%, 3%, 3.5% or 4% by weight of the total weight of the pharmaceutical composition; alternatively, wherein the amount of the disintegrant in a unit dose is 1-20 mg, alternatively, 2-16 mg, alternatively, about 2, 2.5, 3, 6, 9 or 12 mg.

In another aspect, the present disclosure provides the pharmaceutical composition described above, wherein the disintegrant is croscarmellose sodium or crospovidone XL-10, alternatively croscarmellose sodium.

In another aspect, the present disclosure provides the pharmaceutical composition described above, wherein the binder accounts for 1-5%, alternatively, 2-4%, alternatively, about 2%, 2.5%, 3%, 3.5% or 4% by weight of the total weight of the pharmaceutical composition; alternatively, wherein the amount of binder in a unit dose is 1-20 mg, alternatively, 2-16 mg, alternatively, about 2, 2.5, 3, 6, 9 or 12 mg.

In another aspect, the present disclosure provides the pharmaceutical composition described above, wherein the binder is hydroxypropylcellulose EXF or povidone K30, alternatively hydroxypropylcellulose EXF.

In another aspect, the present disclosure provides the pharmaceutical composition described above, wherein the lubricant accounts for 0.1-5%, alternatively, 0.5-2%, alternatively, about 1% by weight of the total weight of the pharmaceutical composition; alternatively, wherein the amount of the lubricant in a unit dose is 0.1-20 mg, alternatively, 0.5-8 mg, alternatively, about 0.5, 1, 2, 3, 4, 5, 6, 7 or 8 mg.

In another aspect, the present disclosure provides the pharmaceutical composition described above, wherein the lubricant is magnesium stearate or sodium stearyl fumarate PRUV, alternatively magnesium stearate.

In another aspect, the present disclosure provides the pharmaceutical composition described above, comprising the following ingredients:
(i) 1-30% by weight of compound A crystal form I,
(ii) 65-95% by weight of microcrystalline cellulose 102 and mannitol 50C (2:1, by weight),
(iii) 2-4% by weight of croscarmellose sodium,
(iv) 2-4% by weight of hydroxypropylcellulose EXF, and
(v) 0.1-5% by weight of magnesium stearate.

In another aspect, the present disclosure provides the pharmaceutical composition described above, wherein the unit dose comprises the following components:
(i) about 5 mg of compound A crystal form I,
(ii) about 45 mg of microcrystalline cellulose 102 and about 25 mg of mannitol 50C,
(iii) about 2.5 mg of croscarmellose sodium,
(iv) about 2.5 mg of hydroxypropylcellulose EXF, and
(v) about 1 mg of magnesium stearate.

In another aspect, the present disclosure provides the pharmaceutical composition described above, wherein the unit dose comprises the following components:
(i) about 25 mg of compound A crystal form VI,
(ii) about 230 mg of lactose monohydrate and about 120 mg of microcrystalline cellulose,
(iii) about 12 mg of croscarmellose sodium,
(iv) about 12 mg of hydroxypropylcellulose EXF, and
(v) about 4 mg of magnesium stearate.

In another aspect, the present disclosure provides the pharmaceutical composition described above, which is a tablet, alternatively a coated tablet; alternatively, the coating agent is Opadry II 85F620077.

### Pharmacology and efficacy

In another aspect, the present disclosure provides a method of treating abnormal cell growth in a subject, comprising administering to the subject an effective amount of free base of compound A, or a pharmaceutically acceptable salt thereof, and a variety of crystal forms thereof.

In one embodiment, the abnormal cell growth is cancer. In another embodiment, the abnormal cell growth is a cancer mediated by anaplastic lymphoma kinase (ALK). In another embodiment, the abnormal cell growth is a genetically altered ALK kinase. In another embodiment, the mutation is L1196M. In another embodiment, the mutation is G1202R. In another embodiment, the mutation is L1196M/G1202R.

In another embodiment, the abnormal cell growth is cancer mediated by ROS1 kinase. In another embodiment, the ROS1 kinase is a genetically altered ROS1 kinase. In another embodiment, the mutation is G2032R.

In another embodiment, the abnormal cell growth is cancer selected from the group consisting of lung cancer, bone cancer, pancreatic cancer, skin cancer, head and neck cancer, skin or intraocular melanoma, uterine cancer, ovarian cancer, rectal cancer, anus cancer, stomach cancer, colon cancer, breast cancer, fallopian tube cancer, endometrial cancer, cervical cancer, vaginal cancer, vulvar cancer, Hodgkin's disease, esophagus cancer, small bowel cancer, endocrine system cancer, thyroid cancer, parathyroid cancer, adrenal gland cancer, soft tissue sarcoma, urethral cancer, penile cancer, prostate cancer, chronic or acute leukemia, lymphocytic lymphoma, bladder cancer, kidney cancer or ureter cancer, renal cell cancer, renal pelvis cancer, central nervous system (CNS) cancer, primary central nervous system lymphoma, spinal cord axial cancer, brainstem glioma, pituitary adenoma, or a combination thereof.

In another embodiment, the abnormal cell growth is NSCLC. In another embodiment, NSCLC is mediated by ALK and/or ROS1. In another embodiment, the NSCLC is NSCLC mediated by genetically altered ALK and/or genetically altered ROS1.

### Example

### Abbreviations

SGF: Simulated gastric fluid
FaSSIF: Simulated intestinal fluid in fasted state
FeSSIF: Simulated intestinal fluid in fed state
HBr: Hydrobromic acid
HCl: Hydrochloric acid
H₂SO₄: Sulfuric acid
PTSA: p-Toluenesulfonic acid
CH₃SO₃H: Methanesulfonic acid
PhSOsH: Benzenesulfonic acid
Oxalic acid: Ethanedioic acid
Maleic acid: Toxilic acid
MeOH: Methanol
EtOH: Ethanol
IPA: Isopropyl alcohol
IBA: Isobutanol
MEK: Methyl ethyl ketone
THF: Tetrahydrofuran
ACN: Acetonitrile
MTBE: Methyl tert-butyl ether
EtOAc: Ethyl acetate
Acetone: Dimethyl ketone
IPrOAc: Isopropyl acetate
H₂O: Water
hr: hour
min: minute
µL: microliter

### General method 1. X-ray powder diffraction (XRPD)

The solid samples obtained from the experiments were analyzed with a D8 advance powder X-ray diffraction analyzer (Bruker) equipped with a LynxEye detector. The samples were tested by D8 advance powder X-ray diffraction analyzer (Bruker) with a 2θ scanning angle ranged from 3° to 40°, a scanning step length of 0.02°, and a scanning speed of 0.3 sec/step. The light tube voltage and light tube current were 40 KV and 40 mA, respectively, when the samples were tested.

### General method 2. Polarizing microscope analysis (PLM)

The instrument model used for PLM analysis was an ECLIPSE LV100POL polarizing microscope (Nikon, Japan).

### General method 3. Nuclear magnetic resonance hydrogen spectroscopy analysis (¹H NMR)

The chemical structure of a solid sample was confirmed by ¹H NMR. ¹H NMR analysis was performed using a Bruker Advance 300 equipped with a B-ACS 120 autosampling system.

### General method 4. Differential scanning calorimetry analysis (DSC)

The instrument model for differential scanning calorimetry analysis was Discovery DSC 250 (TA, USA). About 2 mg of a sample was weighed and placed in a DSC sample pot, and the pot was punctured. The sample was equilibrated at 25 °C, and then heated to 300°C at a ramp rate of 10°C/min.

### General method 5. Thermogravimetric analysis (TGA)

The thermogravimetric analyzer model was Discovery TGA 55 (TA, USA). The sample was placed in an open aluminum sample pot that had been equilibrated and the mass was weighed automatically in a TGA heating oven. The sample was then heated to 300°C at a ramp rate of 10°C/min.

### General method 6. Dynamic moisture adsorption and desorption analysis (DVS)

The samples were tested for hygroscopicity using DVS Intrinsic (SMS, UK). 30-50 mg of a sample was placed into a sample pan, and the change of sample mass with humidity at 25 °C was recorded. The instrument parameters were:

| | |
|---|---|
| Equilibration: | 60 min |
| RH (%) measuring point: | |
| Adsorption: | 0, 10, 20, 30, 40, 50, 60, 70, 80, 90 |
| Desorption: | 90, 80, 70, 60, 50, 40, 30, 20, 10, 0 |

After the hygroscopicity test was completed, the crystal form of the sample was tested by XRPD.

### Example 1 Preparation of Compound A

The following route was used for the synthesis:

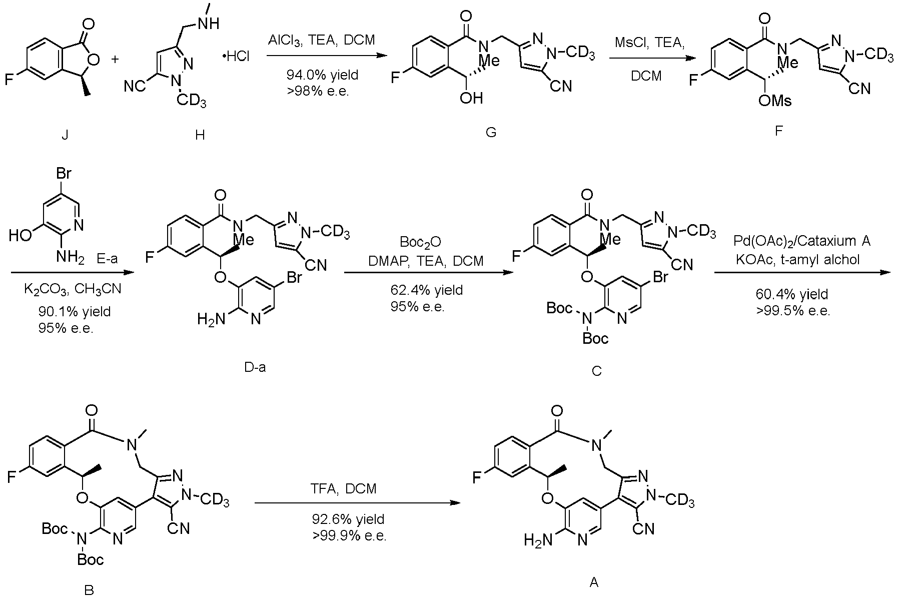

### Step 1: Synthesis of compound G.

To a 250 mL three-necked flask equipped with magnetic stirring were added compound J (7.0 g, 42.2 mmol) and anhydrous dichloromethane (120 mL), and the mixture was stirred until the solution became clear. Compound H (8.77 g, 46.4 mmol) and then triethylamine (4.69 g, 46.4 mmol) were successively added. The mixture was stirred at room temperature under nitrogen atmosphere for 30 minutes to give a pale yellow clear solution for further use.

To another 500 mL three-necked flask equipped with magnetic stirring was added anhydrous aluminum chloride (6.17 g, 46.4 mmol), and the system was evacuated with suction and purged with nitrogen gas. Anhydrous dichloromethane (60 mL) was added under nitrogen atmosphere, and the mixture was cooled to 0°C. in an ice-water bath. Triethylamine (6.39 g, 63.3 mmol) was slowly added dropwise. After the addition was completed, the mixture was stirred at this temperature for 10 minutes. The above solution of raw materials in dichloromethane was slowly added dropwise over 30 minutes. The mixture was reacted with stirring at this temperature for another 2 hours. By TLC (PE:EA=1:1) and HPLC monitoring, the reaction was completed. The reaction was quenched by adding water (200 mL). The organic phase was separated, and the aqueous layer was extracted with dichloromethane (100 mL×2). The organic phases were combined, washed successively with water (100 mL) and then saturated brine (100 mL), dried over anhydrous sodium sulfate, and filtered. The filtrate was concentrated to dryness under reduced pressure to give 12.66 g of a yellow oil in a yield of 94.0% and a purity (HPLC) of >90% (ee>98%). The intermediate is unstable at room temperature, and thus should be directly taken into the next step or stored in a refrigerator at -20°C. LC-MS(APCI): m/z=320.1(M+1)⁺. ¹H NMR(300 MHz, CDCl₃) δ (ppm): 7.92-7.89(m, 1H), 7.27-7.17(m, 2H), 7.03-6.97(m, 1H), 6.84(s, 1H), 4.92(q, J=6.3 Hz, 1H), 4.83(s, 2H), 2.89(s, 3H), 1.50(d, J=6.3 Hz, 3H).

### Step 2: Synthesis of compound F.

To a 250 mL three-necked flask equipped with magnetic stirring were added compound G (12.6 g, 39.5 mmol) and anhydrous dichloromethane (120 mL), and the mixture was stirred until the solution became clear. The mixture was cooled in an ice-water bath. Triethylamine (7.98 g, 79.5 mmol) was added, and then methylsulfonyl chloride (5.85 g, 51.4 mmol) was slowly added dropwise. After the addition was completed, the ice bath was removed, and the mixture was reacted with stirring at room temperature under nitrogen atmosphere for 1 hour. TLC (DCM:MeOH=20:1) showed that the reaction was completed. The reaction was quenched by adding ice-water (100 mL). The organic phase was separated, and the aqueous layer was extracted with dichloromethane (50 mL×2). The organic phases were combined, washed successively with water (50 mL) and then saturated brine (50 mL), dried over anhydrous sodium sulfate, and filtered. The filtrate was concentrated to dryness under reduced pressure, and then dissolved in anhydrous acetonitrile (50 mL) for further use.

### Step 3: Synthesis of compound D-a.

To another 250 mL three-necked flask equipped with magnetic stirring were added compound E-a (11.2 g, 59.3 mmol) and acetonitrile (200 mL), and cesium carbonate (25.7 g, 79.0 mmol) was added with stirring. The mixture was heated to 50°C. under nitrogen atmosphere, and the mixture was stirred at this temperature for 30 min. The above solution of compound F in acetonitrile was slowly added dropwise at 50 °C over 10 minutes. After the dropwise addition was completed, the mixture was reacted with stirring at this temperature for 2 hours. By TLC (DCM:MeOH=20:1) and HPLC monitoring, the reaction was completed. After cooling to room temperature, the reaction was quenched by adding water (200 mL). The reaction solution was diluted with ethyl acetate (300 mL), stirred for 5 minutes, and then filtered through Celite to remove insoluble solids. The filter cake was washed with ethyl acetate (50 mL). The organic layer was separated from the filtrate, and the aqueous phase was extracted with ethyl acetate (60 mL×2). The organic phases were combined, washed with a saturated aqueous solution of sodium carbonate (100 mL×3) and then saturated brine (60 mL), dried over anhydrous sodium sulfate, and filtered. The filtrate was concentrated to dryness under reduced pressure to give 17.5 g of a brown solid in a yield of 90.1% and a purity (HPLC) of >85% (ee>95%). LC-MS(APCI): m/z=390.1(M+1)⁺.

### Step 4: Synthesis of compound C.

To a 250 mL single-necked flask equipped with magnetic stirring were added compound D-a (17.5 g, 35.8 mmol) and dichloromethane (200 mL), and the mixture was stirred until the solution became clear. Triethylamine (14.5 g, 143.2 mmol) and then DMAP (850 mg, 7.2 mmol) were successively added. Boc₂O (23.4 g, 107.4 mmol) was slowly added dropwise, and the mixture was reacted with stirring at room temperature under nitrogen atmosphere overnight. By TLC (DCM:MeOH=20:1) and HPLC monitoring, the reaction was completed. The reaction solution was evaporated under reduced pressure to remove the solvent, and the residue was purified by silica gel column chromatography (EA/PE=0-35%) to give 15.4 g of a white solid in a yield of 62.4% and a purity (HPLC) of >95% (ee>95%). LC-MS(APCI): m/z=590.1(M+1-100)⁺. ¹H NMR(300 MHz, CDCl₃) (δ/ppm): 8.06(d, J=1.8 Hz, 1H), 7.53-7.48(m, 1H), 7.24-7.20(m, 2H), 7.04-6.98(m, 1H), 6.81(s, 1H), 5.66-5.59(m, 1H), 4.89-4.69(m, 2H), 2.97(s, 3H), 1.58(d, J=6.0 Hz, 3H), 1.47(s, 18H).

### Step 5: Synthesis of compound B.

To a 500 mL single-necked flask equipped with magnetic stirring were added compound C (15.4 g, 22.3 mmol) and 2-methyl-2-butanol (300 mL), and the mixture was stirred until the solution became clear. Potassium acetate (6.56 g, 66.9 mmol) was added. The system was evacuated with suction and purged with nitrogen gas three times. Palladium acetate (0.75 g, 3.35 mmol) and n-butylbis(1-adamantyl)phosphine (1.60 g, 4.46 mmol) were quickly added. The system was evacuated with suction and purged with nitrogen gas three times. The reaction solution was heated to 110°C under nitrogen atmosphere, and reacted with stirring at this temperature overnight. By TLC (PE:EA=1:1) and HPLC monitoring, the reaction was completed. The reaction solution was cooled to room temperature, diluted with dichloromethane (300 mL), and filtered through Celite to remove insoluble solids. The filter cake was washed with dichloromethane (50 mL). The filtrates were combined, and concentrated to dryness under reduced pressure. To the residue was added acetonitrile (150 mL), and the mixture was heated to reflux for 1 hour. The oil bath was removed, and the mixture was allowed to slowly cool to room temperature. A large amount of a white solid precipitated out, and the precipitated solid was filtered. The filter cake was washed with acetonitrile (10 mL), and dried to give 8.2 g of a white solids in a yield of 60.4% and a purity (HPLC) of >99.5% (ee>99.9%). LC-MS(APCI): m/z=510.1(M+1-100)⁺. ¹H NMR(300 MHz, CDCl₃) (δ/ppm): 8.22(d, J=1.8 Hz, 1H), 7.29-7.25(m, 1H), 7.22-7.16(m, 2H), 7.03-6.96(m, 1H), 5.76-5.70(m, 1H), 4.42(q, J=14.1 Hz, 2H), 3.15(s, 3H), 1.76(d, J=6.0 Hz, 3H), 1.44(s, 18H).

### Step 6: Synthesis of compound A.

To a 250 mL single-necked flask equipped with magnetic stirring were added compound B (8.2 g, 13.5 mmol) and dichloromethane (100 mL), and the mixture was stirred until the solution became clear. The mixture was cooled in an ice-water bath, and trifluoroacetic acid (20 mL) was slowly added dropwise. After the addition was completed, the ice bath was removed, and the mixture was reacted with stirring at room temperature for 2 hours. By TLC (DCM:MeOH=20:1) and HPLC monitoring, the reaction was completed. The reaction solution was evaporated under reduced pressure to remove the organic solvent. Dichloromethane (100 mL) and a saturated aqueous solution of sodium bicarbonate (60 mL) were added under cooling, and the mixture was stirred for 10 minutes. The organic phase was separated, and the aqueous layer was extracted with dichloromethane (50 mL×2). The organic phases were combined, washed successively with water (30 mL) and then saturated brine (40 mL), dried over anhydrous sodium sulfate, and filtered. The filtrate was concentrated under reduced pressure to give 5.1 g of an amorphous white solid in a yield of 92.6% and a purity (HPLC) of >99.5% (ee>99.9%). LC-MS(APCI): m/z=410.2(M+1)⁺. ¹H NMR(300 MHz, CDCl₃) (δ)ppm 7.79(d, J=1.8 Hz, 1H), 7.31-7.27(m, 1H), 7.23-7.19 (m, 1H),7.06-6.97(m, 1H), 6.87(d, J=1.8 Hz, 1H), 5.75-5.70(m, 1H), 5.09(br s, 2H), 4.40(q, J = 14.1 Hz, 2H), 3.12(s, 3H), 1.78(d, J = 6.6 Hz, 3H).

### Example 2 Preparation of various crystal forms of compound A

In the screening of various crystal forms of the free base of compound A, compound A was used as the starting material to screen various crystal forms of the free base of compound A by means of volatilization at room temperature, stirring in a suspension and precipitation in an anti-solvent. Four anhydrous crystal forms (crystal form I, crystal form IV, crystal form V, crystal form VI) and three solvates (crystal form II, crystal form III and crystal form VII) were found.

### Example 2.1 Treatment of starting material and preparation of compound A crystal form I

To a certain amount of the starting material compound A was added isobutanol as a solvent. The suspension was slurried at room temperature for one day, and then filtered by suction. The solid was dried at 50 °C under vacuum overnight to afford 1 g of compound A crystal form I, which was an anhydrous crystal form.

### Example 2.2 Screening of 13 × 13 (1: 1) binary solvents in a 96-well plate

About 30 mg of compound A crystal form I was added to a vial (8 mL, 13 vials total) and 3 mL of the corresponding solvent (methanol, ethanol, isopropanol, isobutanol, butanone, tetrahydrofuran, acetonitrile, methyl tert-butyl ether, acetone, water, dichloromethane, ethyl acetate and isopropyl acetate, respectively) was added gradually. The mixture was stirred at room temperature for a period of time, and then filtered. The filtrate was set aside for use. The filtrate was used for the screening of binary solvents in a 96-well plate. The above corresponding filtrates were distributed two by two in a 96-well plate in a volume of 100 µL for each filtrate. The 96-well plate was sealed with a sealing film and allowed to volatilize to dryness in a fume hood at room temperature. Information of the 13 solvents in the 96-well plate is given in Table 2.2.

**Table 2.2: Information of 13 × 13 solvents in the 96-well plate**

| ID | MeOH | EtOH | IPA | IBA | MEK | THF | ACN | MTBE | Acetone | H₂O | DCM | EtOAc | IPrOAc |
|---|---|---|---|---|---|---|---|---|---|---|---|---|---|
| MeOH | AM | AM | AM | AM | CR* | AM | AM | AM | AM | CR* | AM | AM | AM* |
| EtOH | / | N/A | GL | CR | CR | N/A | GL | GL | GL | GL | AM | N/A | N/A |
| IPA | / | / | GL | CR* | CR* | N/A | N/A | N/A | GL | GL | GL | GL | GL |
| IBA | / | / | / | N/A | CR* | CR* | N/A | N/A | N/A | N/A | N/A | N/A | N/A |
| MEK | / | / | / | / | N/A | CR* | CR* | N/A | CR* | N/A | CR* | CR* | CR* |
| THF | / | / | / | / | / | N/A | N/A | GL | GL | N/A | AM* | GL | GL |
| ACN | / | / | / | / | / | / | GL | GL | GL | N/A | N/A | GL | GL |
| MTBE | / | / | / | / | / | / | / | GL | N/A | GL | N/A | GL | GL |
| Acetone | / | / | / | / | / | / | / | / | GL | GL | N/A | GL | GL |
| H₂O | / | / | / | / | / | / | / | / | / | GL | N/A | N/A | N/A |
| DCM | / | / | / | / | / | / | / | / | / | / | AM* | N/A | AM* |
| EtOAc | / | / | / | / | / | / | / | / | / | / | / | GL | GL |
| IPrOAc | / | / | / | / | / | / | / | / | / | / | / | / | GL |

| | | | | | | | | | | | | | |
|---|---|---|---|---|---|---|---|---|---|---|---|---|---|
| Note: AM represents amorphous; CR represents crystalline; GL represents glassy. * indicates that the results of the samples in this well as shown in the Table are XRPD results while the results of the samples in other wells are PLM results. | | | | | | | | | | | | | |

Solids precipitated in a 96-well plate were tested for PLM and XRPD. The results showed that a new crystal form was obtained, named compound A crystal form II. The crystal form II was obtained in butanone/isopropanol, butanone/tetrahydrofuran, butanone/acetonitrile, butanone/acetone, butanone/dichloromethane, butanone/ethyl acetate, and butanone/isopropyl acetate. Amorphous or poorly crystallized solids were obtained in other solvents.

### Example 2.3 Research of evaporating crystallization in 13 single solvents

The remaining 13 single filtrates for spreading the 96-well plate in Example 2.2 were placed in a fume hood and allowed to volatilize to dryness at room temperature for 3 days. The samples were dried at 50 °C under vacuum overnight to obtain two new crystal forms. Evaporation in a single solvent of butanone led to a new crystal form, named compound A crystal form III. Evaporation in a single solvent of methanol led to another new crystal form, named compound A crystal form IV Evaporation in other solvents led to amorphous or poorly crystallized solids.

### Example 2.4 Research of slurrying in a suspension at room temperature

To a certain amount of compound A crystal form I were added different solvents. The suspension was slurried at room temperature for one day and then filtered by suction. The samples were dried at 50 °C under vacuum overnight and characterized by XRPD. The test conditions and results are shown in Table 2.4.

**Table 2.4 The test conditions and results of slurrying in a suspension at room temperature**

| No. | Compound A crystal form I (mg) | Solvent | Volume of solvent (µL) | Volume ratio (V:V) | Result |
|---|---|---|---|---|---|
| 1 | 33.2 | IPrOAc | 500 | / | Crystal form I |
| 2 | 33.3 | EtOH | 500 | / | Crystal form I |
| 3 | 31.6 | MTBE/MeOH | 500 | 9:1 | Crystal form I |
| 4 | 31.2 | IPA/EtOAc | 500 | 4:1 | N/A |
| 5 | 33.0 | IBA/EtOAc | 500 | 4:1 | N/A |
| 6 | 35.0 | H₂O/MeOH | 500 | 9:1 | Crystal form V |
| 7 | 32.3 | MeOH | 500 | / | Crystal form VI |
| 8 | 32.3 | H₂O/ACN | 500 | 9:1 | Crystal form VII |

The results showed that in addition to crystal form I obtained in isopropyl acetate, ethanol and MTBE/MeOH (9:1), three new crystal forms were obtained, named crystal form V, crystal form VI and crystal form VII. In the three new crystal forms, crystal form V was obtained in a suspension of water and methanol; crystal form VI was obtained in a suspension of methanol; and crystal form VII was obtained in a suspension of water and acetonitrile.

### Example 2.5 Research of slurrying in a suspension at 50 °C

A suspension of 30 mg of compound A crystal form I in 500 µL of poor solvent was prepared. The suspension was slurried at 50 °C for one day and then filtered by suction. The samples were dried at 50 °C under vacuum overnight and characterized by XRPD. The test conditions and results are shown in Table 2.5.

**Table 2.5 The test conditions and results of slurrying in a suspension at 50 °C**

| No. | Compound A crystal form I (mg) | Solvent | Volume of solvent (µL) | Result |
|---|---|---|---|---|
| 1 | 30.0 | H₂O | 500 | Crystal form I |
| 2 | 30.0 | Heptane | 500 | Crystal form I |
| 3 | 30.0 | IBA | 500 | Crystal form I |
| 4 | 30.0 | IPA | 500 | Crystal form I |
| 5 | 30.0 | MTBE | 500 | Crystal form I |

The results showed that crystal form I was obtained in all solvent systems.

### Example 2.6 Research of precipitation in an anti-solvent

30 mg of compound A crystal form I was added in a 200 µL of good solvent and the mixture was stirred at room temperature. Anti-solvents were added respectively for precipitation testing in an anti-solvent. The resulting solids were subjected to XRPD characterization. The test conditions and results are shown in Table 2.6.

**Table 2.6 The test conditions and results of precipitation in an anti-solvent**

| No. | Compound A crystal form I (mg) | Good solvent/volume | Anti -solvent/volume | Result |
|---|---|---|---|---|
| 1 | 34.1 | Acetone (200 µL) | MTBE (6.2µL) | N/A |
| 2 | 30.8 | THF (200 µL) | MTBE (5 µL) | N/A |
| 3 | 33.2 | ACN (200 µL) | MTBE (4 µL) | N/A |
| 4 | 34.1 | EtOAc (200 µL) | MTBE (2 µL) | Crystal form I |
| 5 | 33.7 | Acetone (200 µL) | IPA (4 µL) | N/A |
| 6 | 33.3 | THF (200 µL) | IPA (4 µL) | N/A |
| 7 | 31.1 | ACN (200 µL) | IPA (4 µL) | N/A |
| 8 | 34.9 | EtOAc (200 µL) | IPA (4 µL) | N/A |

The results showed that only ethyl acetate/MTBE led to crystal form I, and other solvent systems failed to lead to solid precipitation.

### Example 3 Characterization of various crystal forms of the free base of compound A

### Example 3.1 Characterization of compound A crystal form I

Compound A crystal form I was prepared according to the method in Example 2.1 and characterized by XRPD, PLM, DSC, TGA, DVS, NMR, IR, UV and MS. The specific results are as follows:

### XRPD analysis

FIG. 1 shows the XRPD data of compound A crystal form I acquired according to General method 1. A list of XRPD peaks at diffraction angles 2θ° (°2θ)±0.2 °2θ and relative intensities thereof are provided in Table 3.1. The XRPD results indicated that the crystallinity of crystal form I was good.

**Table 3.1: XRPD peak list (2θ°) of compound A crystal form I**

| Peak | Angle °2θ±0.2 °2θ | Relative intensity |
|---|---|---|
| 1 | 7.435 | 13.5 |
| 2 | 9.637 | 38.2 |
| 3 | 10.110 | 23.5 |
| 4 | 11.808 | 10.1 |
| 5 | 12.555 | 38.1 |
| 6 | 14.343 | 34.5 |
| 7 | 14.922 | 14.5 |
| 8 | 16.175 | 100.0 |
| 9 | 17.299 | 68.5 |
| 10 | 18.359 | 13.7 |
| 11 | 19.366 | 34.7 |
| 12 | 19.859 | 23.6 |
| 13 | 20.332 | 7.9 |
| 14 | 21.218 | 54.1 |
| 15 | 22.554 | 8.9 |
| 16 | 23.147 | 8.9 |
| 17 | 23.401 | 19.4 |
| 18 | 23.765 | 6.4 |
| 19 | 23.939 | 11.9 |
| 20 | 24.584 | 8.2 |
| 21 | 25.117 | 16.9 |
| 22 | 25.727 | 14.1 |
| 23 | 26.241 | 6.1 |
| 24 | 26.831 | 10.9 |
| 25 | 27.304 | 9.4 |
| 26 | 28.154 | 4.6 |
| 27 | 28.862 | 12.3 |
| 28 | 29.213 | 3.5 |
| 29 | 30.220 | 9.9 |
| 30 | 30.809 | 4.3 |
| 31 | 31.831 | 4.2 |
| 32 | 32.125 | 5.0 |
| 33 | 32.611 | 2.9 |
| 34 | 34.115 | 4.3 |
| 35 | 35.574 | 2.4 |
| 36 | 38.568 | 1.9 |

### PLM analysis

Compound A crystal form I was subjected to PLM analysis by General method 2. The PLM results indicated that the crystal form I was a granular crystal.

### DSC and TGA thermal analysis

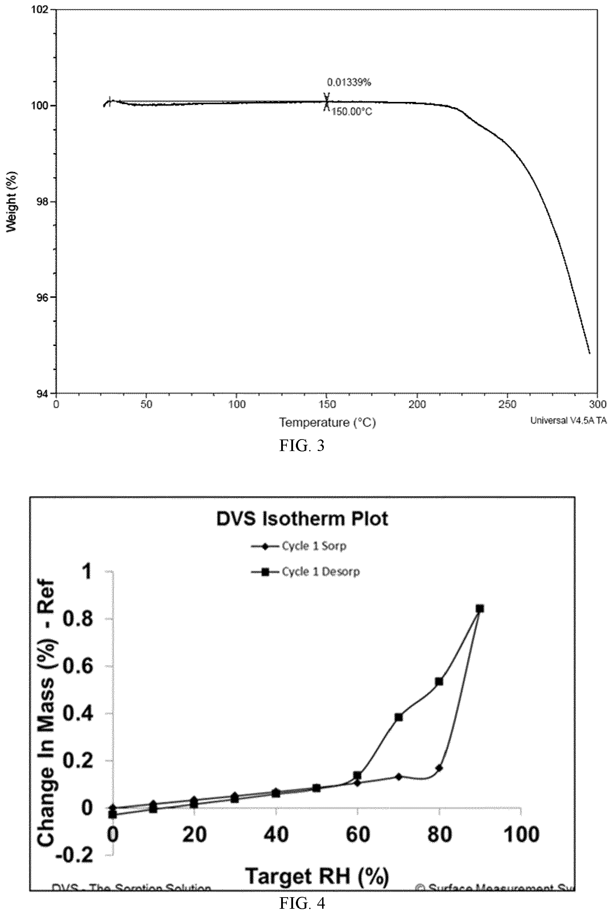

FIGs. 2 and 3 show DSC and TGA curves of compound A crystal form I acquired by General methods 4 and 5, respectively. The DSC results showed a narrow melting endothermic peak at a peak temperature of about 232.48 °C with an onset temperature of about 231.78 °C. The TGA results showed a weight loss of up to 0.01339% at 150 °C. DSC and TGA analyses indicated that the sample was an anhydrous crystal form.

### DVS analysis

FIG. 4 shows the DVS curve of compound A crystal form I acquired by General method 6. The DVS results showed a weight gain of 0.17% as the humidity was increased from 0% RH to 80% RH.

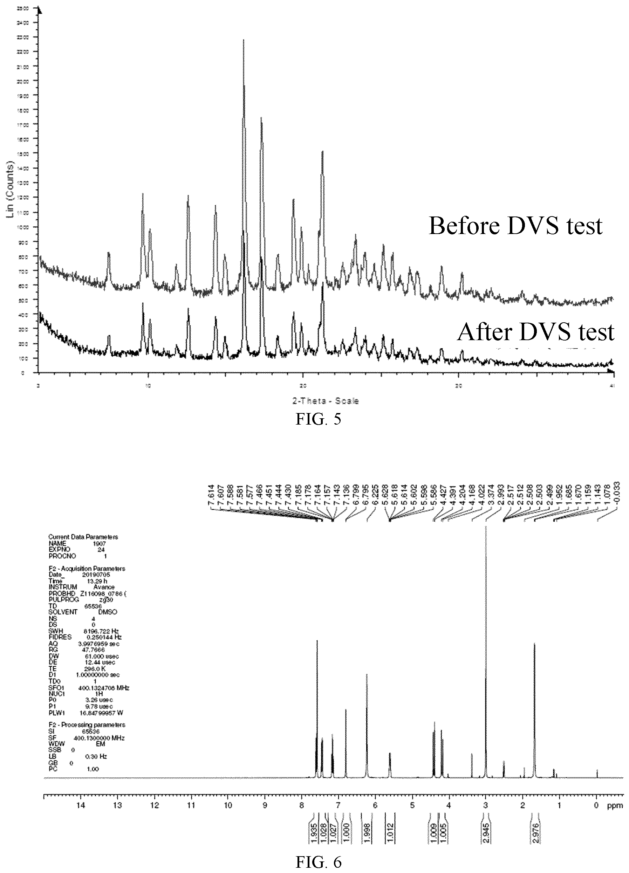

The crystal form of the samples was not changed before and after the DVS test, as shown in FIG 5.

### NMR analysis

Instrument and equipment: Bruker AVANCE III 400 MHz Nuclear Magnetic Resonance Spectrometer

### Solvent: DMSO-d₆

73.66 mg of compound A crystal form I was accurately weighed, and completely dissolved by adding 0.6 mL of DMSO-*d₆*. The solution was transferred to a NMR tube for ¹H-NMR, ¹³C-NMR, DEPT, ¹⁹F-NMR, HSQC, HMBC and COSY tests.

36.81 mg of compound A crystal form I was accurately weighed, and completely dissolved by adding 0.6 mL of DMSO-*d₆*. Additional 50 µL of DMSO-*d₆* was then added. The solution was mixed well and transferred to a NMR tube for D-NMR test.

The NMR atomic distribution structure formula of compound A is shown as follows:

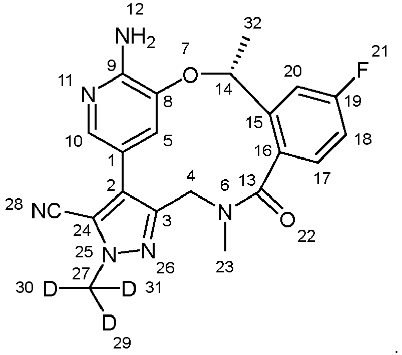

Critical HMBC and COSY related structures of compound A are shown as follows:

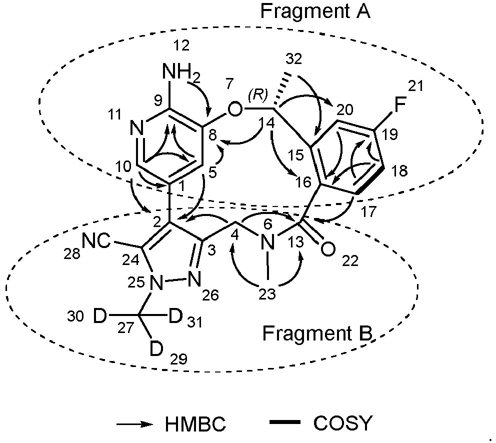

### ¹H-NMR

FIG. 6 shows the ¹H NMR spectrum of compound A crystal form I. Table 3.2 shows the test results of the ¹H NMR spectrum of compound A crystal form I.

**Table 3.2 The test results of the nuclear magnetic resonance hydrogen spectrum of compound A crystal form I**

| Chemical shift (ppm) | Peak shape | Number of protons | Assignment |
|---|---|---|---|
| 7.61 | Overlapping | 1H | H-20 |
| 7.58 | Overlapping | 1H | H-10 |
| 7.45 | dd (J = 8.8, 6.0 Hz) | 1H | H-17 |
| 7.16 | td (J = 8.8, 2.8 Hz) | 1H | H-18 |
| 6.80 | d (J = 1.6 Hz) | 1H | H-5 |
| 6.23 | br s | 2H | NH-12 |
| 5.61 | m | 1H | H-14 |
| 4.41 | d (J = 14.4 Hz) | 1H | H-4a |
| 4.19 | d (J = 14.4 Hz) | 1H | H-4b |
| 2.99 | s | 3H | H-23 |
| 1.68 | d (J = 6.0 Hz) | 3H | H-32 |

The results show that the hydrogen spectrum has 16 hydrogen signals, which in combination with HSQC, include 6 methyl hydrogens, 2 methylene hydrogens, 6 methenyl hydrogens, and 2 active hydrogens. The hydrogen signal with a chemical shift at δ_{H} 6.23 (br s, 2H) has no HSQC correlation, which is assigned to NH-12 based on HMBC correlation with C-8; the aromatic hydrogen signal with a chemical shift at δ_{H} 7.61 (Overlapping, 1H) is assigned to H-20 based on HMBC correlation with C-14, C-15, C-16, C-18, and C-19; δ_{H} 7.58 (Overlapping, 1H) has HMBC correlation with C-1, C-2, C-5, C-8, and C-9, which is assigned to H-10; δ_{H} 7.45 (dd, J = 8.8, 6.0 Hz, 1H) has COSY correlation with δ_{H} 7.16 (td, J = 8.8, 2.8 Hz, 1H), which in combination with HMBC correlation, is assigned to H-17 and H-18, respectively; the chemical shift at δ_{H} 6.80 (d, J = 1.6 Hz, 1H) is assigned to H-5 based on the HMBC correlations with C-1, C-2, C-8, C-9 and C-10; δ_{H} 5.61 (m, 1H) has COSY correlation with the hydrogen signal at δ_{H} 1.68 (J = 6.0 Hz, 3H), which in combination with the HMBC correlation, is assigned to H-14 and H-32, respectively; the methylene hydrogen signals with chemical shifts at δ_{H} 4.41 (d, J = 14.4 Hz, 1H) and δ_{H} 4.19 (d, J = 14.4 Hz, 1H) have HMBC correlations with C-2, C-3, C-13 and C-23, which are assigned to H-4a and H-4b, respectively; the methyl hydrogen signal with a chemical shift at δ_{H} 2.99 (s, 3H) is assigned to H-23 based on HMBC correlations with C-4 and C-13.

### ¹³C-NMR and DEPT

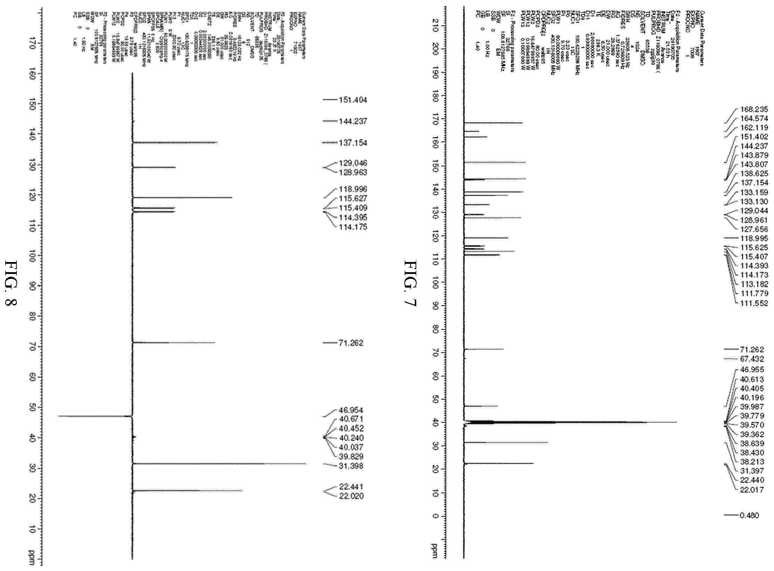

FIGs. 7 and 8 show the ¹³C NMR spectrum and DEPT spectrum of compound A crystal form I, respectively, and Table 3.3 shows the test results of ¹³C NMR spectrum of compound A crystal form I.

**Table 3.3 The test results of the nuclear magnetic resonance carbon spectrum of compound A crystal form I**

| Chemical shift (ppm) | Type of carbon | Assignment |
|---|---|---|
| 168.24 | C=O | C-13 |
| 164.57, 162.12 (J = 245 Hz) | C | C-19 |
| 151.40 | C | C-9 |
| 144.24 | C | C-3 |
| 143.88, 143.81 (J = 7 Hz) | C | C-15 |
| 138.63 | C | C-8 |
| 137.15 | CH | C-10 |
| 133.16, 133.13 (J = 3 Hz) | C | C-16 |
| 129.04, 128.96 (J = 8 Hz) | CH | C-17 |
| 127.66 | C | C-2 |
| 119.00 | CH | C-5 |
| 115.63, 115.41 (J = 22 Hz) | CH | C-18 |
| 114.39, 114.17 (J = 22 Hz) | CH | C-20 |
| 113.18 | C | C-1 |
| 111.78 | C | C-24/C-28 |
| 111.55 | C | C-28/C-24 |
| 71.26 | CH | C-14 |
| 46.96 | CH₂ | C-4 |
| 38.64, 38.43, 38.21 (J = 22 Hz) | CD₃ | C-27 |
| 31.40 | CH₃ | C-23 |
| 22.44 | CH₃ | C-32 |

The results show that the ¹³C-NMR spectrum has a total of 21 carbon signals, which in combination with DEPT, indicated that 2 methyl carbons, 1 methylene carbon, 6 methenyl carbons, 1 carbon attached to deuterium atom, and 11 carbons unattached to hydrogen are contained. According to HSQC data, all the above carbon signals attached to hydrogen are assigned, and the remaining carbons unattached to hydrogen are assigned based on chemical shifts and HMBC data: In the HMBC spectrum, δc 168.24 is associated with H-4, H-17, H-20 and H-23, which is assigned to C-13; δc 164.57, 162.12 are associated with H-14, H-17, H-18 and H-20, which in combination with the coupling constants, are assigned to C-19; δc 151.40 is associated with H-5 and H-10, which is assigned to C-9; δc 144.24 is associated with H-4, which in combination with the chemical shift, is assigned to C-3; δc 143.88, 143.81 are associated with H-14, H-17, H-18, H-20 and H-32, which in combination with the coupling constants, are assigned to C-15; δc 138.63 is associated with H-5, H-10, NH-12, and H-14, which is assigned to C-8; δc 133.16, 133.13 are associated with H-14, H-17, H-18, and H-20, which are assigned to C-16; δc 127.66 is associated with H-4, H-5, and H-10, which is assigned to C-2; δc 113.18 is associated with H-5 and H-10, which in combination with the chemical shift, is assigned to C-1; δc 111.78 and δc 111.55 are assigned to C-24/C-28 and C-28/C-24, respectively, based on their chemical shifts; δc 38.64, 38.43, 38.21 are assigned to C-27 based on the chemical shift and coupling constants.

### D-NMR spectrum

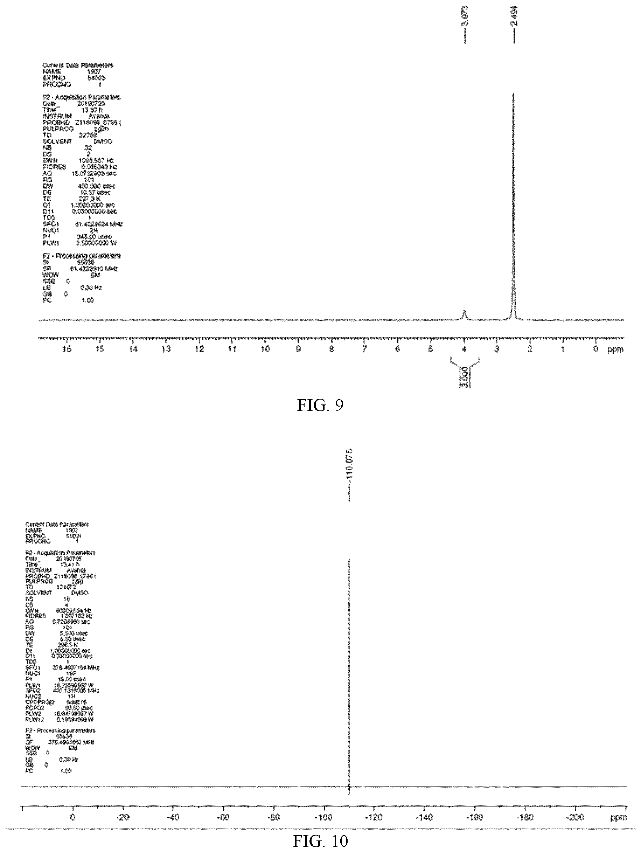

FIG. 9 shows the D-NMR spectrum of compound A crystal form I. In the D-NMR spectrum, the chemical shift at δ 3.97 is signal of deuterium atom.

### ¹⁹F-NMR spectrum

FIG. 10 shows the ¹⁹F-NMR spectrum of compound A crystal form I. In the ¹⁹F-NMR spectrum, the chemical shift at δ 110.08 is signal of fluorine attached to the benzene ring.

### HSQC and HMBC spectrum

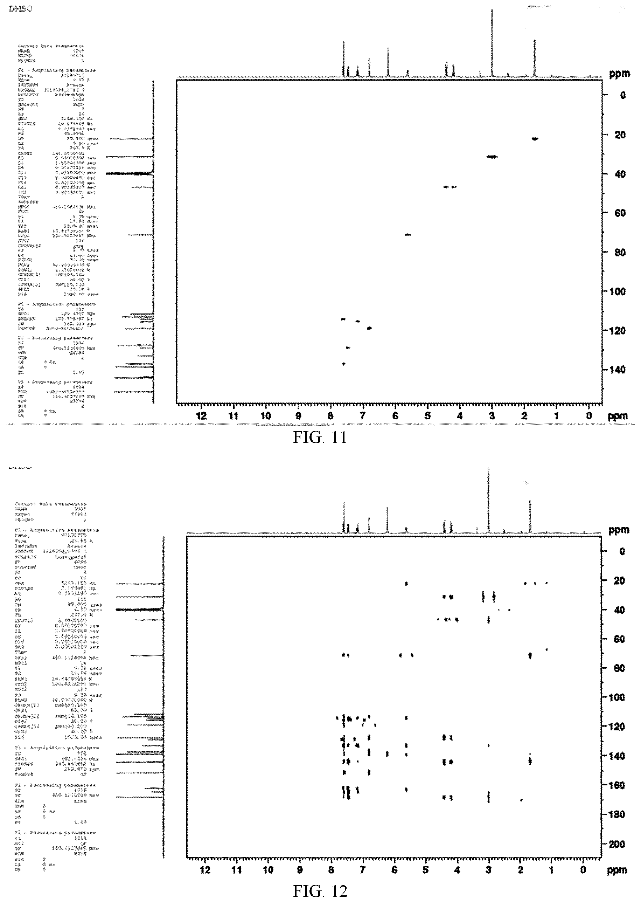

FIGs. 11 and 12 show the NMR HSQC and HMBC spectra of compound A crystal form I. Table 3.4 shows the test results of NMR HSQC and HMBC spectra of compound A crystal form I.

**Table 3.4 Test results of NMR HSQC and HMBC spectra of compound A crystal form I**

| Chemical shift (ppm) | Assignment | HSQC | HMBC |
|---|---|---|---|
| 7.61 | H-20 | 114.39, 114.17 | C-13, C-14, C-15, C-16, C-18, C-19 |
| 7.58 | H-10 | 137.15 | C-1, C-2, C-5, C-8, C-9 |
| 7.45 | H-17 | 129.04, 128.96 | C-13, C-14, C-15, C-16, C-18, C-19, C-20 |
| 7.16 | H-18 | 115.63, 115.41 | C-15, C-16, C-19, C-20 |
| 6.80 | H-5 | 119.00 | C-1, C-2, C-8, C-9, C-10 |
| 6.23 | NH-12 | - | C-8 |
| 5.61 | H-14 | 71.26 | C-8, C-15, C-16, C-19, C-20, C-32 |
| 4.41 | H-4a | 46.96 | C-2, C-3, C-13, C-23 |
| 4.19 | H-4b | | |
| 2.99 | H-23 | 31.40 | C-4, C-13 |
| 1.68 | H-32 | 22.44 | C-14, C-15 |

The results show that in the HMBC spectrum, H-20 is associated with C-13, C-14, C-15, C-16, C-18 and C-19, H-17 is associated with C-13, C-14, C-15, C-16, C-18, C-19 and C-20, H-18 is associated with C-15, C-16, C-19 and C-20, H-14 is associated with C-8, C-15, C-16, C-19, C-20 and C-32, H-32 is associated with C-14 and C-15, H-10 is associated with C-1, C-2, C-5, C-8 and C-9, H-5 is associated with C-1, C-2, C-8, C-9 and C-10, which is consistent with the presence of fragment A in the structure; H-4 is associated with C-2, C-3, C-13 and C-23, H-23 is associated with C-4 and C-13, which is consistent with the presence of fragment B in the structure; H-17 and H-20 are associated with C-13, indicating that C-16 of fragment A is linked to C-13 of fragment B, and H-5 and H-10 are associated with C-2, indicating that C-1 of fragment A is linked to C-2 of fragment B.

### COSY

FIG. 13 shows the NMR COSY spectrum of compound A crystal form I. Table 3.5 shows the test results of the NMR COSY spectrum of compound A crystal form I.

**Table 3.5 The test results of the NMR COSY spectrum of compound A crystal form I**

| ¹H-NMR (ppm) | Assignment | COSY |
|---|---|---|
| 7.45 | H-17 | H-18 |
| 5.61 | H-14 | H-32 |

The results showed that H-17 is associated with H-18 and H-14 is associated with H-32 in the COSY spectrum, further demonstrating the presence of fragment Ain the structure.

### IR analysis

Instrument and equipment: Shimadzu SHIMADZU IR Tracer 100 Fourier Transform Infrared Spectrometer

The infrared absorption spectrum of compound A crystal form I was collected within the wave number range of 4000-400 cm⁻¹ by KBr tableting method. FIG. 14 shows the IR spectrum of compound A crystal form I. Table 3.6 shows the test results of IR spectrum of compound A crystal form I.

**Table 3.6 The test results of IR spectrum of compound A crystal form I**

| Absorption wave number (cm⁻¹) | Type of vibration | Assignment |
|---|---|---|
| 3474, 3383, 3308 | V_{N-H} | NH₂ |
| 3184, 3111 | V_{=C-H} | Benzene ring |
| 2980, 2934 | V_{C-H} | CH₃, CH₂, CH |
| 2228 | ν_{C≡N} | C≡N |
| 1645, 1616 | v_{C=O} | C=O |
| 1499, 1491 | ν_{C=C} | Benzene ring |
| 1433, 1420, 1395, 1368, 1344 | δ_{C-H} | CH₃, CH₂, CH |
| 1252, 1069 | ν_{C-O-C} | Aryl ether |
| 878, 829 | γ_{=C-H} | Benzene ring |

The results show that the chemical structure of compound A crystal form I contains structures of NH₂, C=N, CH₃, CH₂, CH, C=O, benzene ring, and aryl ether. The specific analysis is as follows: 3474, 3383, 3308 cm⁻¹ represent N-H stretching vibration absorption peaks, which are consistent with the structure containing -NH₂; 3184, 3111 cm⁻¹ represent =C-H stretching vibration absorption peaks, 1499, 1491 cm⁻¹ represent C=C double bond stretching vibration absorption peaks, 878, 829 cm⁻¹ represent =C-H out-of-plane bending vibration absorption peaks, which are consistent with the presence of benzene ring structure; 2980, 2934 cm⁻¹ represent saturated C-H bond stretching vibration absorption peaks, 1433, 1420, 1395, 1368, 1344 cm⁻¹ represent the saturated C-H bond bending vibration absorption peaks, which are consistent with the presence of CH₃, CH₂ and CH structures; 2228 cm⁻¹ represents the C=N stretching vibration absorption peak, which is consistent with the structure containing C=N moiety; 1645, 1616 cm⁻¹ represent C=O stretching vibration peaks, which are consistent with the structure containing carbonyl moiety; 1252 cm⁻¹ represents C-O-C asymmetric stretching vibration absorption peak and 1069 cm⁻¹ represents C-O-C symmetric stretching vibration absorption peak, which are consistent with the structure containing aryl ether moiety.

### UV analysis

### Instrument and equipment: UV-2600 UV-Vis Spectrophotometer (Shimadzu Corporation, Japan)

The UV analysis was performed using methanol solution of sample (15.23 µg/mL). FIG. 15 shows the UV absorption spectrum of compound A crystal form I. Table 3.7 shows test results of the UV absorption of compound A crystal form I.

**Table 3.7 The test results of the UV absorption of compound A crystal form I**

| No. | Peak/Valley | Wavelength (nm) | ABS | ε (^{∗}10⁴) |
|---|---|---|---|---|
| 1 | Peak | 317 | 0.3153 | 0.85 |
| 2 | Peak | 206 | 0.9317 | 2.50 |
| 3 | Valley | 278 | 0.1373 | - |

The results show that the absorption peaks at λₘₐₓ 317, 206 nm are absorption peaks of n-π^{∗} transition of the conjugated system and the π-π^{∗} transition of the substituted benzene ring of the compound.

### HR-MS

Instrument and equipment: Waters Acquity I Class UPLC/Xevo G2-XS QT of HRMS ultra-performance liquid chromatography combined with high-resolution mass spectrometry system.

A solution of compound A crystal form I in methanol at a concentration of 15.23 µg/mL was used for the chromatographic analysis.

FIG. 16 shows the high resolution mass spectrum of compound A crystal form I. The results show an ion peak with a mass-to-charge ratio of m/z 410.1822 [M+H]⁺ in the high resolution mass spectrum, which deviates less than 5 ppm from the theoretical value (theoretical value 410.1820, C₂₁H₁₇D₃FN₆O₂), suggesting that the molecular formula of the sample is C₂₁H₁₆D₃FN₆O₂.

### Example 3.2 Characterization of compound A crystal form II

The compound A crystal form II was prepared after evaporation of the solvent mixture of butanone/ethyl acetate according to the method in Example 2.2 and characterized by XRPD, PLM, DSC and TGA. The specific results are as follows:

### XRPD analysis

FIG. 17 shows the XRPD data of compound A crystal form II acquired according to General method 1. A list of XRPD peaks at diffraction angles 2θ° (°2θ)±0.2 °2θ and relative intensities thereof are provided in Table 3.2. The XRPD results indicated that the crystallinity of crystal form II was good.

**Table 3.2: XRPD peak list (2θ°) of compound A crystal form II**

| Peak | Angle °2θ±0.2 °2θ | Relative intensity |
|---|---|---|
| 1 | 7.591 | 100.0 |
| 2 | 10.198 | 4.9 |
| 3 | 12.081 | 24.7 |
| 4 | 13.025 | 7.3 |
| 5 | 13.846 | 4.8 |
| 6 | 14.577 | 12.9 |
| 7 | 15.595 | 10.2 |
| 8 | 16.510 | 7.7 |
| 9 | 16.948 | 14.2 |
| 10 | 17.615 | 14.4 |
| 11 | 20.017 | 8.8 |
| 12 | 20.448 | 14.6 |
| 13 | 21.076 | 3.6 |
| 14 | 22.703 | 3.8 |
| 15 | 23.364 | 17.4 |
| 16 | 24.369 | 8.4 |
| 17 | 24.933 | 4.2 |
| 18 | 25.990 | 4.8 |
| 19 | 26.700 | 3.9 |
| 20 | 27.957 | 4.0 |
| 21 | 28.976 | 3.1 |

### PLM analysis

The compound A crystal form II was subjected to PLM analysis by General method 2. The PLM results indicated that the crystal form II was an irregularly shaped crystal.

### DSC and TGA thermal analysis

DSC and TGA analyses of compound A crystal form II were performed by General methods 4 and 5. The DSC results showed a melting endothermic peak at a peak temperature of about 230.09 °C with an onset temperature of about 227.91 °C. The TGA results showed a weight loss of up to about 6.69% at 160 °C. DSC and TGA analyses indicated that the sample was a solvate of butanone.

### Example 3.3 Characterization of compound A crystal form III

The compound A crystal form III was obtained by evaporation in a single solvent of butanone according to the method in Example 2.3 and characterized by XRPD, PLM, DSC and TGA. The specific results are as follows:

### XRPD analysis

FIG. 18 shows the XRPD data of compound A crystal form III acquired according to General method 1. A list of XRPD peaks at diffraction angles 2θ° (°2θ)±0.2 °2θ and relative intensities thereof are provided in Table 3.3. The XRPD results indicated that the crystallinity of crystal form III was good.

**Table 3.3: XRPD peak list (2θ°) of compound A crystal form III**

| Peak | Angle °2θ±0.2 °2θ | Relative intensity |
|---|---|---|
| 1 | 7.631 | 3.6 |
| 2 | 10.543 | 13.7 |
| 3 | 11.429 | 22.2 |
| 4 | 13.027 | 21.7 |
| 5 | 14.542 | 21.3 |
| 6 | 15.353 | 11.4 |
| 7 | 16.742 | 5.9 |
| 8 | 17.241 | 4.8 |
| 9 | 17.949 | 19.1 |
| 10 | 18.362 | 13.7 |
| 11 | 19.964 | 6.3 |
| 12 | 21.161 | 13.4 |
| 13 | 22.506 | 12.2 |
| 14 | 23.149 | 100.0 |
| 15 | 23.876 | 4.0 |
| 16 | 24.065 | 3.3 |
| 17 | 26.006 | 14.1 |
| 18 | 26.202 | 9.8 |
| 19 | 26.460 | 3.8 |
| 20 | 26.779 | 3.3 |
| 21 | 26.994 | 20.4 |
| 22 | 28.894 | 3.2 |
| 23 | 29.294 | 3.6 |
| 24 | 30.179 | 4.4 |
| 25 | 32.088 | 2.9 |
| 26 | 34.730 | 1.9 |
| 27 | 35.078 | 1.8 |

### PLM analysis

The compound A crystal form III was subjected to PLM analysis by General method 2. The PLM results indicated that the crystal form III was an irregularly shaped crystal.

### DSC and TGA thermal analysis

DSC and TGA analyses of compound A crystal form III were performed by General methods 4 and 5. The DSC results showed a melting endothermic peak at a peak temperature of about 226.13 °C with an onset temperature of about 220.19°C. The TGA results showed a weight loss of up to about 5.31% at 165 °C. DSC and TGA analyses indicated that the sample was a solvate of butanone.

### Example 3.4 Characterization of compound A crystal form IV

The compound A crystal form IV was obtained by evaporation in a single solvent of methanol according to the method in Example 2.3 and characterized by XRPD, PLM, DSC and TGA. The specific results are as follows:

### XRPD analysis

FIG. 19 shows the XRPD data of compound A crystal form IV acquired according to General method 1. A list of XRPD peaks at diffraction angles 2θ° (°2θ)±0.2 °2θ and relative intensities thereof are provided in Table 3.4. The XRPD results indicated that the crystallinity of the crystal form IV was ordinary.

**Table 3.4: XRPD peak list (2θ°) of compound A crystal form IV**

| Peak | Angle °2θ±0.2 °2θ | Relative intensity |
|---|---|---|
| 1 | 9.718 | 10.9 |
| 2 | 10.113 | 100.0 |
| 3 | 11.583 | 31.0 |
| 4 | 11.768 | 35.2 |
| 5 | 12.098 | 25.9 |
| 6 | 12.439 | 23.6 |
| 7 | 13.339 | 23.0 |
| 8 | 16.566 | 8.8 |
| 9 | 17.143 | 46.2 |
| 10 | 17.649 | 22.4 |
| 11 | 19.267 | 26.6 |
| 12 | 20.703 | 19.5 |
| 13 | 21.809 | 23.2 |
| 14 | 22.427 | 19.2 |
| 15 | 23.705 | 8.8 |
| 16 | 25.081 | 23.9 |
| 17 | 27.576 | 18.2 |
| 18 | 28.959 | 12.3 |
| 19 | 29.938 | 9.6 |

### PLM analysis

The compound A crystal form IV was subjected to PLM analysis by General method 2. The PLM results indicated that the crystal form IV was an irregularly shaped crystal.

### DSC and TGA thermal analysis

DSC and TGA analyses of compound A crystal form IV were performed by General methods 4 and 5. The DSC results showed an endothermic peak at a peak temperature of about 231.62 °C with an onset temperature of about 229.83 °C. The TGA results showed a weight loss of up to about 0.28% prior to 200 °C. DSC and TGA analyses indicated that the sample was an anhydrous crystal form.

### Example 3.5 Characterization of compound A crystal form V

The compound A crystal form V was obtained after slurrying in a suspension in a mixed solvent (water:methanol = 9:1) at room temperature according to the method in Example 2.4 and characterized by XRPD, PLM, DSC and TGA. The specific results are as follows:

### XRPD analysis

FIG. 20 shows the XRPD data of compound A crystal form V acquired according to General method 1. A list of XRPD peaks at diffraction angles 2θ° (°2θ)±0.2 °2θ and relative intensities thereof are provided in Table 3.5. The XRPD results indicated that the crystallinity of crystal form V was good.

**Table 3.5: XRPD peak list (2θ°) of compound A crystal form V**

| Peak | Angle °2θ±0.2 °2θ | Relative intensity |
|---|---|---|
| 1 | 4.912 | 28.1 |
| 2 | 6.939 | 100.0 |
| 3 | 7.688 | 9.9 |
| 4 | 9.774 | 30.1 |
| 5 | 10.326 | 18.7 |
| 6 | 10.859 | 17.8 |
| 7 | 11.960 | 7.8 |
| 8 | 12.709 | 41.4 |
| 9 | 14.246 | 29.4 |
| 10 | 14.482 | 33.4 |
| 11 | 15.289 | 16.6 |
| 12 | 16.276 | 55.9 |
| 13 | 16.708 | 15.4 |
| 14 | 17.242 | 42.4 |
| 15 | 17.494 | 76.9 |
| 16 | 18.519 | 25.7 |
| 17 | 19.425 | 34.7 |
| 18 | 19.941 | 15.3 |
| 19 | 21.001 | 27.3 |
| 20 | 21.317 | 29.5 |
| 21 | 21.765 | 7.8 |
| 22 | 22.145 | 8.1 |
| 23 | 22.734 | 26.7 |
| 24 | 23.090 | 19.4 |
| 25 | 23.424 | 14.3 |
| 26 | 24.250 | 23.9 |
| 27 | 24.727 | 9.4 |
| 28 | 25.218 | 28.2 |
| 29 | 25.808 | 10.4 |
| 30 | 26.241 | 15.7 |
| 31 | 26.987 | 10.1 |
| 32 | 27.856 | 7.0 |
| 33 | 28.841 | 10.1 |
| 34 | 29.332 | 20.7 |
| 35 | 29.688 | 26.1 |
| 36 | 30.279 | 7.8 |
| 37 | 31.071 | 10.0 |
| 38 | 31.856 | 10.2 |
| 39 | 33.788 | 5.7 |
| 40 | 34.138 | 7.1 |
| 41 | 35.302 | 7.1 |
| 42 | 38.297 | 4.6 |
| 43 | 38.733 | 4.6 |

### PLM analysis

The compound A crystal form V was subjected to PLM analysis by General method 2. The PLM results indicated that the crystal form V was an irregularly shaped crystal.

### DSC and TGA thermal analysis

DSC and TGA analyses of compound A crystal form V were performed by General methods 4 and 5. The DSC results showed a melting endothermic peak at a peak temperature of about 232.13 °C with an onset temperature of about 230.9 °C. The TGA results showed a weight loss of about 0.22% prior to 200 °C (residual solvent). DSC and TGA analyses indicated that the sample was an anhydrous crystal form.

### Example 3.6 Characterization of compound A crystal form VI

The compound A crystal form VI was obtained after slurrying in a suspension in methanol solvent at room temperature according to the method in Example 2.4 and characterized by XRPD, PLM, DSC and TGA. The specific results are as follows:

### XRPD analysis

FIG. 21 shows the XRPD data of compound A crystal form VI acquired according to General method 1. A list of XRPD peaks at diffraction angles 2θ° (°2θ)±0.2 °2θ and relative intensities thereof are provided in Table 3.6. The XRPD results indicated that the crystallinity of crystal form VI was good.

**Table 3.6: XRPD peak list (2θ°) of compound A crystal form VI**

| Peak | Angle °2θ±0.2 °2θ | Relative intensity |
|---|---|---|
| 1 | 10.247 | 100.0 |
| 2 | 12.198 | 70.6 |
| 3 | 12.514 | 42.8 |
| 4 | 14.302 | 5.1 |
| 5 | 17.258 | 53.8 |
| 6 | 17.596 | 44.5 |
| 7 | 18.659 | 10.4 |
| 8 | 19.406 | 27.9 |
| 9 | 20.432 | 4.1 |
| 10 | 21.020 | 4.4 |
| 11 | 21.888 | 30.3 |
| 12 | 22.479 | 11.9 |
| 13 | 23.014 | 5.9 |
| 14 | 23.799 | 14.7 |
| 15 | 24.410 | 23.3 |
| 16 | 25.158 | 21.0 |
| 17 | 26.259 | 7.3 |
| 18 | 27.599 | 27.5 |
| 19 | 28.504 | 15.3 |
| 20 | 29.902 | 7.4 |
| 21 | 30.434 | 4.9 |
| 22 | 34.102 | 2.6 |
| 23 | 34.453 | 4.1 |
| 24 | 37.089 | 2.5 |

### PLM analysis

Compound A crystal form VI was subjected to PLM analysis by General method 2. The PLM results indicated that the crystal form VI was an irregularly shaped crystal.

### DSC and TGA thermal analysis

DSC and TGA analyses of compound A crystal form VI were performed by General methods 4 and 5. The DSC results showed a melting endothermic peak at a peak temperature of about 232.83 °C with an onset temperature of about 231.56 °C. The TGA results showed substantially no weight loss prior to 200 °C. DSC and TGA analyses indicated that the sample was an anhydrous crystal form.

### Example 3.7 Characterization of compound A crystal form VII

The compound A crystal form VII was obtained after slurrying in a suspension in a mixed solvent (water:acetonitrile = 1:9) at room temperature according to the method in Example 2.4 and characterized by XRPD, PLM, DSC and TGA. The specific results are as follows:

### XRPD analysis

FIG. 22 shows the XRPD data of compound A crystal form VII acquired according to General method 1. A list of XRPD peaks at diffraction angles 2θ° (°2θ)±0.2 °2θ and relative intensities thereof are provided in Table 3.7. The XRPD results indicated that the crystallinity of the crystal form VII was ordinary.

**Table 3.7: XRPD peak list (2θ°) of compound A crystal form VII**

| Peak | Angle °2θ±0.2 °2θ | Relative intensity |
|---|---|---|
| 1 | 7.138 | 100.0 |
| 2 | 9.876 | 51.2 |
| 3 | 12.572 | 31.6 |
| 4 | 12.945 | 38.5 |
| 5 | 14.675 | 30.3 |
| 6 | 17.160 | 26.2 |

### PLM analysis

Compound A crystal form VII was subjected to PLM analysis by General method 2. The PLM results indicated that the crystal form VII was a needle-like crystal.

### DSC and TGA thermal analysis

DSC and TGA analyses of compound A crystal form VII were performed by General methods 4 and 5. The DSC results showed a melting endothermic peak at a peak temperature of about 232.38 °C with an onset temperature of about 230.70 °C. The TGA results showed a weight loss of about 0.35% prior to 200 °C. DSC and TGA analyses indicated that the sample was a solvate.

### Example 4 Trans-crystallization study of compound A

### 4.1 Competitive slurrying testing at room temperature

10 mg of compound A crystal form I, crystal form IV, crystal form V and crystal form VI were added to 500 µL of three different solvent systems (isobutanol, isopropanol and methyl tert-butyl ether), respectively. The mixture was slurried at room temperature for one day. The solids were dried at 50 °C under vacuum overnight and characterized by XRPD. The resulting results are shown in Table 4.1 and FIG. 23.

**Table 4.1 Results of competitive slurrying testing at room temperature**

| No. | Sample (mg) | Solvent | Solvent volume (µL) | Result |
|---|---|---|---|---|
| Compound A crystal form I, IV, V and VI | 40 | IBA | 500 | Crystal form I |
| Compound A crystal form I, IV, V and VI | 40 | IPA | 500 | Crystal form I |
| Compound A crystal form I, IV, V and VI | 40 | MTBE | 500 | Crystal form I |

The results showed that compound A crystal form IV, V and VI were all trans-crystallized to crystal form I in the three solvent systems of isobutanol, isopropanol and methyl tert-butyl ether, further proving that compound A crystal form I is a stable crystal form.

### 4.2 Heating studies

A certain amount of compound A crystal form V and VI were weighed, heated to 190 °C and then characterized by XRPD. The results are shown in FIG. 24. The results show that compound A crystal form V was transformed into crystal form I by heating to 190 °C, further proving that compound A crystal form I is a thermodynamically stable crystal form.

### Example 5 Stability study of compound A crystal form I

Stability influencing factor tests were carried out on the samples of compound A crystal form I. The corresponding placement conditions and test results are listed in Table 5.

**Table 5 Test data of influencing factors of the stability of compound A crystal form I**

| Factor | | Shape | Content | Total impurity content | Crystal form |
|---|---|---|---|---|---|
| High temperature (60°C) | 0 days | White solid | 99.9% | <0.05% | Crystal form I |
| | 5 days | White solid | 98.8% | <0.05% | Crystal form I |
| | 10 days | White solid | 100.4% | <0.05% | Crystal form I |
| | 37 days | Light yellow solid | 99.8% | <0.05% | Crystal form I |
| High humidity (92.5%RH/25 °C) | 0 days | White solid | 99.9% | <0.05% | Crystal form I |
| | 5 days | White solid | 99.5% | <0.05% | Crystal form I |
| | 10 days | White solid | 99.7% | <0.05% | Crystal form I |
| | 37 days | White solid | 99.4% | <0.05% | Crystal form I |
| Illumination (1.2^{∗}10⁶ Lux hr, 200 w·hr/m²) | 0 days | White solid | 99.9% | <0.05% | Crystal form I |
| | 5 days | Light yellow solid | 98.7% | <0.05% | Crystal form I |
| | 10 days | Light yellow solid | 99.1% | <0.05% | Crystal form I |

### Example 6 Preparation of crystalline salts of compound A

The initial attempt to prepare crystalline salts of compound A, using compound A crystal form I as the starting material, was consisted of two stages. The first stage included a solubility study of the starting material and a salt-forming screening in a 96-well plate; and the second stage included scale-up preparation of possibly formed crystalline salts in a milligram scale. These initial attempts identified eight crystal forms of salts of compound A, namely, compound A maleate crystal form I, compound A acetate crystal form I, compound A p-toluenesulfonate crystal form I, compound A hydrobromide crystal form I, compound A sulfate crystal form I, compound A oxalate crystal form I, compound A hydrochloride crystal form I and compound A mesylate crystal form I.

### Example 6.1 Solubility study of starting material (compound A crystal form I)

About 2 mg of the starting material was added to a 2 mL vial, and different solvents were added slowly (50 µL per time), until the sample was dissolved or solubility was less than 1 mg/mL. The solubility results are listed in Table 6.1.

The results showed that the solubilities of the starting material were relatively large in acetone, dichloromethane, tetrahydrofuran, methanol, acetonitrile, butanone and ethyl acetate, all of which were greater than 100 mg/mL; the solubilities were decreased in isopropyl acetate and ethanol, both of which were about 20 mg/mL; and the solubilities were relatively small in isopropanol, MTBE, isobutanol and water, which were about 1-2 mg/mL.

**Table 6.1: Solubility of the free base of the starting material (compound A crystal form I) in different solvents**

| Solvent | Solubility (mg/mL) |
|---|---|
| Acetone | ∼246 |
| Dichloromethane | ∼240 |
| Tetrahydrofuran | ∼215 |
| Methanol | ∼190 |
| Acetonitrile | ∼183 |
| Butanone | ∼173 |
| Ethyl acetate | ∼100 |
| Isopropyl acetate | ∼22 |
| Ethanol | ∼21 |
| Isopropanol | ∼2.5 |
| Methyl tert-butyl ether | ∼2.5 |
| Isobutanol | ∼1.7 |
| Water | ∼1 |

### Example 6.2 Screening of salts in a 96-well plate

A certain amount of hydrobromic acid, hydrochloric acid, sulfuric acid, p-toluenesulfonic acid, methanesulfonic acid, benzenesulfonic acid, oxalic acid and maleic acid were dissolved in methanol and diluted to 10 mL to prepare 8 acid solutions with a concentration of 0.1 M. About 750 mg of the starting material was weighed and added to 25 mL of methanol to prepare a free base solution with a concentration of 30 mg/mL. The 30 mg/mL free base solution was added to the 96-well plate at 100 µL per well, followed by 75 µL, of the corresponding acid solution to each well (37.5 µL, of sulfuric acid was added). The 96-well plate was allowed to volatilize at room temperature, and 100 µL of methanol, ethanol, isopropanol, isobutanol, butanone, tetrahydrofuran, acetonitrile, MTBE, ethyl acetate, acetone, isopropyl acetate, and water were added respectively to each column of wells after complete volatilization of the solvent. The 96-well plate was sealed with a sealing film and the film was punctured. The 96-well plate was placed in a fume hood at room temperature. The solvents were slowly volatilized to dryness, and the resulting solid samples were then characterized by PLM. The conditions for screening salts in a 96-well plate and solid states are listed in Table 6.2.

**Table 6.2 Sample states in screening of salts in a 96-well plate**

| | | 1 | 2 | 3 | 4 | 5 | 6 | 7 | 8 | 9 | 10 | 11 | 12 |
|---|---|---|---|---|---|---|---|---|---|---|---|---|---|
| | | MeOH | EtOH | IPA | IBA | MEK | THF | ACN | MTBE | EtOAc | Acetone | IPrOAc | H₂O |
| A | HBr | CR | CR | CR | CR | N/A | CR | CR | CR | CR | CR | CR | CR |
| B | HCl | N/A | N/A | CR | N/A | CR | CR | CR | CR | CR | CR | CR | CR |
| C | H₂SO₄ | oil | oil | oil | oil | N/A | oil | oil | oil | oil | oil | oil | oil |
| D | PTSA | CR | CR | CR | CR | N/A | CR | CR | CR | CR | CR | CR | CR |
| E | CH₃SO₃ H | oil | oil | oil | oil | N/A | oil | oil | oil | oil | oil | oil | oil |
| F | PhSO₃H | oil | oil | oil | oil | N/A | oil | oil | oil | oil | oil | oil | oil |
| G | Oxalic acid | GL | GL | GL | GL | GL | oil | oil | oil | oil | oil | oil | oil |
| H | Maleic acid | N/A | CR | CR | CR | CR | CR | CR | CR | CR | CR | CR | CR |

| | | | | | | | | | | | | | |
|---|---|---|---|---|---|---|---|---|---|---|---|---|---|
| Note: oil represents oil, CR represents crystal, GL represents glassy. | | | | | | | | | | | | | |

The results from the 96-well plate test showed that white solids were obtained in hydrobromic acid, hydrochloric acid, p-toluenesulfonic acid and maleic acid.

### Example 6.3 Small-scale preparation of compound A maleate crystal form I

About 30.1 mg of the starting material (compound A crystal form I) was weighed and dissolved in 400 µL of ethyl acetate to form a solution, and then 10.3 mg of maleic acid was added. The mixture was stirred at room temperature for 30 min and solids were precipitated. The mixture was stirred for another 30 min and then filtered. The solid obtained by filtration was dried under vacuum at 50 °C for 24 hours.

### Example 6.4 Small-scale preparation of compound A acetate crystal form I

About 29.5 mg of the starting material (compound A crystal form I) was weighed and dissolved in 400 µL of ethyl acetate to form a solution, and then 5.15 µL of acetic acid was added. The mixture was stirred at room temperature for 10 min and solids were precipitated. The mixture was stirred for another 30 min and then filtered. The solid obtained by filtration was dried under vacuum at 50 °C for 24 hours.

### Example 6.5 Small-scale preparation of compound A p-toluenesulfonate crystal form I

About 29.9 mg of the starting material (compound A crystal form I) was weighed and dissolved in 400 µL of ethyl acetate to form a solution, and then 16.8 mg of p-toluenesulfonic acid was added. The mixture was stirred at room temperature for 2.5 h and solids were precipitated. The mixture was stirred for another 30 min and then filtered. The solid obtained by filtration was dried under vacuum at 50 °C for 24 hours.

### Example 6.6 Small-scale preparation of compound A oxalate crystal form I

About 30.0 mg of the starting material (compound A crystal form I) was weighed and dissolved in 400 µL of ethyl acetate to form a solution, and then 11.1 mg of oxalic acid was added. The mixture was stirred at room temperature for 30 min and solids were precipitated. The solid obtained by filtration was dried under vacuum at 50 °C overnight. 30 mg of the dried solid was added to a solvent mixture of 160 µL of isopropanol and 40 µL of water and the mixture was slurried at room temperature for one day. The mixture was then filtered by suction. The solid obtained by suction filtration was dried under vacuum at 50 °C overnight.

### Example 6.7 Small-scale preparation of compound A sulfate crystal form I

About 40.0 mg of the starting material (compound A crystal form I) was weighed and dissolved in 400 µL of ethyl acetate to form a solution, and then 4.71 µL of sulfuric acid was added. The mixture was stirred at room temperature and a solid was precipitated immediately. The solid obtained by filtration was dried under vacuum at 50 °C overnight. 40 mg of the dried solid was added to a solvent mixture of 160 µL of isopropanol and 40 µL of water and the mixture was slurried at room temperature for one day. The mixture was then filtered by suction. The solid obtained by suction filtration was dried under vacuum at 50 °C overnight.

### Example 6.8 Small-scale preparation of compound A hydrobromide crystal form I

About 61.1 mg of the starting material (compound A crystal form I) was weighed and dissolved in 800 µL of ethyl acetate to form a solution, and then 23.62 µL of hydrobromic acid was added. Solids were precipitated immediately at room temperature. The mixture was stirred for another 30 min and then filtered. The solid obtained by filtration was dried under vacuum at 50 °C overnight.

### Example 6.9 Small-scale preparation of compound A hydrochloride crystal form I

Method 1: About 29.8 mg of the starting material (compound A crystal form I) was weighed and dissolved in 300 µL of ethyl acetate to form a solution, and then 6.72 µL of hydrochloric acid was added. Solids were precipitated immediately at room temperature. The mixture was stirred for another 30 min and then filtered. The solid obtained by filtration was dried under vacuum at 50 °C overnight.

Method 2: About 29.3 mg of the starting material (compound A crystal form I) was weighed and dissolved in 180 µL of butanone to form a solution, and then 6.72 µL of hydrochloric acid was added. Solids were precipitated immediately at room temperature. The mixture was stirred for another 30 min and then filtered. The solid obtained by filtration was dried under vacuum at 50 °C overnight.

Method 3: About 29.4 mg of the starting material (compound A crystal form I) was weighed and dissolved in 330 µL of acetone to form a solution, and then 6.72 µL of hydrochloric acid was added. Solids were precipitated immediately at room temperature. The mixture was stirred for another 30 min and then filtered. The solid obtained by filtration was dried under vacuum at 50 °C overnight.

Method 4: About 29.9 mg of the starting material (compound A crystal form I) was weighed and dissolved in 1.4 mL of isopropyl acetate to form a solution, and then 6.72 µL of hydrochloric acid was added. Solids were precipitated immediately at room temperature. The mixture was stirred for another 30 min and then filtered. The solid obtained by filtration was dried under vacuum at 50 °C overnight.

Method 5: About 29.6 mg of the starting material (compound A crystal form I) was weighed and dissolved in 170 µL of acetonitrile to form a solution, and then 6.72 µL of hydrochloric acid was added. Solids were precipitated immediately at room temperature. The mixture was stirred for another 30 min and then filtered. The solid obtained by filtration was dried under vacuum at 50 °C overnight.

### Example 6.10 Small-scale preparation of compound A mesylate crystal form I

About 30.2 mg of the starting material (compound A crystal form I) was weighed and dissolved in 400 µL of ethyl acetate to form a solution, and then 5.84 µL of methanesulfonic acid was added. Solids were precipitated immediately at room temperature. The mixture was stirred for another 30 min and then filtered. The solid obtained by filtration was dried under vacuum at 50 °C overnight.

### Example 7 Characterization of crystalline salts of compound A

### Example 7.1 Characterization of compound A maleate crystal form I

Compound A maleate crystal form I was prepared according to the method in Example 6.3 and characterized by XRPD, PLM, ¹H NMR, DSC, TGA and DVS. The specific results are as follows:

### XRPD analysis

FIG. 25 shows the XRPD data of compound A maleate crystal form I acquired according to General method 1. A list of XRPD peaks at diffraction angles 2θ° (°2θ)±0.2 °2θ and relative intensities thereof are provided in Table 7.1. The XRPD results indicated that the crystallinity of the maleate crystal form I was good.

**Table 7.1: XRPD peak list (2θ°) of compound A maleate crystal form I**

| Peak | Angle °2θ±0.2 °2θ | Relative intensity |
|---|---|---|
| 1 | 7.577 | 24 |
| 2 | 9.737 | 80 |
| 3 | 11.982 | 41 |
| 4 | 12.241 | 100 |
| 5 | 13.601 | 28 |
| 6 | 15.286 | 13 |
| 7 | 16.495 | 32 |
| 8 | 17.186 | 25 |
| 9 | 17.358 | 22 |
| 10 | 17.553 | 11 |
| 11 | 19.625 | 38 |
| 12 | 19.971 | 16 |
| 13 | 22.087 | 18 |
| 14 | 23.080 | 98 |
| 15 | 23.879 | 16 |
| 16 | 24.527 | 36 |
| 17 | 25.239 | 12 |
| 18 | 25.844 | 12 |
| 19 | 26.189 | 14 |
| 20 | 27.506 | 9 |
| 21 | 29.644 | 13 |
| 22 | 31.501 | 10 |

### PLM analysis

Compound A maleate crystal form I was subjected to PLM analysis by General method 2. The PLM results showed that the maleate crystal form I was an irregular crystal with small particle size and with agglomeration.

### ¹H NMR analysis

¹H NMR analysis of compound A maleate crystal form I was performed by General method 3. The ¹H NMR results indicated that the sample had chemical shifts and the maleate was formed with maleic acid and the free base in a 1:1 molar ratio. ¹H NMR (400 MHz, DMSO-d₆) δ 7.64 - 7.55 (m, 2H), 7.48 (dd, *J* = 8.6, 5.7 Hz, 1H), 7.20 (td, *J* = 8.5, 2.7 Hz, 1H), 6.88 (d, *J* = 1.7 Hz, 1H), 6.57 (s, 2H), 6.24 (s, 2H), 5.77 - 5.56 (m, 1H), 4.45 (d, *J* = 14.4 Hz, 1H), 4.21 (d, *J* = 14.4 Hz, 1H), 3.00 (s, 3H), 1.69 (d, *J* = 6.2 Hz, 3H).

### DSC and TGA thermal analysis

DSC and TGA analyses of compound A maleate crystal form I were performed by General methods 4 and 5. The DSC results showed a narrow melting endothermic peak at a peak temperature of 208.87 °C with an onset temperature of about 205.24 °C. The TGA results showed a weight loss of about 0.44% prior to 175 °C (a small amount of residual solvent). DSC and TGA analyses indicated that the sample was an anhydrous crystal form.

### DVS analysis

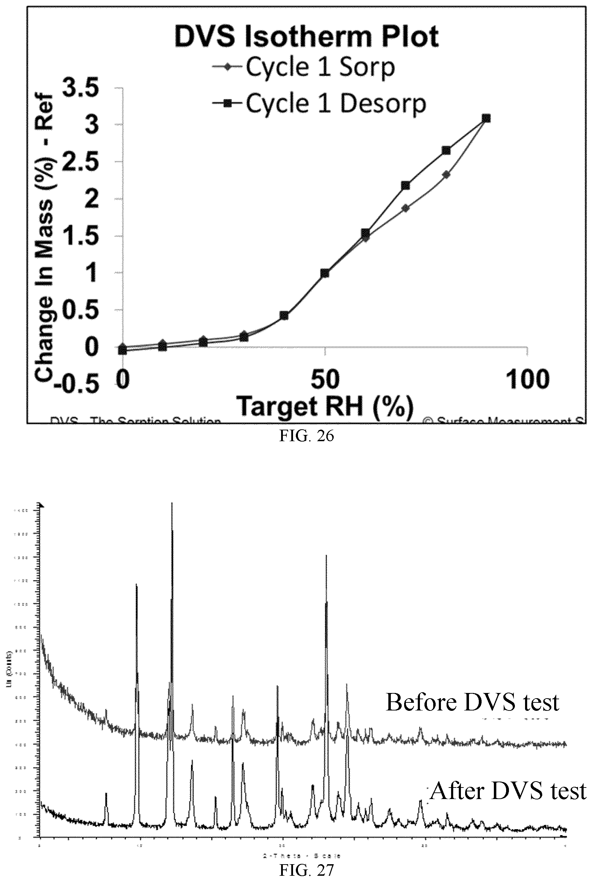

FIG. 26 shows the DVS curve of compound A maleate crystal form I acquired by General method 6. The DVS results showed a weight gain of about 2.3% as the humidity was increased from 10% RH to 80% RH.

The crystal form of the sample was not changed before and after the DVS test, as shown in FIG 27.

### Example 7.2 Characterization of compound A acetate crystal form I

Compound A acetate crystal form I was prepared according to the method in Example 6.4 and characterized by XRPD, PLM, ¹H NMR, DSC, TGA and DVS. The specific results are as follows:

### XRPD analysis

FIG. 28 shows the XRPD data of compound A acetate crystal form I acquired according to General method 1. A list of XRPD peaks at diffraction angles 2θ° (°2θ)±0.2 °2θ and relative intensities thereof are provided in Table 7.2. The XRPD results indicated that the crystallinity of the acetate crystal form I was good.

**Table 7.2: XRPD peak list (2θ°) of compound A acetate crystal form I**

| Peak | Angle °2θ±0.2 °2θ | Relative intensity |
|---|---|---|
| 1 | 10.521 | 35.1 |
| 2 | 11.409 | 46.7 |
| 3 | 12.866 | 50.7 |
| 4 | 13.005 | 26.2 |
| 5 | 14.521 | 45.3 |
| 6 | 15.349 | 17.4 |
| 7 | 16.707 | 10.9 |
| 8 | 17.236 | 12.7 |
| 9 | 17.910 | 39.3 |
| 10 | 18.343 | 15.5 |
| 11 | 19.961 | 13.7 |
| 12 | 21.140 | 27.3 |
| 13 | 22.536 | 24.4 |
| 14 | 23.129 | 100.0 |
| 15 | 23.383 | 7.9 |
| 16 | 23.873 | 7.4 |
| 17 | 24.048 | 5.1 |
| 18 | 25.985 | 22.7 |
| 19 | 26.198 | 6.8 |
| 20 | 26.538 | 4.0 |
| 21 | 26.751 | 3.8 |
| 22 | 26.993 | 13.6 |
| 23 | 27.247 | 7.5 |
| 24 | 28.861 | 4.2 |
| 25 | 29.280 | 7.4 |
| 26 | 30.178 | 7.0 |
| 27 | 34.990 | 2.9 |

### PLM analysis

Compound A acetate crystal form I was subjected to PLM analysis by General method 2. The PLM results indicated that the acetate crystal form I was an irregular crystal with small particle size.

### ¹H NMR analysis

¹H NMR analysis of compound A acetate crystal form I was performed by General method 3. The ¹H NMR results indicated that the sample had chemical shifts and the acetate was formed with acetic acid and the free base in a 1:1 molar ratio. ¹H NMR (400 MHz, DMSO-d₆) δ 11.95 (s, 1H), 7.65 - 7.55 (m, 2H), 7.47 (dd, *J* = 8.5, 5.7 Hz, 1H), 7.18 (td, *J* = 8.5, 2.7 Hz, 1H), 6.82 (d, *J* = 1.7 Hz, 1H), 6.20 (s, 1H), 5.72 - 5.54 (m, 1H), 4.44 (d, *J* = 14.4 Hz, 1H), 4.20 (d, *J* = 14.4 Hz, 1H), 3.00 (s, 3H), 1.91 (s, 3H), 1.68 (d, *J* = 6.2 Hz, 3H).

### DSC and TGA thermal analysis

DSC and TGA analyses of compound A acetate crystal form I were performed by General methods 4 and 5. The DSC results showed a melting endothermic peak at a peak temperature of 231.99 °C with an onset temperature of about 227.27 °C.

The TGA results showed a rapid weight loss of about 0.67% prior to 140 °C (a small amount of residual solvent).

DSC and TGA analyses indicated that the sample was an anhydrous crystal form.

### DVS analysis

FIG. 29 shows the DVS curve of compound A acetate crystal form I acquired by General method 6. The DVS results showed a weight gain of 0.01% as the humidity was increased from 10% RH to 80% RH.

The crystal form of the sample was not changed before and after the DVS test, as shown in FIG 30.

### Example 7.3 Characterization of compound A p-toluenesulfonate crystal form I

Compound A p-toluenesulfonate crystal form I was prepared according to the method in Example 6.5 and characterized by XRPD, PLM, ¹H NMR, DSC, TGA and DVS. The specific results are as follows:

### XRPD analysis

FIG. 31 shows the XRPD data of compound A p-toluenesulfonate crystal form I acquired according to General method 1. A list of XRPD peaks at diffraction angles 2θ° (°2θ)±0.2 °2θ and relative intensities thereof are provided in Table 7.3. The XRPD results indicated that the crystallinity of p-toluenesulfonate crystal form I was good.

**Table 7.3: XRPD peak list (2θ°) of compound A p-toluenesulfonate crystal form I**

| Peak | Angle °2θ±0.2 °2θ | Relative intensity |
|---|---|---|
| 1 | 5.544 | 20.2 |
| 2 | 10.583 | 100.0 |
| 3 | 12.968 | 29.6 |
| 4 | 14.012 | 16.5 |
| 5 | 14.503 | 38.6 |
| 6 | 16.886 | 22.9 |
| 7 | 17.478 | 9.5 |
| 8 | 18.417 | 11.1 |
| 9 | 19.607 | 16.0 |
| 10 | 21.298 | 24.5 |
| 11 | 21.674 | 51.3 |
| 12 | 23.266 | 24.1 |
| 13 | 24.421 | 4.3 |
| 14 | 25.063 | 5.4 |
| 15 | 26.437 | 14.8 |
| 16 | 28.290 | 7.8 |

### PLM analysis

Compound A p-toluenesulfonate crystal form I was subjected to PLM analysis by General method 2. The PLM results indicated that the p-toluenesulfonate crystal form I was an irregular crystal with small particle size.

### ¹H NMR analysis

¹H NMR analysis of compound A p-toluenesulfonate crystal form I was performed by General method 3. The ¹H NMR results indicated that the sample had chemical shifts and the p-toluenesulfonate was formed with p-toluenesulfonic acid and the free base in a 1:1 molar ratio. ¹H NMR (400 MHz, DMSO-d₆) δ 8.12 (s, 1H), 7.66 - 7.42 (m, 5H), 7.26 (td, *J* = 8.5, 2.6 Hz, 1H), 7.20 - 7.05 (m, 2H), 5.86 - 5.72 (m, 1H), 4.48 (d, *J* = 14.6 Hz, 1H), 4.27 (d, *J* = 14.5 Hz, 1H), 3.00 (s, 3H), 2.29 (s, 3H), 1.73 (d, *J* = 6.2 Hz, 3H).

### DSC and TGA thermal analysis

DSC and TGA analyses of compound A p-toluenesulfonate crystal form I were performed by General methods 4 and 5. The DSC results showed a melting endothermic peak at a peak temperature of about 269.08 °C with an onset temperature of about 263.64 °C. The TGA results showed the sample had a slow weight loss of 0.60% prior to 220 °C (a small amount of residual solvent). DSC and TGA analyses indicated that the sample was an anhydrous crystal form.

### DVS analysis

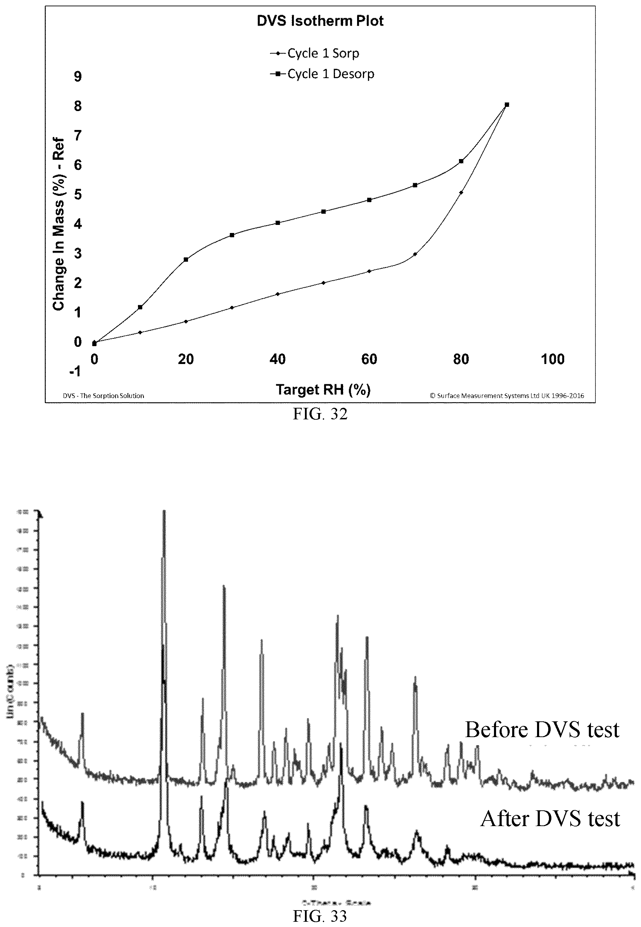

FIG. 32 shows the DVS curve of compound A p-toluenesulfonate crystal form I acquired by General method 6. The DVS results showed a weight gain of 5.1% as the humidity was increased from 10% RH to 80% RH.

The crystal form of the sample was changed before and after the DVS test, and it was speculated that a hydrate may be formed, as shown in FIG. 33.

### Example 7.4 Characterization of compound A oxalate crystal form I

Compound A oxalate crystal form I was prepared according to the method in Example 6.6 and characterized by XRPD, PLM, ¹H NMR, DSC and TGA. The specific results are as follows:

### XRPD analysis

FIG. 34 shows the XRPD data of compound A oxalate crystal form I acquired according to General method 1. A list of XRPD peaks at diffraction angles 2θ° (°2θ)±0.2 °2θ and relative intensities thereof are provided in Table 7.4. The XRPD results indicated that the crystallinity of the oxalate crystal form I was good.

**Table 7.4: XRPD peak list (2θ°) of compound A oxalate crystal form I**

| Peak | Angle °2θ±0.2 °2θ | Relative intensity |
|---|---|---|
| 1 | 4.320 | 31.2 |
| 2 | 5.540 | 27.5 |
| 3 | 6.642 | 100.0 |
| 4 | 8.810 | 4.3 |
| 5 | 10.366 | 18.1 |
| 6 | 10.980 | 15.0 |
| 7 | 11.606 | 5.0 |
| 8 | 12.712 | 4.4 |
| 9 | 13.242 | 19.6 |
| 10 | 14.226 | 8.7 |
| 11 | 18.385 | 3.3 |
| 12 | 18.772 | 7.2 |
| 13 | 19.886 | 2.0 |
| 14 | 20.230 | 1.5 |
| 15 | 21.334 | 4.1 |
| 16 | 21.888 | 5.4 |
| 17 | 22.774 | 3.7 |
| 18 | 23.088 | 6.6 |
| 19 | 27.342 | 2.5 |
| 20 | 30.237 | 5.0 |

### PLM analysis

Compound A oxalate crystal form I was subjected to PLM analysis by General method 2. The PLM results indicated that the oxalate crystal form I was an irregularly shaped crystal.

### ¹H NMR analysis

¹H NMR analysis of compound A oxalate crystal form I was performed by General method 3. The ¹H NMR results showed that the sample had a significant chemical shift of the peak near 5.80 ppm, and the oxalate was formed with oxalic acid and the free base in a 1:1 molar ratio. ¹H NMR (400 MHz, DMSO-d₆) δ 7.58 (d, *J* = 1.7 Hz, 2H), 7.48 (dd, *J* = 8.5, 5.7 Hz, 1H), 7.20 (dd, *J* = 8.5, 2.6 Hz, 1H), 6.84 (s, 1H), 6.52 - 6.16 (m, 2H), 5.63 (d, *J* = 4.7 Hz, 1H), 4.45 (d, *J* = 14.4 Hz, 2H), 4.22 (s, 1H), 3.00 (s, 3H), 1.69 (d, *J* = 6.2 Hz, 3H).

### DSC and TGA thermal analysis

DSC and TGA analyses of compound A oxalate crystal form I were performed by General methods 4 and 5. The DSC results showed a melting endothermic peak at a peak temperature of about 209.28 °C with an onset temperature of about 182.04 °C. The TGA results showed a weight loss of about 0.86% prior to 130°C (a small amount of residual solvent). DSC and TGA analyses showed that the sample was a solvate.

### Example 7.5 Characterization of compound A sulfate crystal form I

Compound A sulfate crystal form I was prepared according to the method in Example 6.7 and characterized by XRPD, PLM, ¹H NMR, DSC and TGA. The specific results are as follows:

### XRPD analysis

FIG. 35 shows the XRPD data of compound A sulfate crystal form I acquired according to General method 1. A list of XRPD peaks at diffraction angles 2θ° (°2θ)±0.2 °2θ and relative intensities thereof are provided in Table 7.5. The XRPD results indicated that the crystallinity of the sulfate crystal form I was good.

**Table 7.5: XRPD peak list (2θ°) of compound A sulfate crystal form I**

| Peak | Angle °2θ±0.2 °2θ | Relative intensity |
|---|---|---|
| 1 | 9.933 | 6.8 |
| 2 | 11.604 | 33.5 |
| 3 | 11.780 | 17.5 |
| 4 | 12.667 | 11.3 |
| 5 | 13.318 | 37.9 |
| 6 | 14.425 | 7.5 |
| 7 | 14.740 | 30.9 |
| 8 | 15.763 | 96.3 |
| 9 | 15.961 | 28.5 |
| 10 | 18.051 | 1.4 |
| 11 | 18.385 | 45.4 |
| 12 | 19.228 | 28.6 |
| 13 | 19.447 | 13.6 |
| 14 | 19.865 | 6.2 |
| 15 | 21.413 | 44.1 |
| 16 | 22.083 | 21.1 |
| 17 | 22.361 | 44.3 |
| 18 | 22.576 | 20.5 |
| 19 | 22.823 | 7.9 |
| 20 | 23.266 | 100.0 |
| 21 | 23.618 | 13.0 |
| 22 | 23.936 | 27.4 |
| 23 | 24.721 | 3.3 |
| 24 | 24.958 | 28.3 |
| 25 | 25.944 | 7.0 |
| 26 | 26.199 | 3.1 |
| 27 | 26.771 | 8.8 |
| 28 | 27.303 | 12.9 |
| 29 | 27.522 | 24.4 |
| 30 | 28.565 | 9.6 |
| 31 | 28.765 | 18.4 |
| 32 | 29.725 | 17.9 |
| 33 | 30.692 | 9.4 |
| 34 | 30.906 | 14.6 |
| 35 | 32.032 | 12.3 |
| 36 | 33.014 | 7.0 |
| 37 | 33.450 | 9.9 |
| 38 | 34.393 | 5.2 |
| 39 | 34.882 | 8.9 |
| 40 | 35.731 | 2.0 |
| 41 | 35.931 | 5.3 |
| 42 | 37.287 | 3.5 |
| 43 | 37.828 | 3.2 |
| 44 | 38.552 | 2.4 |
| 45 | 39.783 | 3.0 |

### PLM analysis

Compound A sulfate crystal form I was subjected to PLM analysis by General method 2. The PLM results indicated that the sulfate crystal form I was an irregularly shaped crystal.

### ¹H NMR analysis

¹H NMR analysis of compound A sulfate crystal form I was performed by General method 3. The ¹H NMR results showed that the sample had a significant chemical shift of the peak near 5.80 ppm, and the sulfate was formed. ¹H NMR (400 MHz, DMSO-d₆) δ 7.61 - 7.48 (m, 3H), 7.23 (dd, J = 8.1, 5.9 Hz, 1H), 7.04 (s, 1H), 5.73 (s, 1H), 4.47 (d, J = 14.4 Hz, 1H), 4.25 (d, J = 14.4 Hz, 1H), 3.00 (s, 3H), 1.72 (d, J = 6.1 Hz, 3H).

### DSC and TGA thermal analysis

DSC and TGA analyses of compound A sulfate crystal form I were performed by General methods 4 and 5. The DSC results showed a melting endothermic peak at a peak temperature of about 250.97 °C with an onset temperature of about 244.89 °C. The TGAresults showed a weight loss of about 2.95% prior to 90 °C, which indicated a hydrate.

### Example 7.6 Characterization of compound A hydrobromide crystal form I

Compound A hydrobromide crystal form I was prepared according to the method in Example 6.8 and characterized by XRPD, PLM, ¹H NMR, DSC and TGA. The specific results are as follows:

### XRPD analysis

FIG. 36 shows the XRPD data of compound A hydrobromide crystal form I acquired according to General method 1. A list of XRPD peaks at diffraction angles 2θ° (°2θ)±0.2 °2θ and relative intensities thereof are provided in Table 7.6. The XRPD results indicated that the crystallinity of the hydrobromide crystal form I was good.

**Table 7.6: XRPD peak list (2θ°) of compound A hydrobromide crystal form I**

| Peak | Angle °2θ±0.2 °2θ | Relative intensity |
|---|---|---|
| 1 | 7.731 | 6.1 |
| 2 | 8.245 | 4.0 |
| 3 | 9.222 | 25.3 |
| 4 | 9.932 | 5.2 |
| 5 | 11.905 | 39.8 |
| 6 | 12.652 | 12.2 |
| 7 | 13.206 | 100.0 |
| 8 | 14.702 | 20.0 |
| 9 | 15.426 | 5.2 |
| 10 | 16.396 | 12.9 |
| 11 | 16.924 | 19.3 |
| 12 | 17.436 | 7.4 |
| 13 | 17.692 | 7.3 |
| 14 | 18.636 | 19.8 |
| 15 | 19.148 | 10.2 |
| 16 | 19.937 | 28.0 |
| 17 | 20.294 | 22.2 |
| 18 | 21.102 | 16.5 |
| 19 | 21.532 | 16.1 |
| 20 | 21.966 | 9.0 |
| 21 | 22.359 | 4.9 |
| 22 | 23.105 | 9.9 |
| 23 | 23.995 | 83.3 |
| 24 | 24.941 | 60.9 |
| 25 | 25.492 | 16.6 |
| 26 | 26.081 | 8.8 |
| 27 | 26.773 | 33.3 |
| 28 | 27.500 | 45.1 |
| 29 | 28.149 | 6.5 |
| 30 | 29.843 | 9.2 |
| 31 | 30.554 | 7.4 |
| 32 | 31.185 | 9.3 |
| 33 | 31.342 | 9.7 |
| 34 | 32.028 | 8.6 |
| 35 | 33.154 | 21.7 |
| 36 | 33.981 | 6.3 |
| 37 | 34.218 | 7.6 |
| 38 | 35.771 | 7.0 |
| 39 | 36.383 | 3.9 |
| 40 | 37.211 | 7.0 |
| 41 | 37.642 | 5.1 |

### PLM analysis

Compound A hydrobromide crystal form I was subjected to PLM analysis by General method 2. The PLM results indicated that the hydrobromide crystal form I was a rod-shaped crystal.

### ¹H NMR analysis

¹H NMR analysis of compound A hydrobromide crystal form I was performed by General method 3. The ¹H NMR results showed that the sample had a significant chemical shift of the peak near 5.80 ppm, and the hydrobromide was formed. ¹H NMR (400 MHz, DMSO-d₆) δ 8.02(br s, 1H), 8.24 - 7.80 (m, 2H), 7.60 (d, *J* = 1.6 Hz, 1H), 7.58 - 7.49 (m, 2H), 7.26 (td, *J* = 8.6, 2.6 Hz, 1H), 7.13 (s, 1H), 5.78 (q, *J* = 6.0 Hz, 1H), 4.48 (d, *J* = 14.6 Hz, 1H), 4.27 (d, *J* = 14.6 Hz, 1H), 3.00 (s, 3H), 1.73 (d, *J* = 6.2 Hz, 3H).

### DSC and TGA thermal analysis

DSC and TGA analyses of compound A hydrobromide crystal form I were performed by General methods 4 and 5. The DSC results showed a melting endothermic peak at a peak temperature of about 240.87 °C with an onset temperature of about 232.49 °C. The TGAresults showed that the sample had a rapid weight loss of about 8.18% prior to 150 °C (solvent residue). DSC and TGA analyses indicated that the sample may be a solvate.

### Example 7.7 Characterization of compound A hydrochloride crystal form I

Compound A hydrochloride crystal form I was obtained by all five preparation methods according to Example 6.9, and the sample obtained by Method 1 was characterized by XRPD, PLM, ¹H NMR, DSC, TGA and DVS. The specific results are as follows:

### XRPD analysis

FIG. 37 shows the XRPD data of compound A hydrochloride crystal form I acquired according to General method 1. A list of XRPD peaks at diffraction angles 2θ° (°2θ)±0.2 °2θ and relative intensities thereof are provided in Table 7.7. The XRPD results indicated that the crystallinity of the hydrochloride crystal form I was good.

**Table 7.7: XRPD peak list (2θ°) of compound A hydrochloride crystal form I**

| Peak | Angle °2θ±0.2 °2θ | Relative intensity |
|---|---|---|
| 1 | 6.699 | 10.5 |
| 2 | 7.944 | 21.3 |
| 3 | 8.280 | 16.9 |
| 4 | 9.380 | 35.3 |
| 5 | 9.991 | 6.2 |
| 6 | 12.079 | 52.0 |
| 7 | 12.749 | 26.8 |
| 8 | 13.319 | 100.0 |
| 9 | 14.111 | 13.5 |
| 10 | 14.758 | 18.0 |
| 11 | 15.586 | 5.1 |
| 12 | 16.589 | 7.7 |
| 13 | 17.103 | 13.1 |
| 14 | 17.361 | 6.8 |
| 15 | 17.828 | 7.6 |
| 16 | 18.618 | 12.8 |
| 17 | 19.367 | 5.5 |
| 18 | 19.996 | 10.6 |
| 19 | 20.449 | 12.7 |
| 20 | 21.062 | 7.7 |
| 21 | 21.830 | 10.9 |
| 22 | 23.151 | 5.9 |
| 23 | 23.530 | 5.0 |
| 24 | 24.093 | 59.9 |
| 25 | 24.920 | 47.0 |
| 26 | 25.118 | 24.7 |
| 27 | 25.544 | 3.8 |
| 28 | 26.240 | 4.3 |
| 29 | 26.613 | 20.8 |
| 30 | 27.006 | 18.4 |
| 31 | 27.559 | 25.7 |
| 32 | 28.406 | 5.6 |
| 33 | 28.624 | 7.2 |
| 34 | 29.633 | 5.9 |
| 35 | 30.573 | 4.5 |
| 36 | 30.828 | 5.9 |
| 37 | 31.248 | 2.9 |
| 38 | 32.245 | 2.8 |
| 39 | 33.016 | 6.4 |
| 40 | 33.329 | 6.8 |
| 41 | 33.585 | 5.1 |
| 42 | 34.218 | 6.1 |
| 43 | 35.700 | 1.8 |
| 44 | 36.227 | 2.1 |
| 45 | 37.126 | 4.3 |
| 46 | 37.803 | 2.3 |
| 47 | 38.436 | 1.8 |

### PLM analysis

Compound A hydrochloride crystal form I was subjected to PLM analysis by General method 2. The PLM results indicated that the hydrochloride crystal form I was a rod-shaped crystal with a large size.

### ¹H NMR analysis

¹H NMR analysis of compound A hydrochloride crystal form I was performed by General method 3. The ¹H NMR results showed that the hydrogen of the sample near 6.80 ppm had a chemical shift of about 0.12 ppm, indicating that the hydrochloride was formed with hydrochloric acid and the free base. ¹H NMR (400 MHz, DMSO) δ 8.24 - 7.85 (m, 3H), 7.62 (s, 1H), 7.54 (ddd, *J* = 14.3, 9.3, 4.2 Hz, 1H), 7.25 (td, *J* = 8.5, 2.6 Hz, 1H), 7.11 (s, 1H), 5.76 (t, *J* = 5.6 Hz, 1H), 4.47 (d, *J* = 14.6 Hz, 1H), 4.27 (d, *J* = 14.5 Hz, 1H), 3.00 (s, 3H), 1.73 (d, *J* = 6.2 Hz, 3H).

### DSC and TGA thermal analysis

DSC and TGA analyses of the collected compound A hydrochloride crystal form I were performed by General methods 4 and 5. The DSC results showed a melting endothermic peak at a peak temperature of about 221.36 °C with an onset temperature of about 207.44 °C. The TGA results showed a weight loss of about 1.67% prior to 125 °C due to a small amount of residual solvent, and a weight loss of about 3.84% from 125 °C to 230 °C. DSC and TGA analyses indicated that the sample may be a solvate.

### DVS analysis

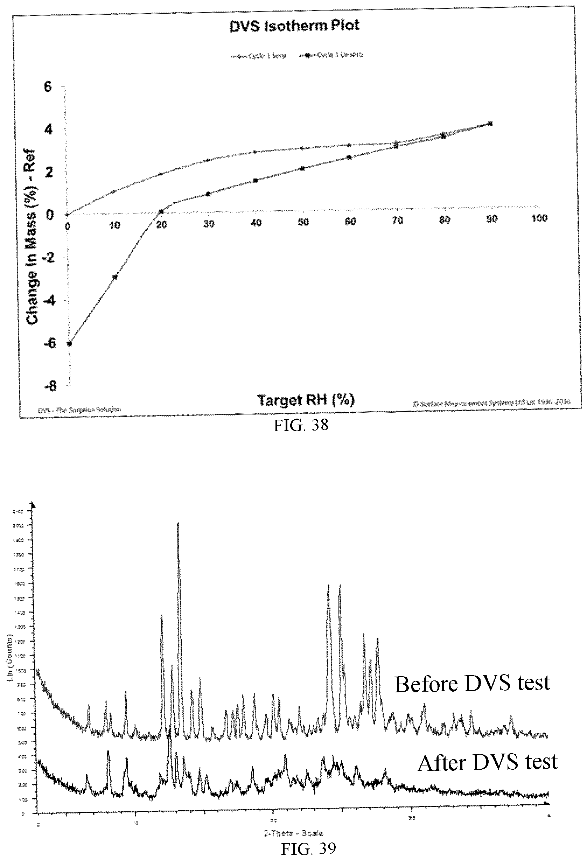

FIG. 38 shows the DVS curve of compound A hydrochloride crystal form I acquired by General method 6. The DVS results showed a weight gain of 3.5% as the humidity was increased from 10% RH to 80% RH.

The crystal form of the sample was changed before and after the DVS test, and it was speculated that a hydrate may be formed, as shown in FIG. 39.

### Example 7.8 Characterization of compound A mesylate crystal form I

Compound A mesylate crystal form I was prepared according to the method in Example 6.10 and characterized by XRPD, PLM and ¹H NMR. The specific results are as follows:

### XRPD analysis

FIG. 40 shows the XRPD data of compound A mesylate crystal form I acquired according to General method 1. A list of XRPD peaks at diffraction angles 2θ° (°2θ)±0.2 °2θ and relative intensities thereof are provided in Table 7.8. The XRPD results showed that the crystallinity of the mesylate crystal form I was poor.

**Table 7.8: XRPD peak list (2θ°) of compound A mesylate crystal form I**

| Peak | Angle °2θ±0.2 °2θ | Relative intensity |
|---|---|---|
| 1 | 8.338 | 100.0 |
| 2 | 9.363 | 40.1 |
| 3 | 10.092 | 31.8 |
| 4 | 11.706 | 91.7 |
| 5 | 12.513 | 28.7 |
| 6 | 13.932 | 80.9 |
| 7 | 14.738 | 56.7 |
| 8 | 15.053 | 35.0 |
| 9 | 15.742 | 47.1 |
| 10 | 16.711 | 24.8 |
| 11 | 18.341 | 82.2 |
| 12 | 19.093 | 37.6 |
| 13 | 21.148 | 50.3 |
| 14 | 21.588 | 72.0 |
| 15 | 22.597 | 51.0 |
| 16 | 23.170 | 33.1 |
| 17 | 23.716 | 27.4 |
| 18 | 24.469 | 35.7 |
| 19 | 24.650 | 49.7 |
| 20 | 24.824 | 35.7 |
| 21 | 25.732 | 69.4 |
| 22 | 30.238 | 33.1 |
| 23 | 32.189 | 29.3 |

### PLM analysis

Compound A mesylate crystal form I was subjected to PLM analysis by General method 2. The PLM results indicated that the mesylate crystal form I was an irregularly shaped crystal.

### ¹H NMR analysis

¹H NMR analysis of compound A mesylate crystal form I was performed by General method 3. The ¹H NMR results showed that the hydrogen of the sample near 5.80 ppm had a significant chemical shift, indicating that the mesylate was formed with methylsulfonic acid and the free base in a 1:1 molar ratio. ¹H NMR (400 MHz, DMSO-d₆) δ 8.17(br s, 1H), 7.61(d, J=4.0 Hz, 1H), 7.54 (ddd, *J* = 10.0, 8.6, 4.2 Hz, 2H), 7.26 (td, *J* = 8.5, 2.6 Hz, 1H), 7.14(s, 1H), 5.89 - 5.65 (m, 1H), 4.48 (d, *J* = 14.6 Hz, 1H), 4.28 (d, *J* = 14.6 Hz, 1H), 3.00(s, 3H), 2.33(s, 3H), 1.73 (d, *J* = 6.2 Hz, 3H).

### Example 8 Accelerated stability study of the free base and crystalline salts of compound A

A certain amount of compound A crystal form I, compound A maleate crystal form I, compound A acetate crystal form I and compound A p-toluenesulfonate crystal form I were put into stability test chambers at 40 °C/75%RH and 60 °C, respectively, for 7 days. The crystals were tested for HPLC and XRPD in different time points.

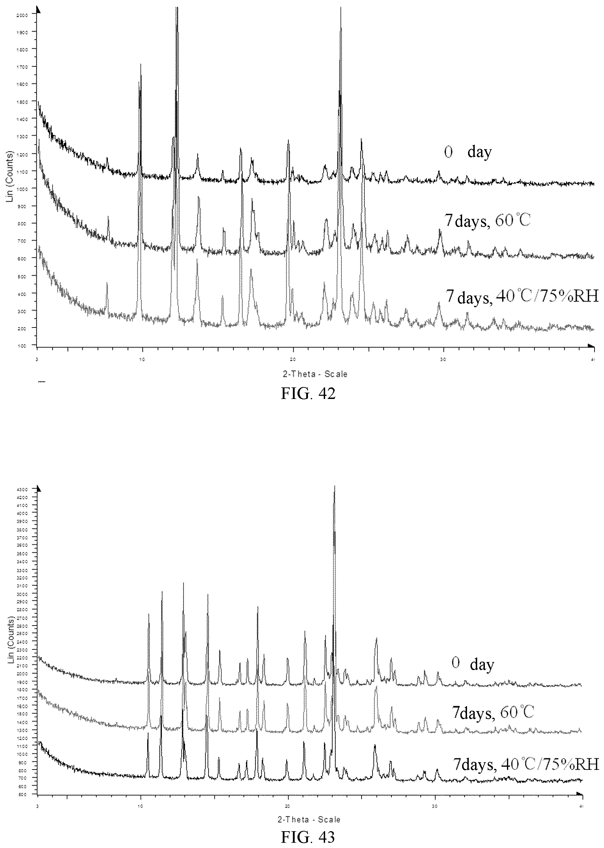

The results showed that the crystal form and purity of compound A crystal form I, compound A maleate crystal form I, compound A acetate crystal form I and compound A p-toluenesulfonate crystal form Iwere not changed after being kept at 40 °C/75% RH and 60 °C for 7 days, which showed good stability, as shown in FIGs. 41-44. The specific results are listed in Table 8.

**Table 8 Results of accelerated stability studies of the free base and crystalline salts of compound A**

| Sample | Conditions | Purity % | Crystal form |
|---|---|---|---|
| Compound A crystal form I | 0 days | 99.2 | Unchanged |
| | 60 °C, 7 days | 99.3 | Unchanged |
| | 40 °C/75% RH, 7 days | 99.4 | Unchanged |
| Compound A maleate crystal form I | 0 days | 99.8 | Unchanged |
| | 60 °C, 7 days | 99.8 | Unchanged |
| | 40 °C/75% RH, 7 days | 99.7 | Unchanged |
| Compound A acetate crystal form I | 0 days | 99.7 | Unchanged |
| | 60 °C, 7 days | 99.8 | Unchanged |
| | 40 °C/75% RH, 7 days | 99.8 | Unchanged |
| Compound A p-toluenesulfonate crystal form I | 0 days | 99.7 | Unchanged |
| | 60 °C, 7 days | 99.7 | Unchanged |
| | 40 °C/75% RH, 7 days | 99.7 | Unchanged |

### Example 9 Solubility study of the free base and crystalline salts of compound A in different vehicles

The solubility and stability of compound A crystal form I, compound A maleate crystal form I and compound A acetate crystal form I in water, SGF (simulated gastric fluid), FaSSIF (simulated intestinal fluid in fasted state) and FeSSIF (simulated intestinal fluid in fed state) were studied. The specific results are listed in Table 9.

**Table 9 Solubility of the free base and crystalline salts of compound A in different vehicles**

| Sample | Vehicle | Temp. | Solubility at 0.5h (mg/mL) | Solubility at 24h (mg/mL) |
|---|---|---|---|---|
| Compound A crystal form I (5 mg/ml) | H₂O | Room temperature | 0.11 | 0.095 |
| Compound A crystal form I (10 mg/ml) | SGF | 37 °C | 10.89 | 10.23 |
| | FaSSIF | 37 °C | 0.17 | 0.11 |
| | FeSSIF | 37 °C | 0.55 | 0.38 |
| Compound A maleate crystal form I (5 mg/ml) | H₂O | Room temperature | 12.92 | 7.46 |
| Compound A maleate crystal form I (10 mg/ml) | SGF | 37 °C | 7.28 | 6.78 |
| | FaSSIF | 37 °C | 1.29 | 2.95 |
| | FeSSIF | 37 °C | 0.63 | 0.60 |
| Compound A acetate crystal form I (5 mg/ml) | H₂O | Room temperature | 0.98 | 0.83 |
| Compound A acetate crystal form I (10 mg/ml) | SGF | 37 °C | 9.52 | 8.47 |
| | FaSSIF | 37 °C | 0.13 | 0.46 |
| | FeSSIF | 37 °C | 0.458 | 0.454 |

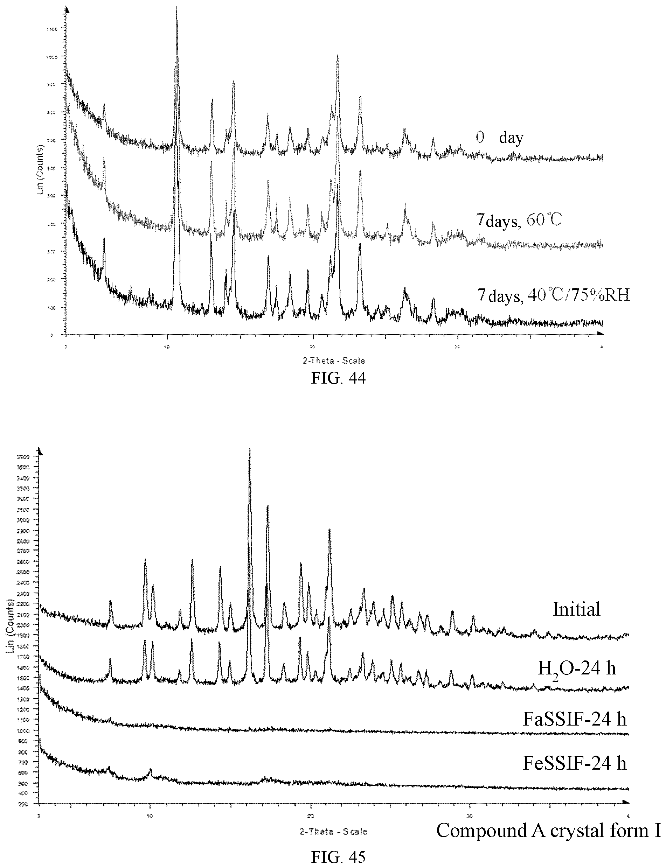

The results showed that compound A crystal form I had good solubility (>5 mg/mL) in SGF biological vehicle, and poor solubility (<3 mg/mL) in water, FaSSIF and FeSSIF biological vehicles. The crystal form of compound A crystal form I was not changed in water. The crystallinity of compound A crystal form I was decreased in FaSSIF and FeSSIF biological vehicles to nearly amorphous, as shown in FIG. 45, indicating that compound A crystal form I was not precipitated as a solid over time in FaSSIF and FeSSIF biological vehicles and remained in solution all the time.

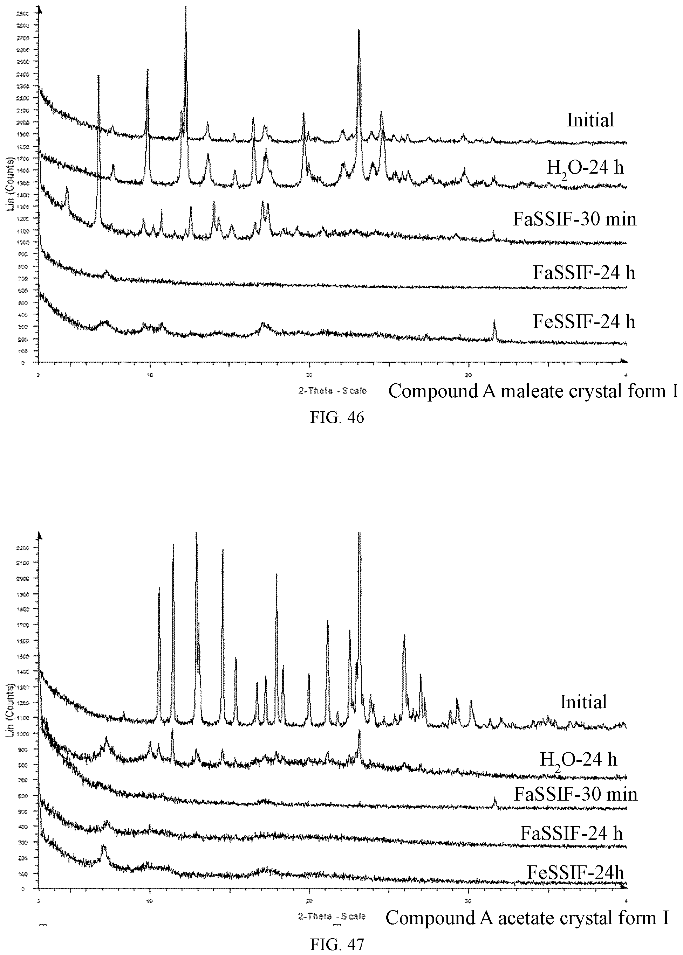

Compound A maleate crystal form I had good solubility in water and SGF biological vehicle (>5 mg/mL) and poor solubility in FaSSIF and FeSSIF biological vehicles (<3 mg/mL). The crystal form of compound A maleate crystal form I was not changed in water. The crystallinity of compound A maleate crystal form I was decreased in FaSSIF and FeSSIF to nearly amorphous after 24 h, as shown in FIG. 46, indicating that compound A maleate crystal form I was not precipitated as a solid over time in FaSSIF and FeSSIF biological vehicles and remained in solution all the time.

Compound A acetate crystal form I had good solubility in SGF biological vehicle (>5 mg/mL) and poor solubility in water, FaSSIF and FeSSIF biological vehicles (<3 mg/mL). The crystallinity of compound A acetate crystal form I was decreased in water, FaSSIF and FeSSIF biological vehicles to nearly amorphous, as shown in FIG. 47, indicating that compound A acetate crystal form I was not precipitated as a solid over time in FaSSIF and FeSSIF biological vehicles and remained in solution all the time.

The solubilities of compound A maleate and acetate in water were significantly increased, compared to that of the free base, from less than 0.2 mg/mL to more than 0.8 mg/mL (even more than 6 mg/mL for the maleate); in the biologically relevant medium FaSSIF, the solubility of the maleate was significantly increased compared to that of the free base.

### Example 10 Single crystal X-ray structure and absolute stereochemistry of compound A

Attempts were made to form single crystals of compound A at room temperature using the slow volatilization method. The experiment was performed in a 1.5 mL vial (commonly used liquid phase analysis vial) by dissolving about 100 mg of compound A crystal form I in acetone, ethyl acetate, isopropyl acetate, and ethanol, respectively. To the above clear solution was added n-heptane as an anti-solvent until the solution became slightly cloudy. 1-2 drops of solvent were added or the temperature was increased, until the solution was clarified again. The cap was loosened and the vial was left to volatilize slowly at room temperature. A single crystal was obtained in a solvent mixture of ethyl acetate and n-heptane one day later.

The collection and analysis of single crystal structure data were done by the Crystallographic Laboratory of Peking University. Single-crystal diffraction data of the samples were acquired using a SuperNova XRD diffraction system from Agilent at 180 K using the Cu target Kα spectral line (*λ* = 1.54178 Å). Data modification and absorption correction were performed using the CrysAlisPro program, and the structure was resolved by a dual linear space algorithm using the SHELXT program. Non-hydrogen atoms may be located in different Fourier graphs, and hydrogen atoms were geometrically filled into their parent atoms. The refinement of the final structure was done based on the *F*² full-matrix least squares method using the SHELXL program.

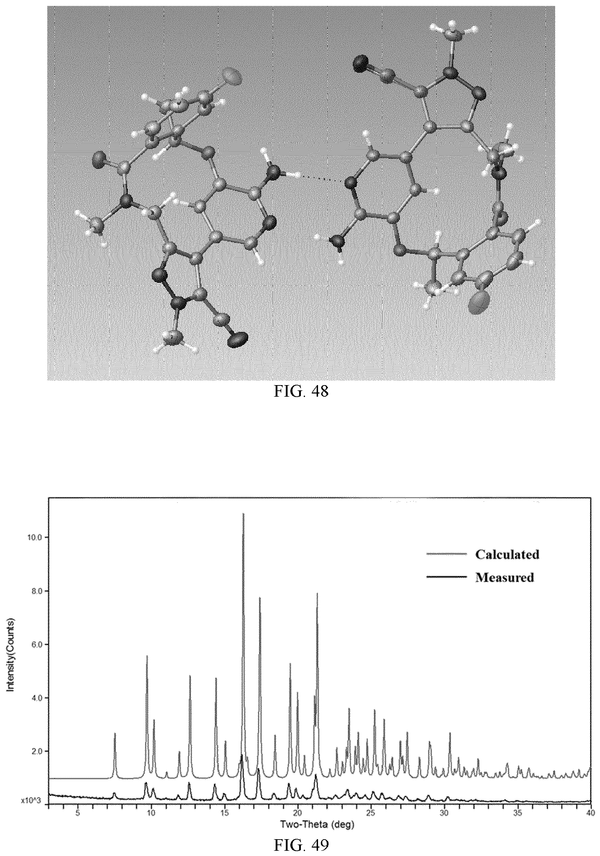

The refined single crystal structure of compound A is shown in FIG. 48. The parameters of the crystal structure obtained by the resolution are listed in Table 10. The single crystal is a massive crystal, has a structural formula of C₂₁H₁₆D₃FN₆O₂, and belongs to the monoclinic crystal system, *P*2₁ space group.

The single crystal sample was tested for XRPD and compared with the calculated values as shown in FIG. 49. All the characteristic peaks in XRPD can correspond to the simulated values and are consistent with the XRPD of compound A crystal form I, indicating that it was a single crystal of compound A crystal form I.

**Table 10 Crystal structure parameters of compound A**

| | |
|---|---|
| Molecular formula | C₂₁H₁₉FN₆O₂ |
| Molecular weight | 406.42 |
| Temperature/K | 180.00(10) |
| Crystal system | Monoclinic |
| Space group | *P2₁* |
| *a*/Å | 11.81120(10) |
| *b*/Å | 14.3957(2) |
| *c*/Å | 11.81800(10) |
| *α*/° | 90 |
| *β*/° | 94.5590(10) |
| *γ*/° | 90 |
| Volume /Å³ | 2003.06(4) |
| *Z* | 4 |
| *Density (calculated value)* g/cm³ | 1.348 |
| *Absorption coefficient*/mm⁻¹ | 0.808 |
| *F*(000) | 848.0 |
| Crystal size /mm³ | 0.23 × 0.2 × 0.13 |
| Radiation source | Cu Kα (λ = 1.54184) |
| 2*θ* range for data collection /° | 7.504 to 133.182 |
| Index ranges | -1 ≤ *h* ≤ 14, -17 ≤ *k* ≤ 17, -14 ≤ *l* ≤ 14 |
| Reflection collected | 28723 |
| Independent reflection | 7063 [*R*ᵢₙₜ = 0.0288, *R*_{sigma} = 0.0212] |
| Data / restraints / parameters | 7063/19/566 |
| Goodness-of-fit on *F*² | 1.016 |
| Final R indices [*I*>=*2σ* (*I*)] | *R₁* = *0.0296, wR₂* = *0.0792* |
| Absolute structure parameter | -0.12(5) |

### Example 11 Representative tablet formulation of compound A crystal form I

Oral film-coated tablets in 5 mg and 25 mg doses were prepared using a powder direct tableting process. The composition of the tablets is provided in Table 11-1.

**Table 11-1 Composition of tablet products in a unit dose**

| Ingredient of formulations | Specification of 5 mg | | Specification of 25 mg | | Function |
|---|---|---|---|---|---|
| | mg/tablet | weight % | mg/tablet | weight % | |
| Plain tablet | | | | | |
| Compound A crystal form I | 5 | 6.25 | 25 | 6.25 | API |
| Microcrystalline cellulose 102 | 46.2 | 57.75 | 231 | 57.75 | Diluent |
| Mannitol 50C | 23.2 | 29 | 116 | 29 | Diluent |
| Croscarmellose sodium | 2.4 | 3 | 12 | 3 | Disintegrant |
| Hydroxypropylcellulose EXF | 2.4 | 3 | 12 | 3 | Binder |
| Magnesium stearate | 0.8 | 1 | 4 | 1 | Lubricant |
| Purified water 1^{∗} | N/A | N/A | N/A | N/A | Lubricant |
| Total plain | 80 | 100 | 400 | 100 | N/A |

| Coating material | | | | | |
|---|---|---|---|---|---|
| Opadry II 85F620077** | 2.4 | 3 | 12 | 3 | Coating agent |
| Purified water 2*** | N/A | N/A | N/A | N/A | Dissolving agent for a coating agent |
| Total coated tablet | 82.4 | N/A | 412 | N/A | N/A |

| | | | | | |
|---|---|---|---|---|---|
| Note: * Purified water 1 was used as a wetting agent in the granulation process and was removed in the drying process. Purified water 1 was not counted in the material account. The general dosage of purified water was 35% by weight. However, the actual dosage can be adjusted according to the actual condition of wet granulation. ** The ingredients of Opadry II 85F620077 are titanium dioxide, polyvinyl alcohol, talc powder, polyethylene glycol, and iron oxide yellow. *** Purified water 2 was used as a solvent during the preparation of the coating solution and was removed during the coating process. Purified water 2 was not counted in the material account. | | | | | |

The tablets of 5 mg and 25 mg specification of this product were formulated in an equal proportion by using the same batch of total mixed material, and then pressing into different specifications of tablets, respectively. Taking the batch of 20,000 tablets of 5 mg specification and 30,000 tablets of 25 mg specification produced in total as representative, the formulation information of the batch is shown in Table 11-2 below.

**Table 11-2 Formulation information of the GMP batch of 5mg/25mg specifications**

| Tablet core | | | | | | |
|---|---|---|---|---|---|---|
| Ingredient | | | Amount (g) | | % | |
| Compound A crystal form I | | | 850.0 | | 6.25 | |
| Microcrystalline cellulose 102 | | | 7854.0 | | 57.75 | |
| Mannitol 50C | | | 3944.0 | | 29 | |
| Croscarmellose sodium | | | 408.0 | | 3 | |
| Hydroxypropylcellulose EXF | | | 408.0 | | 3 | |
| Purified water 1^{∗} | | | 4760.0 | | N/A | |
| Magnesium stearate | | | 136.0 | | 1 | |
| Total tablet core | | | 13600.0 | | 100 | |

| Coating | | | | | | |
|---|---|---|---|---|---|---|
| Opadry II 85F620077** | | | 408.0 | | 3 | |
| Purified water 2*** | | | 2922.0 | | N/A | |
| Total weight of coated tablets | | | 14008.0 | | N/A | |
| Package | | | | | | |

| Product specification | Packaging bottle specification | Sealing condition | | Packaging specification | | Packaging material |
|---|---|---|---|---|---|---|
| 5 mg/tablet | 45 mL | Child-safe cap for oral solid drug with aluminum foil gasket | | 30 tablet/ bottle | | High density polyethylene bottle for oral solid drug |
| 25 mg/tablet | 75 mL | Child-safe cap for oral solid drug with aluminum foil gasket | | 30 tablet/ bottle | | High density polyethylene bottle for oral solid drug |

| | | | | | | |
|---|---|---|---|---|---|---|
| Note: * Purified water 1 was used as a wetting agent in the granulation process and was removed in the drying process. Purified water 1 was not counted in the material account. The general dosage of purified water was 35% by weight. However, the actual dosage can be adjusted according to the actual condition of wet granulation. ** The ingredients of Opadry II 85F620077 are titanium dioxide, polyvinyl alcohol, talc powder, polyethylene glycol, and iron oxide yellow. *** Purified water 2 was used as a solvent during the preparation of the coating solution and was removed during the coating process. Purified water 2 was not counted in the material account. | | | | | | |

### The tablets were prepared as follows:

### 1. Weighing

The API compound A crystal form I and excipients were weighed according to the formulation amount.

### 2. Sieving

Compound A crystal form I was passed through a 120-mesh sieve (the LDPE bag that had ever loaded APIs was washed with about 1/4 of the total amount of mannitol 50C, and the washings were passed through the same 120-mesh sieve). The remaining amount of mannitol 50C was passed through a 60-mesh sieve and magnesium stearate was passed through a 60-mesh sieve.

### 3. Wet granulation

Mixing: Microcrystalline cellulose 102, compound A crystal form I, mannitol 50C, croscarmellose sodium and hydroxypropyl cellulose EXF were added to a wet granulation pot and pre-mixed with a stirring paddle speed of 250 rpm, a shear speed of 400 rpm, and a mixing time of 10 min.

Liquid spraying: After the mixing was completed, the stirring paddle speed was set at 200 rpm, the shear speed was set at 1000 rpm, and the peristaltic pump rotation speed was set at 241.7 rpm. The formulation amount of purified water was sprayed into the material pot of the wet granulator for about 3 min.

Granulation: After the liquid spraying was completed, granulation was conducted for 2 min with a stirring paddle speed at 200 rpm, and a shearing speed at 1000 rpm.

Wet finishing: The granulated material was finished in a finishing machine with a speed at 1500 rpm and a mesh size of 6×6 mm.

Drying: The fluidized bed was set with an inlet air temperature of 50-70 °C, an inlet air volume of 35-120 m³/h, a filter bag shaking period of 0.5s, and a shaking bag interval of 3-5s. The wet particles were pre-heated and then dried to a material moisture of <2% w/w.

Dry finishing: The dried material was finished in a finishing machine with a speed of 1500 rpm and a mesh size of 1.0 mm.

### 4. Total mixing

The material that had been dried and finished was added into a hopper mixer. Magnesium stearate was added and totally mixed with a mixing speed of 20 rpm, and a mixing period of 5 min. A sample was taken and tested for total mixing uniformity.

### 5. Tableting

The punch for the 5 mg tablet was a 6 mm dimple round punch and the punch for the 25 mg tablet was a 10.0 mm dimple round punch. After the equipment had a trial run, the formal production was carried out. Weight, hardness and friability of tablets were monitored online to make the tablets meet the following standards:

**Table 11-3 Tabletting standards for the preparation process of tablets of compound A crystal form I**

| Specification | Item | Standard |
|---|---|---|
| 5 mg | Target weight for single tablet | 80.0 mg |
| | Target weight range for single tablet | 80±4 mg |
| | Hardness range for single tablet | 30 N - 70N |
| | Friability | ≤1% |
| 25 mg | Target weight for single tablet | 400.0 mg |
| | Target weight range for single tablet | 400±20 mg |
| | Hardness range for single tablet | 70 N - 130 N |
| | Friability | ≤1% |

### 6. Coating

12% Opadry coating solution was freshly prepared with purified water.

Pre-heating: The inlet air temperature was set at 50-60°C and the coating pan was pre-heated at a rotation speed of 2 rpm.

Liquid spraying: When the coating pan was pre-heated to an outlet air temperature of 42°C, spray coating was performed.

Parameters of apparatus: the inlet air temperature was set at 50~70°C, the pot speed was set at 5~12 rpm, and the inlet air volume was set at 300±100 m³/h. Pump flow was set at 8 ml/min ~ 80 ml/min, atomization pressure was set at 1.5±1 bar, and atomization angle control pressure was set at 1 ± 0.5 bar for tablets of 5 mg specification and 2.5±1 bar for tablets of 25 mg specification. Coating parameters and coating weight gain were monitored. When the coating weight gain reached the target range of 3.0 ± 0.5%, the spraying was stopped.

Drying: The heating was stopped. The rotation speed of the coating pan was adjusted to 5 rpm, and the inlet air volume was adjusted to 200~500 m³/h. The product was discharged after drying for 5 minutes.

### 7. Packaging

The packaging materials for 5 mg tablets and 25 mg tablets were 45 mL and 75 mL high-density polyethylene bottles for oral solid drug, respectively. 30 tablets were contained in each bottle.

### 8. Labeling

Bottle labels were applied to product bottles, with one label per bottle.

## Claims

1. The crystal form I of the compound of formula (A): 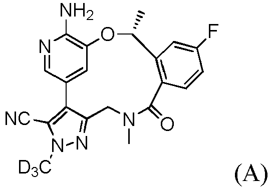 **characterized in that**: the X-ray powder diffraction pattern thereof obtained using CuK_{α} radiation includes at least the characteristic peaks located at the following °2θ: 16.175±0.2, 17.299±0.2 and 21.218±0.2.

2. The crystal form I of the compound of formula (A) of claim 1, **characterized in that**: the X-ray powder diffraction pattern thereof obtained using CuK_{α} radiation further includes the characteristic peaks located at the following °2θ: 9.637±0.2, 12.555±0.2, 14.343±0.2 and 19.366±0.2.

3. The crystal form I of the compound of formula (A) of claim 2, **characterized in that**: the X-ray powder diffraction pattern thereof obtained using CuK_{α} radiation has the following characteristic peaks:
| Angle °2θ±0.2 °2θ | Relative intensity% |
|---|---|
| 9.637 | 38.2 |
| 12.555 | 38.1 |
| 14.343 | 34.5 |
| 16.175 | 100 |
| 17.299 | 68.5 |
| 19.366 | 34.7 |
| 21.218 | 54.1 |

4. The crystal form I of the compound of formula (A) of claim 2 or 3, **characterized in that**: the X-ray powder diffraction pattern thereof obtained using CuK_{α} radiation further includes the characteristic peaks located at the following °2θ: 7.435±0.2, 10.11±0.2, 11.808±0.2, 14.922±0.2, 18.359±0.2, 19.859±0.2, 23.401±0.2, 23.939±0.2, 25.117±0.2, 25.727±0.2, 26.831±0.2 and 28.862±0.2.

5. The crystal form I of the compound of formula (A) of claim 1, **characterized by**: having an X-ray powder diffraction pattern substantially as shown in FIG. 1.

6. The crystal form I of the compound of formula (A) of any one of claims 1-5, further **characterized by**: having an endothermic peak at 232±2°C in differential scanning calorimetry analysis.

7. The crystal form I of the compound of formula (A) of any one of claims 1-6, further **characterized by**: having substantially no weight loss prior to 150°C in thermogravimetric analysis.

8. The crystal form I of the compound of formula (A), **characterized by**: having the following parameters:
| Space groups | *P2₁* |
|---|---|
| *a*/Å | 11.81120(10) |
| *b*/Å | 14.3957(2) |
| *c*/Å | 11.81800(10) |
| *α*/° | 90 |
| *β*/° | 94.5590(10) |
| *γ*/° | 90 |
| Volume/Å³ | 2003.06(4) |

9. The crystal form I of the compound of formula (A) of any one of claims 1-8, **characterized by**: having absorption peaks in an infrared absorption spectrum at the following cm⁻¹: 829±2, 878±2, 1069±2, 1252±2, 1344±2, 1368±2, 1395±2, 1420±2, 1433±2, 1491±2, 1499±2, 1616±2, 1645±2, 2228±2, 2934±2, 2980±2, 3111±2, 3184±2, 3308±2, 3383±2 and 3474±2.

10. The crystal form I of the compound of formula (A) of claim 9, further **characterized by**: having an infrared absorption spectrum substantially as shown in FIG. 14.

11. The crystal form I of the compound of formula (A) of any one of claims 1-10, further **characterized by**: having absorption peaks in a UV spectrum at the following nm: 206±2 and 317±2.

12. The crystal form I of the compound of formula (A) of claim 11, further **characterized by**: having a UV spectrum substantially as shown in FIG. 15.

13. The crystal form II of the compound of formula (A), **characterized in that**: the X-ray powder diffraction pattern thereof obtained using CuK_{α} radiation includes at least the characteristic peaks located at the following °2θ: 7.591±0.2, 12.081±0.2 and 23.364±0.2.

14. The crystal form II of the compound of formula (A) of claim 13, **characterized in that**: the X-ray powder diffraction pattern thereof obtained using CuK_{α} radiation further includes the characteristic peaks located at the following °2θ: 14.577±0.2, 15.595±0.2, 16.948±0.2, 17.615±0.2 and 20.448±0.2.

15. The crystal form II of the compound of formula (A) of claim 14, **characterized in that**: the X-ray powder diffraction pattern thereof obtained using CuK_{α} radiation has the following characteristic peaks:
| Angle °2θ±0.2 °2θ | Relative intensity% |
|---|---|
| 7.591 | 100 |
| 12.081 | 24.7 |
| 14.577 | 12.9 |
| 15.595 | 10.2 |
| 16.948 | 14.2 |
| 17.615 | 14.4 |
| 20.448 | 14.6 |
| 23.364 | 17.4 |

16. The crystal form II of the compound of formula (A) of claim 13, **characterized by**: having an X-ray powder diffraction pattern substantially as shown in FIG. 17.

17. The crystal form II of the compound of formula (A) of any one of claims 13-16, further **characterized by**: having an endothermic peak at 230±2°C in differential scanning calorimetry analysis.

18. The crystal form II of the compound of formula (A) of any one of claims 13-17, further **characterized by**: having a weight loss of about 6.69% prior to 160°C in thermogravimetric analysis.

19. The crystal form III of the compound of formula (A), **characterized in that**: the X-ray powder diffraction pattern thereof obtained using CuK_{α} radiation includes at least the characteristic peaks located at the following °2θ: 23.149±0.2.

20. The crystal form III of the compound of formula (A) of claim 19, **characterized in that**: the X-ray powder diffraction pattern thereof obtained using CuK_{α} radiation further includes the characteristic peaks located at the following °2θ: 11.429±0.2, 13.027±0.2, 14.542±0.2, 17.949±0.2 and 26.994±0.2.

21. The crystal form III of the compound of formula (A) of claim 20, **characterized in that**: the X-ray powder diffraction pattern thereof obtained using CuK_{α} radiation has the following characteristic peaks:
| Angle °2θ±0.2 °2θ | Relative intensity% |
|---|---|
| 11.429 | 22.2 |
| 13.027 | 21.7 |
| 14.542 | 21.3 |
| 17.949 | 19.1 |
| 23.149 | 100 |
| 26.994 | 20.4 |

22. The crystal form III of the compound of formula (A) of claim 20 or 21, **characterized in that**: the X-ray powder diffraction pattern thereof obtained using CuK_{α} radiation further includes the characteristic peaks located at the following °2θ: 10.543±0.2, 15.353±0.2, 18.362±0.2, 21.161±0.2, 22.506±0.2 and 26.006±0.2.

23. The crystal form III of the compound of formula (A) of claim 19, **characterized by**: having an X-ray powder diffraction pattern substantially as shown in FIG. 18.

24. The crystal form III of the compound of formula (A) of any one of claims 19-23, further **characterized by**: having an endothermic peak at 226±2°C in differential scanning calorimetry analysis.

25. The crystal form III of the compound of formula (A) of any one of claims 19-24, further **characterized by**: having a weight loss of about 5.31% prior to 165°C in thermogravimetric analysis.

26. The crystal form IV of the compound of formula (A), **characterized in that**: the X-ray powder diffraction pattern thereof obtained using CuK_{α} radiation includes at least the characteristic peaks located at the following °2θ: 10.113±0.2.

27. The crystal form IV of the compound of formula (A) of claim 26, **characterized in that**: the X-ray powder diffraction pattern thereof obtained using CuK_{α} radiation further includes the characteristic peaks located at the following °2θ: 11.583±0.2, 11.768±0.2, 12.098±0.2, 17.143±0.2 and 19.267±0.2.

28. The crystal form IV of the compound of formula (A) of claim 27, **characterized in that**: the X-ray powder diffraction pattern thereof obtained using CuK_{α} radiation has the following characteristic peaks:
| Angle °2θ±0.2 °2θ | Relative intensity% |
|---|---|
| 10.113 | 100 |
| 11.583 | 31 |
| 11.768 | 35.2 |
| 12.098 | 25.9 |
| 17.143 | 46.2 |
| 19.267 | 26.6 |

29. The crystal form IV of the compound of formula (A) of claim 27 or 28, **characterized in that**: the X-ray powder diffraction pattern thereof obtained using CuK_{α} radiation further includes the characteristic peaks located at the following °2θ: 9.718±0.2, 12.439±0.2, 13.339±0.2, 17.649±0.2, 20.703±0.2, 21.809±0.2, 22.427±0.2, 25.081±0.2, 27.576±0.2 and 28.959±0.2.

30. The crystal form IV of the compound of formula (A) of claim 26, **characterized by**: having an X-ray powder diffraction pattern substantially as shown in FIG. 19.

31. The crystal form IV of the compound of formula (A) of any one of claims 26-30, further **characterized by**: having an endothermic peak at 232±2°C in differential scanning calorimetry analysis.

32. The crystal form IV of the compound of formula (A) of any one of claims 26-31, further **characterized by**: having a weight loss of about 0.28% prior to 200°C in thermogravimetric analysis.

33. The crystal form V of the compound of formula (A), **characterized in that**: the X-ray powder diffraction pattern thereof obtained using CuK_{α} radiation includes at least the characteristic peaks located at the following °2θ: 6.939±0.2, 16.276±0.2 and 17.494±0.2.

34. The crystal form V of the compound of formula (A) of claim 33, **characterized in that**: the X-ray powder diffraction pattern thereof obtained using CuK_{α} radiation further includes the characteristic peaks located at the following °2θ: 4.912±0.2, 9.774±0.2, 12.709±0.2, 14.246±0.2, 14.482±0.2, 17.242±0.2, 18.519±0.2, 19.425±0.2, 21.001±0.2, 21.317±0.2, 22.734±0.2, 25.218±0.2 and 29.688±0.2.

35. The crystal form V of the compound of formula (A) of claim 34, **characterized in that**: the X-ray powder diffraction pattern thereof obtained using CuK_{α} radiation has the following characteristic peaks:
| Angle °2θ±0.2 °2θ | Relative intensity% |
|---|---|
| 4.912 | 28.1 |
| 6.939 | 100 |
| 9.774 | 30.1 |
| 12.709 | 41.4 |
| 14.246 | 29.4 |
| 14.482 | 33.4 |
| 16.276 | 55.9 |
| 17.242 | 42.4 |
| 17.494 | 76.9 |
| 18.519 | 25.7 |
| 19.425 | 34.7 |
| 21.001 | 27.3 |
| 21.317 | 29.5 |
| 22.734 | 26.7 |
| 25.218 | 28.2 |
| 29.688 | 26.1 |

36. The crystal form V of the compound of formula (A) of claim 34 or 35, **characterized in that**: the X-ray powder diffraction pattern thereof obtained using CuK_{α} radiation further includes the characteristic peaks located at the following °2θ: 10.326±0.2, 10.859±0.2, 15.289±0.2, 16.708±0.2, 19.941±0.2, 23.09±0.2, 23.424±0.2, 24.25±0.2, 25.808±0.2, 26.241±0.2, 26.987±0.2, 28.841±0.2, 29.332±0.2, 31.071±0.2 and 31.856±0.2.

37. The crystal form V of the compound of formula (A) of claim 33, **characterized by**: having an X-ray powder diffraction pattern substantially as shown in FIG. 20.

38. The crystal form V of the compound of formula (A) of any one of claims 33-37, further **characterized by**: having an endothermic peak at 232±2°C in differential scanning calorimetry analysis.

39. The crystal form V of the compound of formula (A) of any one of claims 33-38, further **characterized by**: having a weight loss of about 0.22% prior to 200°C in thermogravimetric analysis.

40. The crystal form VI of the compound of formula (A), **characterized in that**: the X-ray powder diffraction pattern thereof obtained using CuK_{α} radiation includes at least the characteristic peaks located at the following °2θ: 10.247±0.2, 12.198±0.2 and 17.258±0.2.

41. The crystal form VI of the compound of formula (A) of claim 40, **characterized in that**: the X-ray powder diffraction pattern thereof obtained using CuK_{α} radiation further includes the characteristic peaks located at the following °2θ: 12.514±0.2, 17.596±0.2, 19.406±0.2, 21.888±0.2 and 27.599±0.2.

42. The crystal form VI of the compound of formula (A) of claim 41, **characterized in that**: the X-ray powder diffraction pattern thereof obtained using CuK_{α} radiation has the following characteristic peaks:
| Angle °2θ±0.2 °2θ | Relative intensity% |
|---|---|
| 10.247 | 100 |
| 12.198 | 70.6 |
| 12.514 | 42.8 |
| 17.258 | 53.8 |
| 17.596 | 44.5 |
| 19.406 | 27.9 |
| 21.888 | 30.3 |
| 27.599 | 27.5 |

43. The crystal form VI of the compound of formula (A) of claim 41 or 42, **characterized in that**: the X-ray powder diffraction pattern thereof obtained using CuK_{α} radiation further includes the characteristic peaks located at the following °2θ: 18.659±0.2, 22.479±0.2, 23.799±0.2, 24.41±0.2, 25.158±0.2 and 28.504±0.2.

44. The crystal form VI of the compound of formula (A) of claim 40, **characterized by**: having an X-ray powder diffraction pattern substantially as shown in FIG. 21.

45. The crystal form VI of the compound of formula (A) of any one of claims 40-44, further **characterized by**: having an endothermic peak at 233±2°C in differential scanning calorimetry analysis.

46. The crystal form VI of the compound of formula (A) of any one of claims 40-45, further **characterized by**: having substantially no weight loss prior to 200°C in thermogravimetric analysis.

47. The crystal form VII of the compound of formula (A), **characterized in that**: the X-ray powder diffraction pattern thereof obtained using CuK_{α} radiation includes at least the characteristic peaks located at the following °2θ: 7.138±0.2 and 9.876±0.2.

48. The crystal form VII of the compound of formula (A) of claim 47, **characterized in that**: the X-ray powder diffraction pattern thereof obtained using CuK_{α} radiation further includes the characteristic peaks located at the following °2θ: 12.572±0.2, 12.945±0.2, 14.675±0.2 and 17.16±0.2.

49. The crystal form VII of the compound of formula (A) of claim 48, **characterized in that**: the X-ray powder diffraction pattern thereof obtained using CuK_{α} radiation has the following characteristic peaks:
| Angle °2θ±0.2 °2θ | Relative intensity% |
|---|---|
| 7.138 | 100 |
| 9.876 | 51.2 |
| 12.572 | 31.6 |
| 12.945 | 38.5 |
| 14.675 | 30.3 |
| 17.16 | 26.2 |

50. The crystal form VII of the compound of formula (A) of claim 47, **characterized by**: having an X-ray powder diffraction pattern substantially as shown in FIG. 22.

51. The crystal form VII of the compound of formula (A) of any one of claims 47-50, further **characterized by**: having an endothermic peak at 232±2°C in differential scanning calorimetry analysis.

52. The crystal form VII of the compound of formula (A) of any one of claims 47-51, further **characterized by**: having a weight loss of about 0.35% prior to 200°C in thermogravimetric analysis.

53. Maleate (1:1) crystal form I of the compound of formula (A), **characterized in that**: the X-ray powder diffraction pattern thereof obtained using CuK_{α} radiation includes at least the characteristic peaks located at the following °2θ: 9.737±0.2, 12.241±0.2 and 23.08±0.2.

54. The maleate (1:1) crystal form I of the compound of formula (A) of claim 53, **characterized in that**: the X-ray powder diffraction pattern thereof obtained using CuK_{α} radiation further includes the characteristic peaks located at the following °2θ: 11.982±0.2, 13.601±0.2, 16.495±0.2, 17.186±0.2, 19.625±0.2 and 24.527±0.2.

55. The maleate (1:1) crystal form I of the compound of formula (A) of claim 54, **characterized in that**: the X-ray powder diffraction pattern thereof obtained using CuK_{α} radiation has the following characteristic peaks:
| Angle °2θ±0.2 °2θ | Relative intensity% |
|---|---|
| 9.737 | 80 |
| 11.982 | 41 |
| 12.241 | 100 |
| 13.601 | 28 |
| 16.495 | 32 |
| 17.186 | 25 |
| 19.625 | 38 |
| 23.08 | 98 |
| 24.527 | 36 |

56. The maleate (1:1) crystal form I of the compound of formula (A) of claim 54 or 55, **characterized in that**: the X-ray powder diffraction pattern thereof obtained using CuK_{α} radiation further includes the characteristic peaks located at the following °2θ: 7.577±0.2, 15.286±0.2, 17.358±0.2, 17.553±0.2, 19.971±0.2, 22.087±0.2, 23.879±0.2, 25.239±0.2, 25.844±0.2, 26.189±0.2, 29.644±0.2 and 31.501±0.2.

57. The maleate (1:1) crystal form I of the compound of formula (A) of claim 53, **characterized by**: having an X-ray powder diffraction pattern substantially as shown in FIG. 25.

58. The maleate (1:1) crystal form I of the compound of formula (A) of any one of claims 53-57, further **characterized by**: having an endothermic peak at 209±2°C in differential scanning calorimetry analysis.

59. The maleate (1:1) crystal form I of the compound of formula (A) of any one of claims 53-58, further **characterized by**: having a weight loss of about 0.44% prior to 175°C in thermogravimetric analysis.

60. Acetate (1:1) crystal form I of the compound of formula (A), **characterized in that**: the X-ray powder diffraction pattern thereof obtained using CuK_{α} radiation includes at least the characteristic peaks located at the following °2θ: 12.866±0.2 and 23.129±0.2.

61. The acetate (1:1) crystal form I of the compound of formula (A) of claim 60, **characterized in that**: the X-ray powder diffraction pattern thereof obtained using CuK_{α} radiation further includes the characteristic peaks located at the following °2θ: 10.521±0.2, 11.409±0.2, 13.005±0.2, 14.521±0.2, 17.91±0.2 and 21.14±0.2.

62. The acetate (1:1) crystal form I of the compound of formula (A) of claim 61, **characterized in that**: the X-ray powder diffraction pattern thereof obtained using CuK_{α} radiation has the following characteristic peaks:
| Angle °2θ±0.2 °2θ | Relative intensity% |
|---|---|
| 10.521 | 35.1 |
| 11.409 | 46.7 |
| 12.866 | 50.7 |
| 13.005 | 26.2 |
| 14.521 | 45.3 |
| 17.91 | 39.3 |
| 21.14 | 27.3 |
| 23.129 | 100 |

63. The acetate (1:1) crystal form I of the compound of formula (A) of claim 61 or 62, **characterized in that**: the X-ray powder diffraction pattern thereof obtained using CuK_{α} radiation further includes the characteristic peaks located at the following °2θ: 15.349±0.2, 16.707±0.2, 17.236±0.2, 18.343±0.2, 19.961±0.2, 22.536±0.2, 25.985±0.2 and 26.993±0.2.

64. The acetate (1:1) crystal form I of the compound of formula (A) of claim 60, **characterized by**: having an X-ray powder diffraction pattern substantially as shown in FIG. 28.

65. The acetate (1:1) crystal form I of the compound of formula (A) of any one of claims 60-64, further **characterized by**: having an endothermic peak at 232±2°C in differential scanning calorimetry analysis.

66. The acetate (1:1) crystal form I of the compound of formula (A) of any one of claims 60-65, further **characterized by**: having a weight loss of about 0.67% prior to 140°C in thermogravimetric analysis.

67. p-Toluenesulfonate (1:1) crystal form I of the compound of formula (A), **characterized in that**: the X-ray powder diffraction pattern thereof obtained using CuK_{α} radiation includes at least the characteristic peaks located at the following °2θ: 10.583±0.2 and 21.674±0.2.

68. The p-toluenesulfonate (1:1) crystal form I of the compound of formula (A) of claim 67, **characterized in that**: the X-ray powder diffraction pattern thereof obtained using CuK_{α} radiation further includes the characteristic peaks located at the following °2θ: 12.968±0.2 and 14.503±0.2.

69. The p-toluenesulfonate (1:1) crystal form I of the compound of formula (A) of claim 68, **characterized in that**: the X-ray powder diffraction pattern thereof obtained using CuK_{α} radiation has the following characteristic peaks:
| Angle °2θ±0.2 °2θ | Relative intensity% |
|---|---|
| 10.583 | 100 |
| 12.968 | 29.6 |
| 14.503 | 38.6 |
| 21.674 | 51.3 |

70. The p-toluenesulfonate (1:1) crystal form I of the compound of formula (A) of claim 68 or 69, **characterized in that**: the X-ray powder diffraction pattern thereof obtained using CuK_{α} radiation further includes the characteristic peaks located at the following °2θ: 5.544±0.2, 14.012±0.2, 16.886±0.2, 18.417±0.2, 19.607±0.2, 21.298±0.2, 23.266±0.2 and 26.437±0.2.

71. The p-toluenesulfonate (1:1) crystal form I of the compound of formula (A) of claim 67, **characterized by**: having an X-ray powder diffraction pattern substantially as shown in FIG. 31.

72. The p-toluenesulfonate (1:1) crystal form I of the compound of formula (A) of any one of claims 67-71, further **characterized by**: having an endothermic peak at 269±2°C in differential scanning calorimetry analysis.

73. The p-toluenesulfonate (1:1) crystal form I of the compound of formula (A) of any one of claims 67-72, further **characterized by**: having a weight loss of about 0.6% prior to 220°C in thermogravimetric analysis.

74. Oxalate (1:1) crystal form I of the compound of formula (A), **characterized in that**: the X-ray powder diffraction pattern thereof obtained using CuK_{α} radiation includes at least the characteristic peaks located at the following °2θ: 4.32±0.2 and 6.642±0.2.

75. The oxalate (1:1) crystal form I of the compound of formula (A) of claim 74, **characterized in that**: the X-ray powder diffraction pattern thereof obtained using CuK_{α} radiation further includes the characteristic peaks located at the following °2θ: 5.54±0.2, 10.366±0.2, 10.98±0.2 and 13.242±0.2.

76. The oxalate (1:1) crystal form I of the compound of formula (A) of claim 75, **characterized in that**: the X-ray powder diffraction pattern thereof obtained using CuK_{α} radiation has the following characteristic peaks:
| Angle °2θ±0.2 °2θ | Relative intensity% |
|---|---|
| 4.32 | 31.2 |
| 5.54 | 27.5 |
| 6.642 | 100 |
| 10.366 | 18.1 |
| 10.98 | 15 |
| 13.242 | 19.6 |

77. The oxalate (1:1) crystal form I of the compound of formula (A) of claim 74, **characterized by**: having an X-ray powder diffraction pattern substantially as shown in FIG. 34.

78. The oxalate (1:1) crystal form I of the compound of formula (A) of any one of claims 74-77, further **characterized by**: having an endothermic peak at 209±2°C in differential scanning calorimetry analysis.

79. The oxalate (1:1) crystal form I of the compound of formula (A) of any one of claims 74-78, further **characterized by**: having a weight loss of about 0.86% prior to 130°C in thermogravimetric analysis.

80. Sulfate crystal form I of the compound of formula (A), **characterized in that**: the X-ray powder diffraction pattern thereof obtained using CuK_{α} radiation includes at least the characteristic peaks located at the following °2θ: 15.763±0.2 and 23.266±0.2.

81. The sulfate crystal form I of the compound of formula (A) of claim 80, **characterized in that**: the X-ray powder diffraction pattern thereof obtained using CuK_{α} radiation further includes the characteristic peaks located at the following °2θ: 11.604±0.2, 13.318±0.2, 14.74±0.2, 15.961±0.2, 18.385±0.2, 19.228±0.2, 21.413±0.2, 22.361±0.2, 23.936±0.2 and 24.958±0.2.

82. The sulfate crystal form I of the compound of formula (A) of claim 81, **characterized in that**: the X-ray powder diffraction pattern thereof obtained using CuK_{α} radiation has the following characteristic peaks:
| Angle °2θ±0.2 °2θ | Relative intensity% |
|---|---|
| 11.604 | 33.5 |
| 13.318 | 37.9 |
| 14.74 | 30.9 |
| 15.763 | 96.3 |
| 15.961 | 28.5 |
| 18.385 | 45.4 |
| 19.228 | 28.6 |
| 21.413 | 44.1 |
| 22.361 | 44.3 |
| 23.266 | 100 |
| 23.936 | 27.4 |
| 24.958 | 28.3 |

83. The sulfate crystal form I of the compound of formula (A) of claim 81 or 82, **characterized in that**: the X-ray powder diffraction pattern thereof obtained using CuK_{α} radiation further includes the characteristic peaks located at the following °2θ: 11.78±0.2, 12.667±0.2, 19.447±0.2, 22.083±0.2, 22.576±0.2, 23.618±0.2, 27.303±0.2, 27.522±0.2, 28.765±0.2, 29.725±0.2, 30.906±0.2 and 32.032±0.2.

84. The sulfate crystal form I of the compound of formula (A) of claim 80, **characterized by**: having an X-ray powder diffraction pattern substantially as shown in FIG. 35.

85. The sulfate crystal form I of the compound of formula (A) of any one of claims 80-84, further **characterized by**: having an endothermic peak at 251±2°C in differential scanning calorimetry analysis.

86. The sulfate crystal form I of the compound of formula (A) of any one of claims 80-85, further **characterized by**: having a weight loss of about 2.95% prior to 90°C in thermogravimetric analysis.

87. Hydrobromide crystal form I of the compound of formula (A), **characterized in that**: the X-ray powder diffraction pattern thereof obtained using CuK_{α} radiation includes at least the characteristic peaks located at the following °2θ: 13.206±0.2, 23.995±0.2 and 24.941±0.2.

88. The hydrobromide crystal form I of the compound of formula (A) of claim 87, **characterized in that**: the X-ray powder diffraction pattern thereof obtained using CuK_{α} radiation further includes the characteristic peaks located at the following °2θ: 9.222±0.2, 11.905±0.2, 19.937±0.2, 26.773±0.2 and 27.5±0.2.

89. The hydrobromide crystal form I of the compound of formula (A) of claim 88, **characterized in that**: the X-ray powder diffraction pattern thereof obtained using CuK_{α} radiation has the following characteristic peaks:
| Angle °2θ±0.2 °2θ | Relative intensity% |
|---|---|
| 9.222 | 25.3 |
| 11.905 | 39.8 |
| 13.206 | 100 |
| 19.937 | 28 |
| 23.995 | 83.3 |
| 24.941 | 60.9 |
| 26.773 | 33.3 |
| 27.5 | 45.1 |

90. The hydrobromide crystal form I of the compound of formula (A) of claim 88 or 89, **characterized in that**: the X-ray powder diffraction pattern thereof obtained using CuK_{α} radiation further includes the characteristic peaks located at the following °2θ: 12.652±0.2, 14.702±0.2, 16.396±0.2, 16.924±0.2, 18.636±0.2, 19.148±0.2, 20.294±0.2, 21.102±0.2, 21.532±0.2, 25.492±0.2 and 33.154±0.2.

91. The hydrobromide crystal form I of the compound of formula (A) of claim 87, **characterized by**: having an X-ray powder diffraction pattern substantially as shown in FIG. 36.

92. The hydrobromide crystal form I of the compound of formula (A) of any one of claims 87-91, further **characterized by**: having an endothermic peak at 241±2°C in differential scanning calorimetry analysis.

93. The hydrobromide crystal form I of the compound of formula (A) of any one of claims 87-92, further **characterized by**: having a weight loss of about 8.18% prior to 150°C in thermogravimetric analysis.

94. Hydrochloride crystal form I of the compound of formula (A), **characterized in that**: the X-ray powder diffraction pattern thereof obtained using CuK_{α} radiation includes at least the characteristic peaks located at the following °2θ: 12.079±0.2, 13.319±0.2 and 24.093±0.2.

95. The hydrochloride crystal form I of the compound of formula (A) of claim 94, **characterized in that**: the X-ray powder diffraction pattern thereof obtained using CuK_{α} radiation further includes the characteristic peaks located at the following °2θ: 9.38±0.2, 12.749±0.2, 24.92±0.2 and 27.559±0.2.

96. The hydrochloride crystal form I of the compound of formula (A) of claim 95, **characterized in that**: the X-ray powder diffraction pattern thereof obtained using CuK_{α} radiation has the following characteristic peaks:
| Angle °2θ±0.2 °2θ | Relative intensity% |
|---|---|
| 9.38 | 35.3 |
| 12.079 | 52 |
| 12.749 | 26.8 |
| 13.319 | 100 |
| 24.093 | 59.9 |
| 24.92 | 47 |
| 27.559 | 25.7 |

97. The hydrochloride crystal form I of the compound of formula (A) of claim 95 or 96, **characterized in that**: the X-ray powder diffraction pattern thereof obtained using CuK_{α} radiation further includes the characteristic peaks located at the following °2θ: 6.699±0.2, 7.944±0.2, 8.28±0.2, 14.111±0.2, 14.758±0.2, 17.103±0.2, 18.618±0.2, 19.996±0.2, 20.449±0.2, 21.83±0.2, 25.118±0.2, 26.613±0.2 and 27.006±0.2.

98. The hydrochloride crystal form I of the compound of formula (A) of claim 94, **characterized by**: having an X-ray powder diffraction pattern substantially as shown in FIG. 37.

99. The hydrochloride crystal form I of the compound of formula (A) of any one of claims 94-98, further **characterized by**: having an endothermic peak at 221±2°C in differential scanning calorimetry analysis.

100. The hydrochloride crystal form I of the compound of formula (A) of any one of claims 94-99, further **characterized by**: having a weight loss of about 1.67% prior to 125°C, and a weight loss of about 3.84% between 125 and 230°C in thermogravimetric analysis.

101. Mesylate (1:1) crystal form I of the compound of formula (A), **characterized in that**: the X-ray powder diffraction pattern thereof obtained using CuK_{α} radiation includes at least the characteristic peaks located at the following °2θ: 8.338±0.2.

102. The mesylate (1:1) crystal form I of the compound of formula (A) of claim 101, **characterized in that**: the X-ray powder diffraction pattern thereof obtained using CuK_{α} radiation further includes the characteristic peaks located at the following °2θ: 11.706±0.2, 13.932±0.2, 14.738±0.2, 18.341±0.2, 21.148±0.2, 21.588±0.2, 22.597±0.2 and 25.732±0.2.

103. The mesylate (1:1) crystal form I of the compound of formula (A) of claim 102, **characterized in that**: the X-ray powder diffraction pattern thereof obtained using CuK_{α} radiation has the following characteristic peaks:
| Angle °2θ±0.2 °2θ | Relative intensity% |
|---|---|
| 8.338 | 100 |
| 11.706 | 91.7 |
| 13.932 | 80.9 |
| 14.738 | 56.7 |
| 18.341 | 82.2 |
| 21.148 | 50.3 |
| 21.588 | 72 |
| 22.597 | 51 |
| 25.732 | 69.4 |

104. The mesylate (1:1) crystal form I of the compound of formula (A) of claim 102 or 103, **characterized in that**: the X-ray powder diffraction pattern thereof obtained using CuK_{α} radiation further includes the characteristic peaks located at the following °2θ: 9.363±0.2, 10.092±0.2, 12.513±0.2, 15.053±0.2, 15.742±0.2, 19.093±0.2, 23.17±0.2, 23.716±0.2, 24.469±0.2, 24.65±0.2, 24.824±0.2, 30.238±0.2 and 32.189±0.2.

105. The mesylate (1:1) crystal form I of the compound of formula (A) of claim 101, **characterized by**: having an X-ray powder diffraction pattern substantially as shown in FIG. 40.

106. A pharmaceutical composition comprising the crystal form of any one of claims 1-105, and a pharmaceutically acceptable excipient.

107. A pharmaceutical composition, comprising the following ingredients:
(i) the crystal form of any one of claims 1-105,
(ii) a diluent,
(iii) a disintegrant,
(iv) a binder, and
(v) a lubricant.

108. The pharmaceutical composition of claim 107, wherein the crystal form accounts for 1-30%, alternatively, 2-20%, alternatively, 3-15%, yet alternatively, about 4%, 5%, 6%, 7%, 8%, 9% or 10% by weight of the total weight of the pharmaceutical composition, based on the weight of the free base of the compound; alternatively, wherein the amount of the crystal form in a unit dose is 1-100 mg, alternatively, 2-50 mg, alternatively, 3-40 mg, alternatively, about 5, 10, 15, 20, 25, 30, 35 or 40 mg.

109. The pharmaceutical composition of any one of claims 107-108, wherein the diluent accounts for 65-95%, alternatively, 70-90%, alternatively, about 80%, 81%, 82%, 83%, 84%, 85%, 86%, 87%, 88%, 89% or 90% by weight of the total weight of the pharmaceutical composition; alternatively, wherein the amount of the diluent in a unit dose is 50-380 mg, alternatively, 60-360 mg, alternatively, 70-350 mg, such as, about 70 mg or 350 mg.

110. The pharmaceutical composition of any one of claims 107-109, wherein the diluent is selected from the group consisting of microcrystalline cellulose, anhydrous calcium hydrogen phosphate, and mannitol, e.g., microcrystalline cellulose 102, mannitol 100SD, and mannitol 50C, and a mixture thereof; alternatively, where both of microcrystalline cellulose 102 and mannitol 50C are present, the weight ratio of microcrystalline cellulose 102 to mannitol 50C is 5:1 to 1:5, alternatively 3:1 to 1:2, alternatively about 2:1.

111. The pharmaceutical composition of any one of claims 107-110, wherein the disintegrant accounts for 1-5%, alternatively, 2-4%, alternatively, about 2%, 2.5%, 3%, 3.5% or 4% by weight of the total weight of the pharmaceutical composition; alternatively, wherein the amount of the disintegrant in a unit dose is 1-20 mg, alternatively, 2-16 mg, alternatively, about 2, 2.5, 3, 6, 9 or 12 mg.

112. The pharmaceutical composition of any one of claims 107-111, wherein the disintegrant is croscarmellose sodium or crospovidone XL-10, alternatively croscarmellose sodium.

113. The pharmaceutical composition of any one of claims 107-112, wherein the binder accounts for 1-5%, alternatively, 2-4%, alternatively, about 2%, 2.5%, 3%, 3.5% or 4% by weight of the total weight of the pharmaceutical composition; alternatively, wherein the amount of binder in a unit dose is 1-20 mg, alternatively, 2-16 mg, alternatively, about 2, 2.5, 3, 6, 9 or 12 mg.

114. The pharmaceutical composition of any one of claims 107-113, wherein the binder is hydroxypropylcellulose EXF or povidone K30, alternatively hydroxypropylcellulose EXF.

115. The pharmaceutical composition of any one of claims 107-114, wherein the lubricant accounts for 0.1-5%, alternatively, 0.5-2%, alternatively, about 1% by weight of the total weight of the pharmaceutical composition; alternatively, wherein the amount of the lubricant in a unit dose is 0.1-20 mg, alternatively, 0.5-8 mg, alternatively, about 0.5, 1, 2, 3, 4, 5, 6, 7 or 8 mg.

116. The pharmaceutical composition of any one of claims 107-115, wherein the lubricant is magnesium stearate or sodium stearyl fumarate PRUV, alternatively magnesium stearate.

117. The pharmaceutical composition of any one of claims 107-116, which comprises the following components:
(i) 1-30% by weight of compound A crystal form I,
(ii) 65-95% by weight of microcrystalline cellulose 102 and mannitol 50C (2:1, by weight),
(iii) 2-4% by weight of croscarmellose sodium,
(iv) 2-4% by weight of hydroxypropylcellulose EXF, and
(v) 0.1-5% by weight of magnesium stearate.

118. The pharmaceutical composition of claim 117, wherein the unit dose comprises the following components:
(i) about 5 mg of compound A crystal form I,
(ii) about 45 mg of microcrystalline cellulose 102 and about 25 mg of mannitol 50C,
(iii) about 2.5 mg of croscarmellose sodium,
(iv) about 2.5 mg of hydroxypropylcellulose EXF, and
(v) about 1 mg of magnesium stearate.

119. The pharmaceutical composition of claim 117, wherein the unit dose comprises the following components:
(i) about 25 mg of compound A crystal form VI,
(ii) about 230 mg of lactose monohydrate and about 120 mg of microcrystalline cellulose,
(iii) about 12 mg of croscarmellose sodium,
(iv) about 12 mg of hydroxypropylcellulose EXF, and
(v) about 4 mg of magnesium stearate.

120. The pharmaceutical composition of any one of claims 107-119, which is a tablet, alternatively a coated tablet; alternatively, the coating agent is Opadry II 85F620077.

121. Use of the crystal form of any one of claims 1-105 in the manufacture of a medicament for the treatment and/or prevention of diseases mediated by ALK and ROS1 kinases and mutants thereof.

122. Use of the crystal form of any one of claims 1-105 in the manufacture of a medicament for the treatment and/or prevention of the following diseases: cell proliferative diseases, inflammation, infection, immunological diseases, organ transplantation, viral diseases, cardiovascular diseases or metabolic diseases, such as non-small cell lung cancer, lung cancer, head and neck cancer, breast cancer, prostate cancer, esophageal cancer, rectal cancer, colon cancer, nasopharyngeal cancer, uterine cancer, pancreatic cancer, lymphoma, blood cancer, osteosarcoma, melanoma, kidney cancer, stomach cancer, liver cancer, bladder cancer, thyroid cancer, large intestine cancer, rheumatoid arthritis, osteoarthritis, rheumatoid spondylitis, gout, asthma, bronchitis, rhinitis, chronic obstructive pulmonary disease, or cystic fibrosis.

123. The crystal form of any one of claims 1-105, for use in the treatment and/or prevention of diseases mediated by ALK and ROS1 kinases and mutants thereof.

124. The crystal form of the compound of formula (A) of any one of claims 1-105, for use in the treatment and/or prevention of cell proliferative diseases, inflammation, infection, immunological diseases, organ transplantation, viral diseases, cardiovascular diseases or metabolic diseases, such as non-small cell lung cancer, lung cancer, head and neck cancer, breast cancer, prostate cancer, esophageal cancer, rectal cancer, colon cancer, nasopharyngeal cancer, uterine cancer, pancreatic cancer, lymphoma, blood cancer, osteosarcoma, melanoma, kidney cancer, stomach cancer, liver cancer, bladder cancer, thyroid cancer, large intestine cancer, rheumatoid arthritis, osteoarthritis, rheumatoid spondylitis, gout, asthma, bronchitis, rhinitis, chronic obstructive pulmonary disease, or cystic fibrosis.

125. A method of treating and/or preventing diseases mediated by ALK and ROS1 kinases and mutants thereof in a subject, comprising administering to the subject the crystal form of any one of claims 1-105.

126. A method of treating and/or preventing the following disease in a subject, comprising administering to the subject the crystal form of compound of formula (A) of any one of claims 1-105: cell proliferative diseases, inflammation, infection, immunological diseases, organ transplantation, viral diseases, cardiovascular diseases or metabolic diseases, such as non-small cell lung cancer, lung cancer, head and neck cancer, breast cancer, prostate cancer, esophageal cancer, rectal cancer, colon cancer, nasopharyngeal cancer, uterine cancer, pancreatic cancer, lymphoma, blood cancer, osteosarcoma, melanoma, kidney cancer, stomach cancer, liver cancer, bladder cancer, thyroid cancer, large intestine cancer, rheumatoid arthritis, osteoarthritis, rheumatoid spondylitis, gout, asthma, bronchitis, rhinitis, chronic obstructive pulmonary disease, or cystic fibrosis.
